# EUROPEAN PATENT SPECIFICATION

(11) **EP 0 956 508 B1**
(45) Date of publication and mention of the grant of the patent: **09.04.2003**
(21) Application number: 98953249.4
(22) Date of filing: 02.10.1998
(51) Int. Cl.: G01N 33/68

(54) **ICAM-4 AND DIAGNOSTIC USES THEREOF**
ICAM-4 UND DESSEN DIAGNOSTISCHE VERWENDUNGEN
ICAM-4 ET SES UTILISATIONS DIAGNOSTIQUES

(30) Priority: 02.10.1997 US 942867
(43) Date of publication of application: 17.11.1999
(73) Proprietor: ICOS CORPORATION, Bothell, WA 98021 (US)
(72) Inventor: KILGANNON, Patrick, D., Bothell, WA 98011 (US); GALLATIN, W., Michael, Mercer Island, WA 98040 (US)
(74) Representative: Richardson, Kate
(86) International application number: US9820876
(87) International publication number: WO99018441

(56) References cited:
- WO-A-92/22323
- WO-A-96/40916

## Description

### FIELD OF THE INVENTION

The present invention relates generally to cellular adhesion molecules and more particularly to the cloning and expression of DNA encoding a heretofore unknown polypeptide designated "ICAM-4" which possesses structural relatedness to the intercellular adhesion molecules ICAM-1, ICAM-2, and ICAM-R.

### BACKGROUND OF THE INVENTION

Research spanning the last decade has significantly elucidated the molecular events attending cell-cell interactions in the body, especially those events involved in the movement and activation of cells in the immune system, and more recently, those involved in development and normal physiological function of cells in the nervous system. See generally, Springer, *Nature, 346:* 425-434 (1990) regarding cells of the immune system, and Yoshihara, *et al. Neurosci.Res. 10*:83-105 (1991) and Sonderegger and Rathjen, *J. Cell Biol. 119*:1387-1394 (1992) regarding cells of the nervous system. Cell surface proteins, and especially the so-called Cellular Adhesion Molecules ("CAMS") have correspondingly been the subject of pharmaceutical research and development having as its goal intervention in the processes of leukocyte extravasation to sites of inflammation and leukocyte movement to distinct target tissues, as well as neuronal differentiation and formation of complex neuronal circuitry. The isolation and characterization of cellular adhesion molecules, the cloning and expression of DNA sequences encoding such molecules, and the development of therapeutic and diagnostic agents relevant to inflammatory processes and development and function of the nervous system have also been the subject of numerous U.S. and foreign applications for Letters Patent. See Edwards, *Current Opinion in Therapeutic Patents, 1(11)*: 1617-1630 (1991) and particularly the published "patent literature references" cited therein.

Of fundamental interest to the background of the present invention are the prior identification and characterization of certain mediators of cell adhesion events, the "leukointegrins," LFA-1, MAC-1 and gp 150.95 (referred to in WHO nomenclature as CD18/CD11a, CD18/CD11b, and CD18/CD11c, respectively) which form a subfamily of heterodimeric "integrin" cell surface proteins present on B lymphocytes, T lymphocytes, monocytes and granulocytes. See, e.g., Table 1 of Springer, *supra*, at page 429. Also of interest are other single chain adhesion molecules (CAMs) that have been implicated in leukocyte activation, adhesion, motility and the like, which are events attendant to the inflammatory process. For example, it is presently believed that prior to the leukocyte extravasation which characterizes inflammatory processes, activation of integrins constitutively expressed on leukocytes occurs and is followed by a tight ligand/receptor interaction between the integrins (e.g., LFA-1) and one or both of two distinct intercellular adhesion molecules (ICAMs) designated ICAM-1 and ICAM-2 which are expressed on blood vessel endothelial cell surfaces and on other leukocytes.

Like the other CAMs characterized to date, [e.g., vascular adhesion molecule (VCAM-1) as described in PCT WO 90/13300 published November 15, 1990; and platelet endothelial cell adhesion molecule (PECAM-1) as described in Newman *et al., Science, 247*: 1219-1222 (1990) and PCT WO 91/10683 published July 25, 1991], ICAM-1 and ICAM-2 are structurally homologous to other members of the immunoglobulin gene superfamily in that the extracellular portion of each is comprised of a series of domains sharing a similar carboxy terminal motif. A "typical" immunoglobulin-like domain contains a loop structure usually anchored by a disulfide bond between two cysteines at the extremity of each loop. ICAM-1 includes five immunoglobulin-like domains; ICAM-2, which differs from ICAM-1 in terms of cell distribution, includes two such domains; PECAM-1 includes six; VCAM includes six or seven, depending on splice variations, and so on. Moreover, CAMs typically include a hydrophobic "transmembrane" region believed to participate in orientation of the molecule at the cell surface and a carboxy terminal "cytoplasmic" region. Graphic models of the operative disposition of CAMs generally show the molecule anchored in the cell membrane at the transmembrane region with the cytoplasmic "tail" extending into the cell cytoplasm and one or more immunoglobulin-like loops extending outward from the cell surface.

A number of neuronal cells express surface receptors with extracellular Ig-like domains, structurally similarity to the ICAMs. See for example, Yoshihara, *et al., supra.* In addition to Ig-like domains, many adhesion molecules of the nervous system also contain tandemly repeated fibronectin-like sequences in the extracellular domain.

A variety of therapeutic uses has been projected for intercellular adhesion molecules, including uses premised on the ability of ICAM-1 to bind human rhinovirus. European Patent Application 468 257 A published January 29, 1992, for example, addresses the development of multimeric configurations and forms of ICAM-1 (including full length and truncated molecular forms) proposed to have enhanced ligand/receptor binding activity, especially in binding to viruses, lymphocyte associated antigens and pathogens such as *Plasmodium falciparum.*

In a like manner, a variety of uses has been projected for proteins immunologically related to intercellular adhesion molecules. WO91/16928, published November 14, 1991, for example, addresses humanized chimeric anti-ICAM-1 antibodies and their use in treatment of specific and non-specific inflammation, viral infection and asthma. Anti-ICAM-1 antibodies and fragments thereof are described as useful in treatment of endotoxic shock in WO92/04034, published March 19, 1992. Inhibition of ICAM-1 dependent inflammatory responses with anti-ICAM-1 anti-idiotypic antibodies and antibody fragments is addressed in WO92/06119, published April 16, 1992.

Despite the fundamental insights into cell adhesion phenomena which have been gained by the identification and characterization of intercellular adhesion proteins such as ICAM-1 and lymphocyte interactive integrins such as LFA-1, the picture is far from complete. It is generally believed that numerous other proteins are involved in inflammatory processes and in targeted lymphocyte movement throughout the body. For example, published PCT Application WO 93/14776 (published August 5, 1993) discloses the cloning and expression of an ICAM-Related protein, ICAM-R. The DNA and amino acid sequences of ICAM-R are set out in SEQ ID NO. 4 herein. This new ligand has been found to be expressed on human lymphocytes, monocytes and granulocytes.

Of particular interest to the present application, still another ICAM-like surface molecule was identified which has a tissue specific expression unlike that of any known ICAM molecule. Mori, *et al*., [*Proc.Natl.Acad.Sci.(USA) 84*:3921-3925 (1987)] reported identification of a telencephalon-specific antigen in rabbit brain, specifically immunoreactive with monoclonal antibody 271A6. This surface antigen was named telencephalin. Imamura, *et al*., [*Neurosci.Letts. 119*:118-121 (1990)], using a polyclonal antibody to assess localized expression, asserted that expression of telencephalin in visual cortex of cats showed variation in layers of the tissue, and also reported telencephalin expression was variable as a function of development. Oka, *et al*., *[Neuroscience 35*:93-103 (1990)] subsequently reported isolation of telencephalin using monoclonal antibody 271A6. The publication reports a molecular weight for the surface molecule of about 500 kD and that the molecule was composed of four subunits, each with a native molecular weight of 130 kD and approximately 100 kD following *N*-glycanase treatment. Yoshihiro, *et al.,* [*Neuroscience*, Research Supplement 18, p. S83 (1994)], reported the cDNA and amino acid sequences for rabbit telencephalin at the 17th Annual Meeting of the Japan Neuroscience Society in Nagoya, Japan, December 7-9, 1993, and the 23rd Annual Meeting of the Society for Neuroscience in Washington, D.C., November 9, 1993 [Society for Neuroscience Abstracts 19 (1-3) p. 646 (1993)]. The deduced amino acid sequence reported suggested that the 130 kD telencephalon is an integral membrane protein with nine extracellular immunoglobulin (Ig)-like domains. The distal eight of these domains showed homology to other ICAM Ig-like domains. This same information was reported by Yoshihara, *et al*., in *Neuron 12*:543-553 (1994).

PCT Application WO 96/40916 (published 19 December 1996) discloses ICAM-4 polynucleotides and polypeptides, as well as a method of screening for neuropathology by detecting and quantitating ICAM-4 in a body fluid.

There thus continues to be a need in the art for the discovery of additional proteins participating in human cell-cell interactions and especially a need for information serving to specifically identify and characterize such proteins in terms of their amino acid sequence. Moreover, to the extent that such molecules might form the basis for the development of therapeutic and diagnostic agents, it is essential that the DNA encoding them be elucidated. Such seminal information would *inter alia*, provide for the large scale production of the proteins, allow for the identification of cells naturally producing them, and permit the preparation of antibody substances or other novel binding proteins specifically reactive therewith and/or inhibitory of ligand/receptor binding reactions in which they are involved.

### BRIEF SUMMARY OF THE INVENTION

Disclosed herein are purified and isolated polynucleotides (e.g., DNA sequences, RNA transcripts and anti-sense oligonucleotides thereof) encoding a novel polypeptide, "ICAM-4," as well as polypeptide variants (including fragments and deletion, substitution, and addition analogs) thereof which display one or more ligand/receptor binding biological activities and/or immunological properties specific to ICAM-4. ICAM-4-specific ligand/receptor binding biological activities encompass interactions of both the ICAM-4 extracellular and cytoplasmic domains with other molecules (e.g., in processes of cell-cell adhesion and/or signal transduction). Preferred DNA sequences include genomic and cDNA sequences as well as wholly or partially chemically synthesized DNA sequences. A presently preferred polynucleotide is set out in SEQ ID NO: 1 and encodes rat species ICAM-4. Biological replicas (i.e., copies of isolated DNA sequences made *in vivo* or *in vitro*) of the disclosed DNA sequences are contemplated. Also disclosed are autonomously replicating recombinant constructions such as plasmid and viral DNA vectors incorporating ICAM-4 sequences and especially vectors wherein DNA encoding ICAM-4 or an ICAM-4 variant is operatively linked to an endogenous or exogenous expression control DNA sequence.

According to another aspect, host cells, especially unicellular host cells such as procaryotic and eucaryotic cells, are stably transformed with the disclosed DNA sequences in a manner allowing the desired polypeptides to be expressed therein. Host cells expressing such ICAM-4 and ICAM-4 variant products can serve a variety of useful purposes. To the extent that the expressed products are "displayed" on host cell surfaces, the cells may constitute a valuable immunogen for the development of antibody substances specifically immunoreactive with ICAM-4 and ICAM-4 variants. Host cells are conspicuously useful in methods for the large scale production of ICAM-4 and ICAM-4 variants wherein the cells are grown in a suitable culture medium and the desired polypeptide products are isolated from the cells or from the medium in which the cells are grown.

Novel ICAM-4 described herein may be obtained as isolates from natural cell sources, but, along with ICAM-4 variant products, are preferably produced by recombinant procedures involving host cells. A presently preferred amino acid sequence for an ICAM-4 polypeptide is set out in SEQ ID NO: 2. The products may be obtained in fully or partially glycosylated, partially or wholly de-glycosylated, or non-glycosylated forms, depending on the host cell selected for recombinant production and/or post-isolation processing. ICAM-4 variants may comprise water soluble or insoluble monomeric, multimeric or cyclic ICAM-4 fragments which include all or part of one or more of the domain regions specified above and having a biological or immunological property of ICAM-4 including, *e.g*., the ability to bind to a binding partner of ICAM-4 and/or inhibit binding of ICAM-4 to a natural binding partner. ICAM-4 variants may also comprise polypeptide analogs wherein one or more of the specified amino acids is deleted or replaced: (1) without loss, and preferably with enhancement, of one or more biological activities or immunological characteristics specific for ICAM-4; or (2) with specific disablement of a particular ligand/receptor binding function. Analog polypeptides including additional amino acid (*e. g.*, lysine or cysteine) residues that facilitate multimer formation are contemplated.

Also comprehended are antibody substances (*e.g*., monoclonal and polyclonal antibodies, antibody fragments, single chain antibodies, chimeric antibodies, CDR-grafted antibodies and the like) and other binding proteins (*e.g.*, polypeptides and peptides) which are specific (*i.e*., non-reactive with the ICAM-1, ICAM-2, and ICAM-R intercellular adhesion molecules to which ICAM-4 is structurally related) for ICAM-4 or ICAM-4 variants. Also comprehended are hybridoma cell lines which specifically secrete the disclosed monoclonal antibodies. Presently preferred hybridomas include those designated 127A, 127H, 173E, 179I, and 179H. Antibody substances can be developed using isolated natural or recombinant ICAM-4 or ICAM-4 variants or cells expressing such products on their surfaces. Binding proteins are additionally useful for characterization of binding site structure(s) (*e.g.,* epitopes and/or sensitivity of binding properties to modifications in ICAM-4 amino acid sequence).

Binding proteins are useful, in turn, in compositions for immunization as well as for purifying polypeptides and identifying cells displaying the polypeptides on their surfaces. They are also manifestly useful in modulating (i.e., blocking, inhibiting or stimulating) ligand/receptor binding biological activities involving ICAM-4, especially those ICAM-4 effector functions involved in specific and non-specific immune system, responses.. Anti-idiotypic antibodies specific for anti-ICAM-4 antibody substances and uses of such anti-idiotypic antibody substances in modulating immune responses are also contemplated. The invention further discloses methods of screening for neuropathology in an individual comprising the steps of: a) obtaining a fluid sample from the individual; b) contacting the sample with an antibody specifically immunoreactive with ICAM-4; c) quantitating the level of ICAM-4/antibody binding in the sample; and d) comparing the level of ICAM-4/antibody binding in the sample to the level of ICAM-4/antibody binding in individuals (controls) known to be free of the neuropathology. Assays for the detection and quantification of ICAM-4 on cell surfaces and in body fluids, such as serum or cerebrospinal fluid, may involve, for example, a single antibody substance or multiple antibody substances in a "sandwich" assay format. In detecting ICAM-4 in a body fluid, antibodies are also useful for assessing the occurrence of neuropathologies which can be correlated to increased levels of circulating ICAM-4. Such neuropathologies include, but are not limited to, cerebral ischemia (*i.e*., stroke) resulting from various disorders including, for example, thrombosis, embolism, cerebral aneurysmal hemorrhage, vasospasm, and the like. Quantitation of circulating ICAM-4 can also distinguish between various forms of epilepsy and may also permit determination of the stage of AIDS progression. Still other neurodegenerative disorders for which measurement of circulating ICAM-4 can be useful for diagnosis include various forms of Alzheimer's disease and other cortical dementias (such as Pick's disease, diffuse cortical Lewy body disease, and frontal lobe degeneracy), subcortical dementias (including Parkinson's disease, Huntington's disease, and progressive supranuclear), a number of the primary psychiatric disorders (such as depression, schizophrenia and psychosis), as well as nongenetic dementias arising from, for example, infections, vasculitis, metabolic and nutritional disorders (*e.g*., thyroid, vitamin B12 deficiency), vascular disorders (multiple infarct, lacunar state, Binswanger's disease), toxic encephalopathies (*e.g*., exposure to carbon monoxide, heavy metals or other industrial pollutants) and tumors.

The scientific value of the information contributed through the disclosures of DNA and amino acid sequences of ICAM-4 is manifest. As one series of examples, knowledge of the sequence of a cDNA for ICAM-4 makes possible the isolation by DNA/DNA hybridization of genomic DNA sequences encoding ICAM-4 and specifying ICAM-4 expression control regulatory sequences such as promoters, operators and the like. DNA/DNA hybridization procedures carried out with the disclosed DNA sequences under stringent conditions are likewise expected to allow the isolation of DNAs encoding allelic variants of ICAM-4, other structurally related proteins sharing one or more of the biological and/or immunological properties specific to ICAM-4, and proteins homologous to ICAM-4 from other species. DNAs are useful in DNA/RNA hybridization assays to detect the capacity of cells to synthesize ICAM-4. Also made available by this disclosure are anti-sense polynucleotides relevant to regulating expression of ICAM-4 by those cells which ordinarily express the same. As another series of examples, knowledge of the DNA and amino acid sequences of ICAM-4 makes possible the generation by recombinant means of ICAM-4 variants such as hybrid fusion proteins (sometimes referred to as "immuno-adhesions") characterized by the presence of ICAM-4 protein sequences and immunoglobulin heavy chain constant regions and/or hinge regions. See, Capon *et al., Nature, 337*: 523-531 (1989); Ashkenazi *et al., P.N.A.S. (USA), 88*: 10535-10539 (1991); and PCT WO 89/02922, published April 6, 1989. ICAM-4 variant fusion proteins may also include, for example, selected extracellular domains of ICAM-4 and portions of other cell adhesion molecules.

DNA disclosed herein also permits identification of untranslated DNA sequences which specifically promote expression of polynucleotides operatively linked to the promoter regions. Identification and use of such promoter sequences are particularly desirable in instances, for example gene transfer, which can specifically require heterologous gene expression in a limited neuronal environment. Also comprehended are vectors comprising the disclosed promoters, as well as chimeric gene constructs wherein the promoter is operatively linked to a heterologous polynucleotide sequence and a transcription termination signal.

The DNA and amino acid sequence information provided by the present disclosure also makes possible the systematic analysis of the structure and function of ICAM-4 and definition of those molecules with which it will interact on extracellular and intracellular levels. The idiotypes of anti-ICAM-4 monoclonal antibodies are representative of such molecules and may mimic natural binding proteins (peptides and polypeptides) through which ICAM-4 intercellular and intracellular activities are modulated or by which ICAM-4 modulates intercellular and intracellular events. Alternately, they may represent new classes of modulators of ICAM-4 activities. Anti-idiotypic antibodies, in turn, may represent new classes of biologically active ICAM-4 equivalents. *In vitro* assays for identifying antibodies or other compounds that modulate the activity of ICAM-4 may involve, for example, immobilizing ICAM-4 or a natural ligand to which ICAM-4 binds, detectably labelling the nonimmobilized binding partner, incubating the binding partners together and determining the effect of a test compound on the amount of label bound wherein a reduction in the label bound in the presence of the test compound compared to the amount of label bound in the absence of the test compound indicates that the test agent is an inhibitor of ICAM-4 binding.

The DNA sequence information disclosed herein also makes possible the development, by homologous recombination or "knockout" strategies [see, *e.g*., Kapecchi, *Science, 244:* 1288-1292 (1989)], of rodents that fail to express a functional ICAM-4 protein or that express a variant ICAM-4 protein. Such rodents are useful as models for studying the activities of ICAM-4 and ICAM-4 modulators *in vivo.*

According to the present invention, there is provided a method of distinguishing between various forms of epilepsy comprising the steps of: a) contacting a fluid sample obtained from a first individual with an antibody specifically immunoreactive with ICAM-4, b) quantitating the level of ICAM-4/antibody binding in the sample; and c) comparing the level of ICAM-4/antibody binding in the sample to the level of ICAM-4/antibody binding in a second individual having a known form of epilepsy selected from Grand Mal Seizures, Syncope, or temporal lobe epilepsy (TLE).

### DETAILED DESCRIPTION OF THE INVENTION

Relevant to are disclosure of the present application are the design and construction of oligonucleotide probes for PCR amplification of ICAM related DNAs; use of the probes to amplify a human genomic fragment homologous to, but distinct from DNAs encoding ICAM-1 and ICAM-2; screening of cDNA libraries with the genomic fragment to isolate additional ICAM-R coding sequences; screening of cDNA libraries to isolate a full length human cDNA sequence encoding ICAM-R; characterization of DNA and amino acid sequence information for ICAM-R, especially as related to ICAM-1 and ICAM-2; development of mammalian host cells expressing ICAM-R; assessment of indications of ICAM-R participation in adhesion events involving CD18-dependent and CD18-independent pathways; inhibition of cell adhesion to ICAM-R by ICAM-R-derived peptides; expression of variants of ICAM-R; preparation and characterization of anti-ICAM-R antibodies and fragments thereof; mapping of ICAM-R epitopes recognized by anti-ICAM-R monoclonal antibodies; assessment of the distribution and biochemical characterization of ICAM-R and RNA encoding the same; assessment of ICAM-R in homotypic cell-cell adhesion and immune cell activation/proliferation; characterization of ICAM-R monoclonal antibodies; and assessment of differential phosphorylation and cytoskeletal associations of the cytoplasmic domain of ICAM-R. Also disclosed was the identification of a rodent ICAM-encoding DNA that, at the time, appeared to be the rat homolog of human ICAM-R, and the use of this DNA to construct and express DNAs encoding glutathione-S-transferase fusion proteins. The detailed description of how this rodent DNA was identified is reproduced herein as Example 1. As more of the rodent ICAM-coding sequence was identified, it became apparent that the rodent ICAM DNA did not encode a rat species homolog of human ICAM-R, but, in fact, encoded a novel ICAM polypeptide, herein named ICAM-4. In order to appreciate the events which led to the identification of ICAM-4, a chronology is provided which is followed by a detailed description of the invention.

A first rodent genomic ICAM-4 sequence was identified which encoded a region homologous to domain 2 (herein SEQ ID NO: 3) of human ICAM-R (herein as SEQ ID NO: 4). A second, overlapping genomic DNA (herein SEQ ID NO: 5) was also identified which encoded both the domain 2 region of SEQ ID NO: 3, and sequences for ICAM-1. Using SEQ ID NO: 3 as a probe, a rodent spleen cDNA (herein SEQ ID NO: 6) was identified which encoded domains 2 through 5 as well as a fifth domain not previously observed as an ICAM domain. At this time, these newly identified rodent DNAs appeared to encode a rodent homolog of human ICAM-R, however alignment of 3' regions of these DNAs with other ICAMs proved difficult.

The subsequent isolation of a 1 kb cDNA clone from a rat spleen library, and amplification of an RT-PCR fragment indicated that a portion of both the cDNA and genomic clones had not been sequenced. Another RT-PCR amplification product (SEQ ID NO: 7) confirmed this omission. It was determined that a fragment of 177 bp was excised from the genomic and cDNA clones by *Eco*RI digestion *of* the clones to isolate these sequences from λ phage for DNA sequencing studies. Reanalysis of SEQ ID NOs: 5 and 6 in light of these other sequences permitted identification of more accurate and complete sequences for the originally isolated genomic and cDNA clones, presented in corrected form herein as SEQ ID NOs: 8 and 9.

In order to identify a complete coding sequence for ICAM-4, a rat brain cDNA (SEQ ID NO: 10) was isolated, and 5' end sequence determined by 5' rapid amplification of cDNA ends (5' RACE), the amplification product set forth in SEQ ID NO: 11. Combining information from the RT-PCR clone (SEQ ID NO: 7), the brain cDNA (SEQ ID NO: 10) and the RACE amplification product (SEQ ID NO: 11) permitted identification of the complete coding sequence for ICAM-4 (SEQ ID NO: 1).

The present invention is thus illustrated by the following examples. More particularly, Example 1 addresses cloning of a partial rodent ICAM-4 DNA. Example 2 describes Northern blot analysis of rodent ICAM-4 transcription. Example 3 describes isolation of a full length rodent ICAM-4 cDNA. Example 4 relates the *in situ* hybridization of rodent ICAM-4 in brain tissue. Example 5 addresses generation of ICAM-4 fusion proteins in prokaryotes. Example 6 describes production of monoclonal antibodies specific for rat ICAM-4/GST fusion proteins. Example 7 describes expression of soluble rat ICAM-4 proteins in a baculovirus expression system. Example 8 addresses production of monoclonal antibodies specific for rat ICAM-4 expressed in a baculovirus system. Example 9 describes immunocytochemical analysis of rat ICAM-4 expression. Example 10 relates cloning of a human genomic ICAM-4-encoding DNA. Example 11 addresses cloning of a human ICAM-4-encoding cDNA. Example 12 describes Northern analysis of human ICAM-4 expression. Example 13 describes generation of human ICAM-4/GST fusion proteins. Example 14 addresses production of monoclonal antibodies immunospecific for human ICAM-4. Example 15 describes development of a capture assay for determining the concentration of soluble ICAM-4 in a particular fluid. Example 16 applies the capture assay method in assessing ICAM-4 concentration in the serum of stroke patients. Example 17 relates to assessment of ICAM-4 transcription in a rat epilepsy model. Example 18 describes measurement of circulating ICAM-4 concentration as an assessment of various neurodegenerative disorders. Example 19 addresses cloning of a promoter region for human ICAM-4.

### EXAMPLE 1

### Cloning of Rat ICAM-Related DNA

### A. Isolation of a Rat Genomic ICAM-Related Domain 2 DNA

A rat genomic library constructed in λ EMBL3 was screened a with [³²P]-labeled probe generated by PCR from DNA encoding human ICAM-3 domain 2 The sequence of the probe is set forth in SEQ ID NO: 12. Library plaques were transferred to Hybond N+ nylon membranes (Amersham, Arlington Heights, IL). Screening of all cDNA and genomic libraries was performed according to standard protocols. Prehybridization and hybridizations were carried out in a solution of 40-50% formamide, 5X Denhardt's, 5X SSPE and 1.0% SDS at 42°C. Probes ([³²P]-labeled) were added at a concentration of 10⁵-10⁶ cpm/ml of hybridization solution. Following 16-18 hours of hybridization, nylon membranes were washed extensively at room temperature in 2X SSPE with 0.1 % SDS and subsequently exposed to X-ray film at -80°C overnight. Positive plaques were subjected to one or more rounds of hybridization to obtain clonal phage. DNA prepared from lysate of the positive clones was subcloned into pBS+ and sequenced.

A first genomic clone encoding a rat ICAM-related domain 2 was identified that was determined to be homologous to domain 2 regions in other ICAM family members, yet was distinct from the previously reported nucleotide sequences for rat ICAM-1 [Kits, *et al., Biochem.Biophys.-Acta 1131*:108-110 (1992)] or mouse ICAM-2 [Xu, *et al., J.Immunol. 149*:2560-2565 (1992)]. The nucleic acid and deduced amino acid sequences for this clone were disclosed as purportedly variant forms of rat ICAM-R and are set out in SEQ ID NOs: 3 and 13, herein, respectively.

A second, overlapping clone was also identified with the same probes and was determined to contain the ICAM domain 2 sequence of SEQ ID NO: 3 and 5' DNA encoding at least part of rat ICAM-1. The nucleic acid sequence for this clone is set forth herein as SEQ ID NO: 5. This second clone indicated that the ICAM-related gene fragment of the first clone and the gene encoding rat ICAM-1 are located on the same rat chromosome within 5 kb of each other.

### B. Isolation of Rat ICAM-Related cDNA

In order to identify a more complete protein coding sequence for the ICAM-related polypeptide, [³²P]-labeled DNA encoding the domain 2 sequence from the rat genomic clone identified in Section A (SEQ ID NO: *3*)*, supra,* was used to screen a number of cDNA libraries from various rat and mouse cell types, including rat macrophage (Clontech, Palo Alto, CA), peripheral blood lymphocyte (PBL) (Clontech), T cell (constructed in-house), and spleen (Clontech), and mouse PBL (Clontech), T cell (constructed in-house), and B cell (constructed in-house).

A single clone was identified in a rat spleen cDNA library (Clontech) which contained five Ig-like domains, four of which were homologous to domains 2 through 5 in both ICAM-1 and ICAM-R. Moreover, this clone included 3' DNA encoding an apparent fifth Ig-like domain which had not been previously identified in any other ICAM polypeptide. In addition, the clone contained an unusual 3' sequence subsequently determined to be a partial intron (discussed *infra*) located between domains 4 and 5, suggesting that the clone was the product of an immature or aberrantly spliced transcript. The presence of the unique domain and the determination that the 3' region did not properly align with other known ICAMs suggested that the ICAM-related DNA potentially encoded a novel rat ICAM polypeptide. The nucleic acid sequence for this spleen cDNA clone is set forth in SEQ ID NO: 6.

### C. Re-analysis of Rat cDNA and Genomic DNAs

Subsequently, it was determined that the partial rat spleen cDNA clone (SEQ ID NO: 6 herein) and the rat liver genomic clone (SEQ ID NO: 5 herein) were missing an internal 177 bp EcoRI fragment that was part of each of these clones but lost in a subcloning step when the library inserts were removed from the λ vector with *Eco*RI digestion and ligated into a sequencing vector. The observation that the cDNA and genomic clones might be missing a coding fragment became apparent upon alignment of the rat genomic and cDNA sequences with various RT-PCR amplification products, including SEQ ID NO: 7, which revealed a gap in the rat sequence.

Subsequent isolation and sequence alignment of a cDNA from a spleen library using the spleen cDNA clone (SEQ ID NO: 6) as a probe provided a first indication that a portion of the spleen cDNA and genomic clones were not sequenced. Further confirmation of this idea became apparent upon amplification of an RT-PCR fragment, spanning domains 3 through 5, using a 5' primer (RRD3 5 'Xho, containing a 5' *Xho*I restriction site to facilitate cloning) set out in SEQ ID NO: 14, and a 3' primer (RRD5 3 'Hind, containing a *Hind*III site to facilitate cloning) set out in SEQ ID NO: 15. Alignment of these two DNAs clearly revealed that the cDNA and genomic clones had lost a fragment prior to sequencing; this idea was further supported following sequencing of the RT-PCR DNA discussed *infra*. It was concluded that restriction digestion with *Eco*RI to remove the cDNA and genomic fragments prior to sequencing resulted in the excision of a 177 bp fragment that was not detected visually in the agarose gel separation of the clones from the λ phage sequences. Subsequent sequence analysis confirmed the location of two *Eco*RI sites flanking a 177 bp fragment in both of the original clones.

The 177 bp *Eco*RI fragment is situated between nucleotides 719 and 896 in the rat partial cDNA clone as set out in SEQ ID NO: 9 and between nucleotides 2812 and 2989 in the partial genomic clone as set out in SEQ ID NO: 8.

### D. DNA Isolated by RT-PCR Clone

RT-PCR was utilized to generate more complete sequence information for the rat ICAM-related gene. Sequence information from the genomic clone (SEQ ID NO: 3) was used to design sense primers complementary to a region 5' of the protein coding region, as determined from the cDNA clone, and antisense primers designed complementary to coding sequences and regions 3' to the coding sequence in the cDNA clone (SEQ ID NO: 6).

Template cDNA for PCR reactions was prepared as follows. Approximately 2 µg of poly A⁺RNA isolated from rat spleen cells was denatured by heating at 65°C in a 10 µl volume. Following denaturation, 0.1 µl RNasin (Invitrogen, San Diego, CA), 5 µl 5X RTase Buffer (BRL, Bethesda, MD), 2 µl random hexamer (pd(N)6 at 100 µg/ml) (Pharmacia, Piscataway, NJ), 6 µl dNTPs (2 mM each) and 2 µl AMV RTase (BRL) were added and the reaction was incubated at 42°C for 60-90 min. Reactions were stored at -20°C until needed.

An initial series of experiments was conducted to identify oligonucleotides primer pairs that produced an amplification product in PCR reactions using rat spleen cDNA as the template. Various 5' sense primers were paired in PCR with a 3' primer which was designed to be complementary to an internal, coding sequence; the 3' primer was designated RRD2 3-1 and is set forth in SEQ ID NO: 16. (In the ultimately isolated RT-PCR product, SEQ ID NO: 7, *infra*, primer RRD2 3-1 corresponded to nucleotides 719 through 736.) Similarly, various 3' antisense primers were paired with a 5' primer designed complementary to another internal, coding sequence; the 5' primer in these reactions was designated RGen3900S and is set forth in SEQ ID NO: 17. (In SEQ ID NO: 7, *infra,* primer RGen3900S corresponded to nucleotides 1719 through 1736.) Based on the size of the amplification products and the ability of these products to hybridize with the partial cDNA clone, one pair of primers was determined to be most efficient and was used in subsequent PCR amplifications. The 5' primer was designated RGen780S (SEQ ID NO: 18) and the 3' primer was designated RGen4550AS (SEQ ID NO: 19). (In SEQ ID NO: 7, *infra,* primer RGen780S corresponded to nucleotides 1 through 18, and primer RGen4550AS corresponded to nucleotides 2197 through 2214.)

This primer pair was used in PCR under a variety of conditions to optimize amplification. A total of 15 different PCR buffers that varied in pH and Mg⁺⁺ concentration were used at two different annealing temperatures, and a sample of the product from each reaction was separated on a 1% agarose gel. Because no amplification product could be detected by visual inspection of the ethidium bromide stained gel from any of the reaction conditions, more sensitive Southern hybridization was employed to detect the PCR products.

Aliquots of the amplified DNA were separated by electrophoresis, transferred to a Hybond N+ nylon membrane using conventional Southern blotting wicking techniques, and hybridized with the entire rat cDNA which was [³²P]-labeled. Hybridization conditions were essentially as described for the library screening procedure in Section A, *supra*. Autoradiography indicated that a small amount of DNA of approximately 2.2 kb had been generated in two of the reactions, and the remainder of the amplification product from the two reactions was separated on an agarose gel. The 2.2 kb region was eluted from the gel, even though no band was evident upon visual inspection, and used as a template in another PCR reaction using the same primers (SEQ ID NOs: 18 and 19), Tris-HCl buffer, pH 8.0, containing 1 mM Mg⁺⁺, and 55°C annealing temperature. The amplification product from the secondary PCR was visible in the gel and was eluted and cloned into a pBS⁺ plasmid (Stratagene, La Jolla, CA) for sequence analysis.

The resulting RT-PCR clone was determined to contain 2214 bp as set forth in SEQ ID NO: 7. The clone encoded domains 2 through 6 found in the rat spleen cDNA clone, an additional amino terminal domain 1, an additional carboxy terminal domain 7, and 164 bp of what appeared to be a further carboxy terminal domain 8. Immediately 5' to domain 1 was an additional 144 bp sequence presumed to have been derived from an intron between the leader and the first domain. This clone did not contain a 5' leader sequence or 3' transmembrane and cytoplasmic regions. In addition to the previously identified domain 6 in the spleen cDNA clone, the 7th and 8th domains in the RT-PCR clone supported the hypothesis that this clone was a novel rodent ICAM.

### EXAMPLE 2

### Northern Blot Analysis

In order to further investigate the possibility that the ICAM-related clones identified in Example 1 encoded a novel ICAM polypeptide as suggested by the unique Ig-like domains, tissue specific expression was examined by Northern blot analysis to permit comparison with the previously reported expression patterns of human ICAMs [ICAM-1, Dustin, *et al*., *J.Immunol. 137*:245-254 (1986); ICAM-2, Staunton, *et al., Nature 339*:61-64 (1989); ICAM-R, de Fourgerolles and Springer, *J.Exp.Med. 175*:185-190 (1992)].

Total cellular RNA from rat lung, brain, spinal cord, liver, digestive tract, thymus, lymph nodes, and spleen was prepared using STAT60 RNA isolation reagents (Tel-test "B", Inc, Friendswood, Texas) according to the manufacturer's suggested protocol. Poly A⁺ RNA was purified from total RNA using oligo dT cellulose columns. Approximately 5 µg of RNA derived from each tissue was separated on a 1 % formaldehyde agarose gel, and transferred to hybond-C nitrocellulose membranes (Amersham).

A fragment of the rat spleen cDNA from Example 1 corresponding to domains 2 through 4 (nucleotides 1 through 724 in SEQ ID NO: 6) was subcloned into pBluescript SK⁺ (Stratagene) and an antisense riboprobe was generated by *in vitro* transcription using ³²P-labeled UTP and approximately 500 ng of linearized template according to a manufacturer's (Boehringer Mannheim, Indianapolis, IN) suggested protocol. The membrane-bound RNA was prehybridized in a solution containing 50% formamide, 5X SSC, 1X PE (50 mM Tris-HCl, pH 7.5, 0.1% sodium pyrophosphate, 0.2% polyvinylpyrrolidone, 0.2% ficoll, 5 mM EDTA, 1% SDS) and 150 µg/ml denatured salmon sperm DNA. The radiolabeled probe was denatured by boiling and added to the prehybridization solution to a final concentration of 1 x 10⁶ cpm/ml. Hybridization was allowed to proceed for 16-18 hours at 65°C. The membranes were then washed at 65°C in 2X SSC containing 0.1 % SDS and subsequently exposed to X-ray film for 3-16 hours.

The Northern blot analysis indicated that the ICAM-related cDNA identified in Example 1 was expressed only in rat brain, a tissue specificity not previously reported for any other ICAM polypeptides. This expression pattern, in combination with the unique Ig-like domains not known to exist in other ICAM polypeptides, indicated that the ICAM-related clone was a novel member of the ICAM family of proteins, and was named ICAM-4.

The fact that the initially identified cDNA clones were detected in a rat spleen library suggested that a subset of cells in the spleen may express ICAM-4 at low levels. However, a properly spliced clone could not be detected in numerous hemopoietic cDNA libraries which led to doubt if ICAM-4 protein is actually expressed in tissue other than brain. One explanation for the detection of ICAM-4 cDNA in spleen is that the sensitivity of PCR may have amplified a trace amount of transcript even though these tissues do not express the encoded protein.

### EXAMPLE 3

### Isolation of Full Length Rat ICAM-4 cDNA

### A. Identification of a Rat Brain cDNA Clone

In view of the tissue specific expression of ICAM-4, brain tissue mRNA was utilized in an attempt to isolate a full length cDNA encoding ICAM-4. Two probes, one complementary to domains 1 through 2 and a second complementary to domains 3 through 5 of the spleen cDNA clone identified in Example 1 (SEQ ID NO: 7), were radiolabeled and used to screen a rat brain cDNA library in λgt10 which was previously constructed in-house. Hybridization conditions were as described in Example 1, and positive plaques were subjected to one or more rounds of screening to obtain clonal phage.

Nine positive clones were identified, two of which hybridized to both probes. The longest of the two clones, designated clone 7, contained 2550 bp encoding four of the five Ig-like domains found in the probe cDNA. In addition, clone 7 encoded four other Ig-like domains not found in the probe. Putative transmembrane and cytoplasmic domains were identified which were followed by a stop codon, a poly-adenylation signal, and a poly A tail. Clone 7 was lacking at least one 5' Ig-like domain as determined by comparison to the RT-PCR clone (SEQ ID NO: 7), and also lacked a leader sequence; re-screening of the library did not yield any longer clones which contained these sequences. The nucleic acid sequence for clone 7 is set forth in SEQ ID NO: 10.

### B. Determination of the 5' End

In order to isolate domain 1 and other 5' sequences, a PCR technique termed 5' Rapid Amplification of cDNA Ends (RACE) [PCR Protocols: A Guide to Methods and Applications, Innis, et al., (eds) Academic Press: New York (1990) pp:28-38] was employed using a 5' RACE kit (Clontech). This technique utilizes an internal primer paired with a second primer complementary to an adapter sequence ligated to the 5' end of cDNA library molecules. PCR with this primer pair will therefore amplify and facilitate identification of the intervening sequences. Overlapping sequence information can then be used to generate a complete sequence of the gene.

RACE-ready cDNA from rat brain (supplied with kit) was used in a PCR with the kit oligonucleotide and an antisense primer based on an internal ICAM-4 sequence. The 3' antisense primer, designated Spot714AS, was designed according to an ICAM-4 domain 4 sequence and is set forth in SEQ ID NO: 20. The amplification product resulting from this primer pair was subsequently subjected to a secondary PCR using the same 5' kit primer paired with a 3' primer complementary to a region in ICAM-4 domain 1. The second 3' primer was designated RRACE2 and is set forth in SEQ ID NO: 21. Each primer used in the secondary PCR contained an *Eco*R1 site to facilitate cloning of the resulting amplification products into pBS⁺ (Stratagene). The resulting plasmid DNA which contained the 5' end of the gene was identified by hybridization to a rat ICAM-4 domains 1 and 2 probe, corresponding to nucleotides 1 through 736 in SEQ ID NO: 7. Partial sequence information for domain 1 and the hydrophobic leader was determined from the resulting amplification product.

The product from the 5' RACE method was a DNA fragment 222 bp long containing 60 bp upstream of the initiating methionine residue, an 82 bp leader sequence, and an 80 bp sequence from domain 1. The amplification product is set forth in SEQ ID NO: 11.

### C. Full Length Sequence of Rat ICAM-4

A composite clone of the full length ICAM-4 was constructed from the sequence information derived from the 5' RACE method (SEQ ID NO: 11), the RT-PCR clone (SEQ ID NO: 7) and the brain cDNA clone 7 (SEQ ID NO: 10). The full length gene for rat ICAM-4 was determined to contain 2985 bp with a single open reading frame encoding a deduced 917 amino acid protein. A putative Kozak sequence is located upstream of the methionine residue in the leader sequence. A 27 amino acid hydrophobic leader sequence is followed by nine Ig-like domains, a transmembrane region and a 58 amino acid cytoplasmic tail. The composite ICAM-4 cDNA is set for in SEQ ID NO: 1, and the deduced amino acid sequence is set forth in SEQ ID NO: 2.

Like other ICAM polypeptides, ICAM-4 contains extracellular, transmembrane, and cytoplasmic domains. In the extracellular domain, the amino terminus of ICAM-4 is a leader sequence comprising amino acids 1 through 27 which is followed by nine immunoglobulin (Ig)-like domains, a characteristic unique to ICAM-4 in that ICAM-1, ICAM-2, and ICAM-R contain five, two, and five extracellular Ig-like domain, respectively. In ICAM-4, domain 1 comprises amino acids 28 through 118; domain 2 comprises amino acids 119 through 224; domain 3 comprises amino acids 225 through 321; domain 4 comprises amino acids 322 through 405; domain 5 comprises amino acids 406 through 488; domain 6 comprises amino acids 489 through 569; domain 7 comprises amino acids 570 through 662; domain 8 comprises amino acids 663 through 742: and domain 9 comprises amino acids 743 through 830. Within each domain, a characteristic "loop" structure is formed by a disulfide bond between cysteine residues located generally at opposite ends of the domain amino acid sequence. Other structural features of ICAM-4 include the transmembrane region comprising amino acids 831 through 859 and the cytoplasmic region comprising amino acids 860 through 917.

Comparison of amino acid sequence homology of each domain in rat ICAM-4 with the other members of the ICAM family was limited to the corresponding sequences of human ICAM-1, ICAM-2, and ICAM-R since sequence information for all three rodent homologs has not been previously reported. In the first domain, the rodent ICAM-4 shows 21, 30, and 28 percent identity with human ICAM-1, ICAM-2, and ICAM-R, respectively. The second domain is more conserved, with the amino acid percent identities being 60, 42 and 62 with ICAM-1, -2, and -3, respectively. Domains 3-5 show percent identities of 48, 49, and 40 with ICAM-1 and 60, 59 and 29 respectively for ICAM-R. Interestingly, rat ICAM-4 domains 6 through 8 are most homologous with domain 5 (ranging from 29-42% identical), possibly arising from a gene segment duplication event. The ninth and final extracellular domain aligns poorly with other ICAM domains but has 22 % identity with the 3rd and 6th domains of human VCAM-1, another member of the Ig family of protein which participate in cell adhesion. The cytoplasmic tail is 58 amino acids long. This is longer than the other members of the ICAM family wherein human ICAM-1, -2, and -3 contain 28, 26, and 37 amino acids,respectively. As with the ninth domain, rat ICAM-4 cytoplasmic tail is most homologous with the cytoplasmic tail of human VCAM-1, which contains only 19 amino acids. The membrane proximal 19 amino acids of rat ICAM-4 share 7 amino acid residues with VCAM-1 (37%).

Finally, functional binding to LFA-1 (CD11a/CD18) maps to the first domain in the ICAMs. Vonderheide *et al*., [*J. Cell. Biol*., *125:215*-222 (1994)] identified a sequence motif purportedly involved in integrin binding. Despite the relatively low homology between rat ICAM-4 and other ICAMs in domain 1, this binding sequence motif is conserved, suggesting that rat ICAM-4 may be a ligand for LFA- I and perhaps other integrins.

### EXAMPLE 4

### In situ Hybridization in Brain Tissue

In order to localize the specific brain tissue which expressed ICAM-4, *in situ* hybridization with ICAM-4 domain 1 and ICAM-4 domains 3 through 4 anti-sense riboprobes was employed. The probes were labeled by *in vitro* transcription using ³⁵S-labeled UTP.

Frozen tissue sections of normal rat brain were fixed in 4% paraformaldehyde for 20 minutes, rinsed and dehydrated, and the fixed RNA denatured for 2 minutes in 2X SSC, 70% formamide at 70°C prior to hybridization. Tissue sections were hybridized overnight at 50°C in a solution containing 50% formamide, 0.3 M NaCI, 20 mM Tris-HCl, pH 7.4, 5 mM EDTA, 10% dextran sulfate, IX Denhardt, 0.5 mg/ml yeast RNA, 100 mM DTT and a probe concentration of 50,000 cpm/µl. Slides were washed once in 4X SSC, 10 mM DTT at room temperature for 60 minutes, once in 50% formamide, 2X SSC, 10 mM DTT at 60°C for 40 minutes, and once in each 2X SSC and 1X SSC for 30 minutes each at room temperature. Specificity of hybridization was determined in parallel experiments performed with the same protocol but also including a more stringent wash in 50% formamide, 1X SSC, 10 mM DTT at 60°C for 40 minutes. After washing, the slides were dipped in NTB2 emulsion (Kodak, Rochester, NY) and exposed from 2 to 21 days before being developed and counter-stained. Negative controls included sense probes generated from ICAM-4 domain 1 and ICAM-4 domain 3 through 4 sense riboprobes, in addition to a human immunodeficiency virus (HIV-1) riboprobe.

The signal detected in brain tissue was primarily, localized in the gray matter with the strongest signal in the cerebral cortex and hippocampus. The hybridization profile was consistent with ICAM-4 expression primarily in cerebral neurons.

### EXAMPLE 5

### Generation of ICAM-4 fusion proteins

Rat ICAM-4/glutathione S-transferase (GST) fusion proteins were generated using the prokaryote expression vector pGEX (Pharmacia, Alameda, CA) in order to generate monoclonal antibodies against specific ICAM-4 polypeptide fragments.

PCR primers corresponding to the 5' and 3' ends of domain 1 and the 5' and 3' ends of domain 2 were used to amplify DNA fragments encoding the individual domains. The resulting fragments were separately cloned into an *Eco*RI site of pGEX-2T; DNA sequence analysis confirmed the correct orientation and reading frame. Transformants were subsequently screened for their ability to produce fusion protein of the appropriate molecular weight.

Both ICAM-4 domain 1/GST and ICAM-4 domain 2/GST fusion proteins remained in the insoluble fraction after the bacteria were lysed by sonication in PBS containing 1 % SDS. The insoluble protein fraction from 100 ml cultures were boiled in SDS loading dye and separated on a 10% preparative polyacrylamide-SDS gel. The gel was stained in ice cold 0.4 M KCl and the fusion protein bands were excised. Fusion proteins were electroeluted from the gel slices in dialysis tubing in buffer containing 25 mM Tris-HCl and 192 mM glycine. Approximate protein concentration was determined by OD₂₈₀ and purity of the preparation was determined on SDS-PAGE stained with Coomassie blue.

### EXAMPLE 6

### Production of Monoclonal Antibodies Against Rat ICAM-4/GST Fusion Proteins

Balb/c mice were immunized by subcutaneous injection with 40-50 µg ICAM-4 domain-2/GST fusion protein (described in Example 5) emulsified in Freund's complete adjuvant (FCA). Two weeks later, the mice were again immunized by subcutaneous injection with the same protein, emulsified however in Freund's incomplete adjuvant. Two final intraperitoneal immunizations given two weeks after the second immunization included soluble antigen with no adjuvant given at two week intervals. Serum from each immunized mouse was assayed by ELISA for its ability to specifically react with rat ICAM-4 produced by the baculovirus expression system described *infra.*

The spleen from mouse #1654 was sterilely removed and placed in 10 ml serum-free RPMI 1640. A single-cell suspension was formed by grinding the spleen tissue between frosted ends of two glass microscope slides submerged in serum free RPMI 1640 (Gibco, Burlington, Ottawa, Canada) supplemented with 2 mM L-glutamine, 1 mM sodium pyruvate, 100 units/ml penicillin, and 100 µg/ml streptomycin. The cell suspension was filtered through a sterile 70-mesh Nitex cell strainer (Becton Dickinson, Parsippany, NJ), and washed twice with RPMI followed by centrifuging at 200 x g for 5 minutes. The resulting pellet from the final wash was resuspended in 20 ml serum-free RPMI. Thymocytes taken from three naive Balb/c mice were prepared in an identical manner.

Prior to fusion, NS-1 myeloma cells were maintained in log phase growth in RPMI with 11 % Fetalclone serum (FBS) (Hyclone Laboratories, Logan, Utah) for three days. Once harvested, the cells were centrifuged at 200 x g for 5 minutes, and the pellet was washed twice as described in the foregoing paragraph. After washing, the cell suspension was brought to a final volume of 10 ml in serum free RPMI. A 20 µl aliquot was removed and diluted 1:50 with serum free RPMI, and a 20 µl aliquot of this dilution was removed, mixed with 20 µl 0.4% trypan blue stain in 0.85% saline (Gibco), loaded onto a hemacytometer (Baxter Healthcare, Deerfield, IL) and the cells counted. Approximately 2.425 x 10⁸ spleen cells were combined with 4.85 x 10⁷ NS-1 cells, the mixture centrifuged and the supernatant removed. The resulting pellet was dislodged by tapping the tube and 2 ml of 50% PEG 1500 in 75 mM Hepes, pH 8.0, (Boehringer Mannheim, Indianapolis, IN) was added with stirring over the course of 1 minute. Subsequently, an additional 14 ml serum free RPMI was added over 7 minutes. The cell suspension was centrifuged at 200 x g for 10 minutes and the supernatant discarded. The pellet was resuspended in 200 ml RPMI containing 15 % FBS, 100 µM sodium hypoxanthine, 0.4 µM aminopterin, 16 µM thymidine (HAT) (Gibco), 25 units/ml IL-6 (Boehringer Mannheim) and 1.5 x 10⁶ thymocytes/ml. The suspension was first placed in a 225 cm² flask (Corning, Essex, United Kingdom) at 37°C for four hours before being dispensed into ten 96-well flat bottom tissue culture plates (Corning) at 200 µl/well. Cells in the plates were fed on days 3, 4, 5, and 6 post fusion by aspirating approximately 100 µl from each well with a 20 G needle (Becton Dickinson), and adding 100 µl/well plating medium described above except containing 10 units/ml IL-6 and lacking thymocytes.

The fusion plates were screened initially by antigen capture ELISA as follows. Immulon 4 plates (Dynatech, Cambridge, MA) were coated overnight at 4°C with 100 ng/well of either domain 1-GST or domain 2-GST fusion protein in 50 mM carbonate buffer. The plates were blocked with 100 µl/well 0.5% fish skin gelatin (Sigma, St. Louis, MO) in PBS for 30 minutes at 37°C. After blocking, the plates were washed 3X with PBS containing 0.05 % Tween 20 (PBST) and 50 µl/well of hybridoma supernatant from each fusion was added. After incubation at 37°C for 30 minutes, the plates were washed as described above, and 50 µl of a 1:3500 dilution of horseradish peroxidase-conjugated goat anti-mouse IgG (Fc) (Jackson ImmunoResearch, West Grove, Pennsylvania) was added. Plates were again incubated for 30 minutes and washed 4X with PBST. Substrate, 100 µl/well, consisting of 1 mg/ml o-phenylene diamine (Sigma) and 0.1 µl/ml 30% H₂O₂ in 100 mM citrate, pH 4.5, was added. The color reaction was allowed to proceed 10 minutes and quenched with the addition of 50 µl/well of 15% H₂SO₄. Absorbance at 490 nm was then determined on an automated plate reader (Dynatech).

Wells which were positive for domain 2-GST protein, but not for domain 1-GST protein, were then screened by ELISA against a Baculovirus supernatant (described *infra*). ELISA was performed as described above except that the Immulon 4 plates were initially coated overnight with Baculovirus supernatant diluted 1:4 in 50 mM carbonate buffer. Three wells (103A, 103B and 103F) were cloned two to three times, successively, by doubling dilution in RPMI, 15% FBS, 100 µM sodium hypoxanthine, 16 µM thymidine, and 10 units/ml IL-6. Wells of clone plates were scored visually after 4 days and the number of colonies in the least dense wells was recorded. Selected wells of each cloning were again assayed by ELISA after 7 to 10 days against either domain 1-GST protein and domain 2-GST protein, or Baculovirus supernatant.

The monoclonal antibodies produced by the hybridomas were isotyped by ELISA. Immulon 4 plates (Dynatech) were coated at 4°C with 50 µl/well goal anti-mouse IgA, IgG, or IgM (Organon Teknika, Durham, NC) diluted 1:5000 in 50 mM carbonate buffer, pH 9.6. Wells were blocked for 30 minutes at 37°C with 1 % BSA in PBS, washed 3X with PBST. A 1:10 dilution of hybridoma culture supernatant (50 µl) was added to each plate, incubated, and washed as above. After removal of the last wash, 50 µl horseradish peroxidase-conjugated rabbit anti-mouse IgG₁, G₂ₐ, G_{2b}, or G₃ (Zymed, San Francisco, CA) (diluted 1:1000 in PBST with 1% normal goat serum) was added. Plates were incubated as above, washed 4X with PBST and 100 µl substrate, was added. The color reaction was quenched after 5 minutes with addition of 50 µl 15% H₂SO₄, and absorbance at 490 nm determined on a plate reader (Dynatech).

Results indicated that antibodies 103A, 103B, and 103F were all IgG₁ isotype. These antibodies were subsequently used in immunocytochemical analyses, Western blotting, and for purification of protein expressed in baculovirus.

### EXAMPLE 7

### Baculovirus Expression of Rat ICAM-4

A baculovirus expression system (Invitrogen) was used to generate soluble protein corresponding to domains 1 through 6 of ICAM-4. Because the leader sequence for ICAM-4 was not known at the time, the expression construct was made containing the coding sequence for ICAM-4 fused 3' to the ICAM-1 leader sequence in proper reading frame. Specific details regarding construction of the ICAM-1/ICAM-4 expression plasmid is as follows.

Rat ICAM-1 DNA encoding the five Ig-like domains was amplified by PCR using primers which incorporated several features to facilitate construction of the fusion plasmid. The 5' oligonucleotide primer included *Hind*III and *Bgl*II sites, in addition to a consensus Kozak sequence upstream of the first methionine in the leader sequence. The 3 ' oligonucleotide primer included a coding sequence for six histidines followed by a stop codon and a *Hind*III cloning site. The PCR amplification product was cloned into a *Hind*III-digested pBS⁺ vector and sequence analysis confirmed the appropriate construction. An internal *Sma*I site in the ICAM-1 leader sequence and another *Sma*I site in the vector's multiple cloning region (3' to ICAM-1 Ig-like domain 5) were digested which removed most of the ICAM-1 coding sequence. After these manipulations, the linearized, blunt-ended vector contained a portion of the upstream multiple cloning region (those restriction sites 5' of the original *Hind*III site in the multiple cloning region), the Kozak sequence and most of the ICAM-1 leader sequence.

The coding sequence for rat ICAM-4 domains 1 through 6 was amplified by PCR utilizing primers designed to permit cloning of this sequence into the linearized vector described above. The 5' oligonucleotide primer included an *Eco*RV site and the codons needed to complete the ICAM-1 leader sequence. The 3' oligonucleotide primer included codons for six histidine residues. a stop codon, and *Hind*III and *Eco*RV restriction sites. The amplification product from this PCR was digested with *Eco*RV to produce a blunt-ended sequence which was then ligated into the blunt-ended *Sma*I-digested pBS⁺ linearized vector. The entire sequence containing the ICAM-1 leader sequence 5' to the ICAM-4 domains 1 through 6 was removed from the construct with *Bgl*II and *Hind*III digestion and the purified ICAM-1/ICAM-4 fusion sequence cloned directly into a *Bgl*II/*Hind*III- digested pBluesac III vector (Invitrogen).

Protein production by the recombinant virus was assayed for by ELISA, initially using immune sera from mice immunized with rat ICAM-4 domain-2/GST fusion protein described in Example 5. In later work, monoclonal antibodies generated from those mice were used to purify ICAM-4 protein produced by the recombinant baculovirus in SF9 cells.

### EXAMPLE 8

### Production of Monoclonal Antibodies Against Baculovirus-expressed Rat ICAM-4

Rat ICAM-4 domains 1-6 were expressed in the baculovirus expression system as described in Example 7. The recombinant protein was purified using monoclonal antibody 103A (as described in Example 6).

Briefly, 30 mg of purified monoclonal 103A (in 100 mM sodium borate, 500 mM sodium chloride) were coupled to three grams of Activated Cyanogen Bromide Sepharose 4B (Pharmacia, Piscataway, NJ). Baculovirus supernatant containing recombinant rat ICAM-4 (domains 1-6) was loaded on the Sepharose column overnight at 4°C. The column was washed in calcium- magnesium-free phosphate buffered saline (CMF-PBS) and bound material was eluted in 50 mM citric acid, 500 mM NaCl pH 4.0. The sample was neutralized with 1/10 volume Tris pH 10 and stored at -20°C. The purified protein separated on SDS-PAGE appeared greater than 90% pure and migrated at approximately 80 kD.

Mice were immunized with the purified recombinant rat ICAM-4 domains 1-6 protein in a similar manner as described in Example 6. The spleen from mouse #1945 was used for fusion #127. The fusion protocol was as described in Example 6. The fusion wells were screened by ELISA on the recombinant ICAM-4 protein. The secondary screen included immunocyto-chemistry on rat brain sections (as below described in Example 9). Four additional antibodies specific for rat ICAM-4 were cloned out of this fusion: 127A, 127E, 127F and 127H. The immunocytochemical staining pattern of each antibody on rat brain sections was the same as observed with monoclonal antibody 103A (see Example 9). The monoclonal antibodies were tested for their ability to bind the D1/GST and D2/GST fusion proteins (described in Example 5). Monoclonal antibody 127A recognized the D1/GST fusion protein and 127H recognized the D2/GST fusion protein. These two distinct binding specificities along with the others that did not bind either GST protein suggest that at least 3 different epitopes were being recognized by the panel of antibodies. Hybridomas 127A and 127H were deposited May 31, 1995 and June 1, 1995, respectively, with the American Type Culture Collection, 12301 Parklawn Drive, Rockville, Maryland 20852, and assigned Accession Numbers HB11905 and HB11911, respectively.

### EXAMPLE 9

### Immunocytochemistry of Rat ICAM-4 Expression

Immunocytochemistry with monoclonal antibody 103A was performed to localize the protein production within the rat brain.

A brain was harvested from a normal adult female Lewis rat, sagittally sectioned, and washed in RNase-free 1X PBS on ice for 30 min. The brain sections were then placed in Tissue Tek II cryomolds (Miles Laboratories, Inc., Naperville, IL) with a small amount of O.C.T. compound (Miles, Inc., Elkhart, IN). The brains were centered in the cryomold, the cryomold filled with OCT compound, then placed in a container with 2-methylbutane (Aldrich Chemical Company, Inc., Milwaukee, WI) and the container placed in liquid nitrogen. Once the tissue and OCT compound in the cryomold were frozen, the blocks were stored at -80°C until sectioning.

The tissue was sectioned at 6 µm thickness, adhered to Vectabond (Vector Laboratories, Inc., Burlingame, CA) coated slides and allowed to air-dry at room temperature overnight until use. The sections were fixed in ethyl ether (Malinckrodt, Paris, KY) for 5 minutes at room temperature. Once the slides were removed from the ether, the reagent was allowed to evaporate. Each tissue section was blocked with 150 µl 50% Normal rat serum (Sigma) and 2% bovine serum albumin (BSA) (Sigma) in IX PBS (made with sodium phosphates only) for 30 minutes at room temperature. After blocking, the solution was gently blotted from the sections and the purified supernatant antibody 103A (1.65 mg/ml) was diluted 1:10 in the blocking solution and 150 µl applied to each tissue section. The slides were placed in a humidity chamber and incubated at 4°C overnight.

The next day the antibody solution was blotted gently from the section and the slides washed three times in 1X PBS for four minutes in each wash. The excess PBS was aspirated from the slide and 100 µl of the secondary, rat anti mouse-biotin conjugated antibody (Jackson ImmunoResearch Laboratories), diluted 1:100 in a solution of 10% normal rat serum and 2% BSA in IX PBS, applied to the tissues. Incubation was allowed to proceed for one hour at room temperature. The sections were washed two times in 1X PBS for four minutes in each wash, then 100 µl of ABC reagent from an Elite Rat IgG Vectastain ABC kit (Vector Laboratories, Inc., Burlingame, CA), prepared according to the product insert, was applied to each section. Incubation was allowed to proceed for 30 minutes at room temperature. After incubation, the slides were washed two times in 1X PBS (four minutes each wash) and 150 µl of Vector VIP Peroxidase Substrate Solution (Vector Laboratories, Inc.. Burlingame, CA) applied to each section for approximately ten minutes. After color development, the sections were rinsed under running tap water for five minutes, counterstained with Mayer's hematoxylin (Sigma) for 20 seconds, and rinsed again in gently running tap water for five minutes. The slides were dehydrated across a graded series of ethanols, passed through xylene and mounted with Accumount 60 (Stephens Scientific, Riverdale, NJ).

Immunohistochemistry of rat brain sections strained with mAb 103A indicated that rat ICAM-4 is expressed in the neuronal cells of the hippocampus. Staining pattern suggested that the protein might be limited to the neuronal processes (dendrites). Brain sections stained in a similar manner with an irrelevant antibody or second step reagent alone do not show the distinct expression pattern seen with MAb 103A.

### EXAMPLE 10

### Cloning of a Human ICAM-4 Genomic DNA

During the cloning of rat ICAM-4 from genomic DNA, it was discovered that ICAM-4 and ICAM-1 were located within 5 kb of each other and this information was utilized in an attempt to clone the human homologue of ICAM-4.

Genome Systems Inc. (St. Louis, MO) amplified fragments in a human P1 library by PCR using human ICAM-1 domain 3 primers, a sense primer designed complementary to human ICAM-1 domain 3 (H-1/D3 S) and an antisense primer designed complementary to human ICAM-1 domain 3 (H-1/D3 AS). These primers are set forth in SEQ ID NOs: 22 and 23, respectively.

Two clones, designated 1566 and 1567, were identified and subjected to further analysis. Both P1 clones contained approximately 75-95 kb genomic DNA inserts. The clones were digested with *Bam*H1, separated with agarose gel electrophoresis, and blotted onto nylon membranes. Southern blots hybridization were performed under either low stringency (30% formamide) or high stringency (60% formamide) at 42°C with human ICAM-1, ICAM-3 or rat ICAM-4 radiolabeled probes; other constituents of the hybridization solution were as described in Example 1. The low stringency hybridization series was washed at room temperature in 2X SSPE containing 0.1 % SDS. The high stringency hybridization was washed at 65 °C in 0.2X SSPE containing 0.1 % SDS. The washed membranes were exposed to X-ray film for 3.5 hours.

The differential hybridization indicated that human ICAM- 1 was contained on a 5.5 kb *Bam*H1 fragment while human ICAM-3 was located on a 4.0 kb and a 1.5 kb *Bam*H1 fragment. The human ICAM-1 and ICAM-R fragments were subcloned into pBS + and their identity confirmed by limited sequence analysis.

A 7.0 kb *Bam*H1 fragment that hybridized with rat ICAM-4 under high stringency conditions was subcloned and further fragmented with *Rsa*I restriction digestion. Three *Rsa*I fragments that hybridized with rat ICAM-4 were identified and their sequences determined. Based on homology to rat ICAM-4, these fragments appeared to contain domains 2, 3, 4, 5 and part of domain 6.

### EXAMPLE 11

### Cloning of a Human ICAM-4 cDNA

The fragments of genomic DNA corresponding to domains 2-5 of human ICAM-4 (described in Example 10) were used as probes to screen a λgt10 Human hippocampus cDNA library (Clontech, Palo Alto, CA). The library screening protocol was essentially as described in Example 1.

The longest human ICAM-4 clone (#18) that was found in that library was only 992 bp (SEQ ID: 24) and corresponded to roughly the middle of the predicted 3 kb gene. The 992 bp DNA insert from clone 18 (SEQ ID: 24) was used as a probe to screen a λZAPII human hippocampus cDNA library (Stratagene, La Jolla, CA). This library yielded a number of positive clones. The longest clone, #34, was 2775 bp (SEQ ID: 25). Based on alignments to the full length rat ICAM-4, it was predicted that this clone was missing the leader sequence and approximately 30 bp at the 5' end of domain 1. The poly A⁺ tail at the 3' end was missing, but the translation stop codon was present.

A fragment of DNA corresponding to the first 3 domains (nucleotides 1 to 840 in clone #34) was used as a probe to screen a λgt10 cDNA library derived from human cerebral cortex (Clontech, Palo Alto, CA). One clone, 16-1 (SEQ ID: 26), was identified as having 1557 bp, and included 39 bp of 5' untranslated DNA, a leader sequence and sequence information through the fifth domain. Overlapping clones #34 (SEQ ID: 25) and 16-1 (SEQ ID: 26) were used to generate a composite of the full length human ICAM-4 sequence (SEQ ID: 27).

The full length gene is 2927 bp long and encodes a 924 amino acid protein. The ICAM-4 nucleotide sequence is set out in SEQ ID NO: 27 and the amino acid sequence is set out in SEQ ID NO: 28. Sequence alignment with the full length rat ICAM-4 gene (SEQ ID: 11) revealed an overall DNA sequence identity of 82 % and 85 % identity at the amino acid level. The apparent 9 Ig like extracellular domain structure of the protein is conserved between rat and human. The leader sequence extends from amino acid 1 to 28; domain I from amino acid 29 to 117; domain 2 from amino acid 118 to 224; domain 3 from amino acid 225 to 320; domain 4 from amino acid 321 to 405; domain 5 from amino acid 406 to 488; domain 6 from amino acid 489 to 570; domain 7 from amino acid 571 to 663; domain 8 from amino acid 664 to 743; domain 9 from amino acid 744 to 837; the transmembrane region from amino acid 838 to 857 and the cytoplasmic tail from amino acid 858 to 924.

Human ICAM-4 (HuICAM-4), in addition to being genetically linked to ICAM-1 and ICAM-R, also showed certain common structural features that group them together as a family of molecules. A domain by domain alignment of HuICAM-4 with the other members of the ICAM family shows varying degrees of homology. Domain 1 amino acid sequence of HuICAM-4 is 21, 30 and 26% identical to domain I of ICAMs 1, 2 and 3 respectively. Domain 2 of HuICAM-4 is 61, 39 and 62% identical to ICAMs 1, 2 and 3 respectively. Domain 3 of HuICAM-4 is 50 and 65% identical to ICAMs 1 and 3 respectively. Domain 4 of HuICAM-4 is 54 and 64% identical to ICAMs 1 and 3 respectively. Domains 5-8 of HuICAM-4 are most homologous to the fifth domains of ICAM-1 and 3, with percent identities ranging from 33-47 for ICAM-1 domain 5 and 21-31 for ICAM-R domain 5. The ninth domain of HuICAM-4 aligns poorly with the other members of the ICAM family but is homologous to domains 3 (24% identical) and 6 (23% identical) of HuICAM-1.

### EXAMPLE 12

### Northern Analysis of Human ICAM-4 Expression

Two human multiple tissue Northern (MTN) blots were purchased from Clontech (Palo Alto, CA). These contained at least 2 µg of poly A⁺ RNA from 16 different human tissues (as shown in Table 1) run on a denaturing formaldehyde 1.2% agarose gel and transferred to nylon membrane. The blots were prehybridized for three hours at 42°C in 10 ml of a solution containing 5X SSPE, 10X Denhardts solution, 50% formamide, 2% SDS and 100 µg/ml denatured salmon sperm DNA. The blots were hybridized in the above solution with a radiolabeled human ICAM-4 probe (clone #18, SEQ ID; 24) for 16 hours at 42°C. The following day, the blots were washed in a solution of 0.1X SSC/0.1% SDS at room temperature followed by a wash at 50°C. The blots were exposed to x-ray film at -80°C for 24 hours. Results of the analysis are shown below in Table 1.

Only the lane containing RNA from the brain hybridized to the ICAM-4 probe, giving a single band at approximately 3 kb. Longer exposure (five days) confirmed that only the brain had a detectable level of message. In order to determine if all lanes contained comparable amounts of RNA of comparable quality, the same blot was hybridized with a control β-actin probe. Blots were stripped of the ICAM-4 probe by treatment with a boiling solution of 0.1 % SDS for 15 minutes, and subsequently probed in a similar manner with a β actin probe provided by the manufacturer. Except for minor variation in amounts, all lanes were shown to have good quality RNA.

**TABLE 1**

| **Northern Tissue Analysis of Human ICAM-4 Expression** | | |
|---|---|---|
| | **PROBE** | |
| Tissue | ICAM-4 | β-Actin |
| Heart | - | +++ |
| Brain | + | ++ |
| Placenta | - | +++ |
| Lung | - | +++ |
| Liver | - | +++ |
| Skeletal muscle | - | +++ + |
| Kidney | - | +++ |
| Pancreas | - | ++ |
| Spleen | - | +++ |
| Thymus | - | +++ |
| Prostate | - | +++ |
| Testis | - | +++ |
| Ovary | - | +++ |
| Small intestine | - | +++ |
| Colon | - | +++ |
| Peripheral blood leukocyte | - | +++ |

Two additional Northern blots were purchased from Clontech that contained poly A⁺ RNA from 16 different sub-regions of human brain (as shown in Table 2). Blots were probed in a manner similar to that used for tissue analysis and results are shown in Table 2. RNA quality and quantity loaded was checked by probing the blots with a β actin probe.

All of the regions that showed ICAM-4 expression are part of the telencephalon, with the exception of the thalamus which is considered part of the diencephalon. The hippocampus and cerebral cortex appeared to have the highest level of expression. The transcript size in all cases was the same, 3 kb. The exquisite tissue distribution of the ICAM-4 expression suggests that the promoter region may contain elements that confer the observed developmental and spatial expression of the gene product. The utility of such information may provide insight into the understanding of control of neural gene expression in general.

**TABLE 2**

| **Northern Brain Cell Type Analysis of Human ICAM-4 Expression** | | |
|---|---|---|
| | **PROBE** | |
| Brain Region | ICAM-4 | β-Actin |
| Amygdala | ++ | +++ |
| Caudate nucleus | ++ | +++ |
| Corpus callosum | + | +++ |
| Hippocampus | ++ | +++ |
| Hypothalamus | - | +++ |
| Substantia nigra | - | +++ |
| Subthalamic nucleus | + | +++ |
| Thalamus | + | +++ |
| Cerebellum | - | +++ |
| Cerebral cortex | +++ | +++ |
| Medulla | - | +++ |
| Spinal cord | - | +++ |
| Occipital pole | ++ | +++ |
| Frontal lobe | ++ | +++ |
| Temporal lobe | ++ | +++ |
| Putamen | ++ | +++ |

### EXAMPLE 13

### Generation of Human ICAM-4/IgG Fusion Proteins

Human ICAM-4/IgG1 fusion proteins expression plasmids were constructed to produce proteins for generating monoclonal antibodies and for use in adhesion assays to identify potential ICAM-4 ligands. Two constructs were made; the first included DNA encoding domains 1-3 of HuICAM-4 and the second, domains 4-8. Both were linked to the Fc region of human IgG1 in vector pDCS1 that uses the cytomegalovirus (CMV) promoter to drive expression and the signal sequence from IgG4 to facilitate secretion of the molecules.

PCR primers (shown below as SEQ ID NOs: 29-32) were designed to generate the necessary DNA fragments for sub-cloning. The "sense" primer for the 5' end of domain 1 (HI4-D1(s), SEQ ID NO: 29) was designed to fill in 30 base pairs of domain I missing in clone #34. Primers HI4-D1(S) (SEQ ID NO: 29) and HI4-D3(AS) (SEQ ID NO: 30) were used to generate a DNA fragment encoding domains 1-3 of human ICAM-4, corresponding to a region in SEQ ID NO: 1 from nucleotide 130 to nucleotide 996. Primers HI4-D3(S) (SEQ ID NO: 31) and HI4-D8(AS) (SEQ ID NO: 32) were used to generate a DNA fragment encoding domains 4-8 of human ICAM-4, corresponding to a region in SEQ ID NO: 30 from nucleotide 997 to nucleotide 2268. Each 5' primer encoded *a BamH*I restriction site (GGATCC, indicated in bold below) and each 3' (antisense) primer contained *a Xho*I site (CTCGAG, indicated in bold below) to facilitate subcloning 5' to the IgGI gene. All oligonucleotides contain spacer nucleotides (underlined, below) at the 5' end to permit restriction digestion.

PCR reactions were carried out in a 50 µl volume using buffers supplied by Perkin Elmer with the AmpliTaq enzyme. Primers were added at a final concentration of 10 µg/ml and all four dNTPs were included at 2 mM. The reactions were continued through 30 cycles of denaturation (94°C for four minutes), annealing (50°C for two minutes) and extension (72°C for one minute). PCR products were visualized on agarose gels and an aliquot of each reaction was used to subclone the PCR products into vector pCRII (Invitrogen, SanDiego, CA). Sequence analysis was performed to detect possible errors resulting from the amplification process and to confirm proper orientation. Appropriate clones were digested with *Bam*HI and *Xho*I and fragments separated with agarose gel electrophoresis. Purified fragments were ligated into a pDCS 1 vector previously digested with *Bam*HI and *Xho*I and the resulting plasmids were sequenced to confirm proper orientation and reading frame.

Human ICAM-4 domains 1-3 and 4-8/IgG1 fusion proteins were obtained following transient transfection of the expression plasmids into COS7 cells and isolation of the secreted protein from the culture media. Transfection was carried out as follows. Adherent COS7 cells at approximately 50-60% confluence were washed with CMF-PBS and subsequently contacted with 10-15 µg of plasmid DNA in 7.5 ml serum-free DMEM media (Gibco, Gaithersburg, MD) containing 6 µl of 0.25 M chloroquine (Sigma, St. Louis, MO). An additional 7.5 ml of serum-free media containing 150 µl of DEAF dextran (50 mg/ml) (Sigma, St. Louis, MO) were added and the plates incubated 2-3 hours before the media was removed and replaced with 10% DMSO (Mallinckrodt, McGaw Park, Illinois) in PBS. After a one minute incubation, the DMSO solution was removed and replaced with fresh media containing 5 % FBS. Each transfection included multiple plates, and media from cells expressing the same protein were pooled for protein isolation.

Media were collected every three days over the course of 3-4 harvests. Proteins were purified using a 0.4 - 0.8 ml Procep A column (Bioprocessing Ltd, England) pre-equilibrated with 35 mM Tris, 150 mM NaCl, pH 7.5. Culture media was loaded onto the column two times at a flow rate of less than 60 column volumes per hour. The column was washed one time with each of 20 column volumes of Tris/NaCl buffer, 20 column volumes of 0.55 M diethanolamine, pH 8.5, and 20 column volumes of 50 mM citric acid, pH 5.0. The fusion proteins were eluted into one ml fractions using 50 mM citric acid pH 3.0 and each fraction was neutralized with 1/10 volume 1 M Tris, pH 9.5. Protein concentration was determined by OD₂₈₀. and purity was determined using SDS-PAGE.

A significant contamination from bovine IgG (present in the FBS) was noted. Even though the domains 1-3 fusion protein was predicted to be smaller than the domains 4-8 fusion protein, both migrated at approximately 90 kD. One possible explanation for the observation is that the smaller domains 1-3 fusion protein may be more heavily glycosylated than the larger domains 4-8 fusion protein.

In addition to use of the purified proteins for monoclonal antibody production, described below, the proteins will also be used in adhesion assays to identify ICAM-4 ligands.

### EXAMPLE 14

### Monoclonal Antibody Production

The purified protein described in Example 13 was utilized to generate monoclonal antibodies using an immunization protocol as described in Example 6.

The spleen from mouse #2250 (immunized with HuICAM-4 D1-3/IgG1) was used for fusion 172 and the spleen from mouse #2272 (immunized with HuICAM-4 D4-8/IgG1) was used for fusion 173. The fusion protocol utilized was as described in Example 6. Fusion plates were screened by EUSA (essentially as described in Example 6) using each HuICAM-4/IgG1 fusion protein. Fusion well supernatants that recognized the immunogen protein, and no other, were considered for cloning. Immunocytochemistry on human hippocampus sections was used as a secondary screen.

One primary clone from each fusion was positive by immunocytochemistry and was cloned. One of the two clones failed to grow upon cloning, leaving only one candidate to pursue, clone 173E which was derived from the HuICAM-4 D4-8/IgG1 immunized mouse. Hybridoma 173E was deposited June 1, 1995 with the American Type Culture Collection, 12301 Parklawn Drive, Rockville, Maryland 20852, and assigned Accession Number HB11912.

From another fusion derived from a mouse immunized with a soluble ICAM-4 fragment corresponding to domains 1-3, six clones (179A, 179B, 179D, 179H, 179I, and 179K) were found to be specific for HuICAM4 domains 1 through 3 (D1-3). All six antibodies in the 179 series bound to the dendritic processes in the dentate gyrus, as well as the polymorphic and pyramidal cell layers. The monoclonal antibody 179A stained neuronal cell bodies from these areas in addition to the dendritic processes. The hybridoma cell lines producing antibodies 179I and 179H were deposited on June 10, 1996 with the American Type Culture Collection, 12301 Parklawn Drive, Rockville Maryland, 20852 and assigned Accession Numbers HB 12123 and HB 12124, respectively.

Additional fusions are similarly performed to generate other antibodies specifically immunoreactive with particular ICAM-4 regions.

### EXAMPLE 15

### Capture Assay Development

The six monoclonal antibodies from fusion 179 were tested in various combinations for their ability to capture and detect soluble ICAM-4 in solution. The assay, as described below, was established in order to evaluate soluble ICAM-4 levels in human fluids in relation to normal and disease conditions.

Antibody 179I was coated on Immulon 4 (Dynatech) 96 well plates at 3 µg/ml, 125 µl/well for two hours at 37°C. The antibody solution was removed by aspiration and the wells were blocked for 30 minutes at room temperature with 300 µl of blocking solution containing 5 % Teleostean gelatin in calcium-free, magnesium-free PBS (CMF-PBS). The blocking solution was removed by aspiration, a 100 µl of sample fluid diluted in Omni Diluent (CMF-PBS, 1% gelatin, and 0.05% Tween 20) was added to each well, and the mixture incubated at 37°C for 30 minutes. The plates were washed three times with PBST (CMF-PBS, 0.05 % Tween 20). Antibody 179H was biotinylated at 1.5 mg/ml using NHS-LC-Biotin (Pierce) following suggested manufacturer's protocol, diluted 1:2000, and added to the wells (100 µl/well). The resulting mixture was incubated for 30 minutes at 37°C and the plates washed three times with PBST. Streptavidin-HRP (Pierce) was added (100 µl, 0.25 µg/ml) to each well and this mixture incubated at 37°C for 30 minutes. The plates were washed four times with PBST before addition of 100 µl of Tetramethylbenzidine (Sigma) (10 mg/ml stock in DMSO) diluted 1:100 in buffered substrate (13.6 g/L sodium acetate trihydrate, pH to 5.5 with I M citric acid, with 150 µl/L 30% hydrogen peroxide added just prior to developing). The reaction was allowed to develop for 30 minutes at room temperature in the dark, after which the reaction was stopped with addition of 50 µl/well 15% H₂SO₄. The absorbance was read at 450 nm.

Results indicated that the assay was capable of detecting soluble HuICAM-4 D1-3 recombinant protein at a concentration as low as 5-10 ng/ml with the linear portion of the curve being in the 10 - 100 ng/ml range. No cross-reactivity to HuICAM4 D4-8 was observed when this protein region was tested at I and 10 µg/ml.

### EXAMPLE 16

### Assessment of Soluble ICAIM-4 in Serum from Stroke Patients

In order to assess the role of ICAM-4 in neurologic diseases and conditions, serum from twenty-eight patients suffering from acute stroke and twenty young healthy volunteers (not age matched) was assayed as described above for differences in serum concentration of soluble ICAM-4.

Results indicated that serum from the healthy volunteers had no detectable level of ICAM-4. Twenty out of twenty-eight acute stroke patients, however, had detectable levels of soluble ICAM-4. The signal from the positive stroke patients corresponded to a range of 5-38 ng/ml of the standard (soluble ICAM-4 D1-3 recombinant protein).

### EXAMPLE 17

### ICAM-4 mRNA Levels in Hippocampus in a Rat Model of Epilepsy

Levels of rat ICAM-4 mRNA expressed were assessed in hippocampus of rats treated in a manner to create a kindling epileptogenesis animal model [Lothman, *et al., Brain Res.* 360:83-91 (1985)]. In the model, the rat hippocampus is stimulated with a series of subconvulsive electric shocks through an electrode implanted in the region of the brain which gradually elicits severe behavioral seizures. The kindling process involves twelve stimulations per day administered every other day for eight days. Once fully kindled, a single stimulus can elicit behavioral seizures and histologic changes that are similar to human epilepsy. Fully kindled rats received two stimulations per day over a two week period and animals were sacrificed 24 hours after the last stimulation. The hippocampus was removed and dissected for RNA preparation.

Total RNA was prepared from each sample using the guanidinium/phenol/chloroform extraction procedure [Chomezynski and Sacchi, *Anal. Biochem*. 162:156-159 (1987)]. RNA was separated on denaturing formaldehyde agarose gels, transferred to nylon membranes, and hybridized with radiolabelled rat ICAM-4 and glyceraldehyde-3-phosphate dehydrogenase (GAPDH) specific DNA probes. GAPDH is a basally expressed gene that is commonly used as a control to detect lane to lane variation in the amount of RNA loaded on a gel. Fluctuations in the ratio of the ICAM-4/GAPDH are interpreted as changes in the level of ICAM-4 expression. Hybridizing bands for ICAM-4 and GAPDH were quantitated with a phosphorimager and a ratio of ICAM-4/GAPDH determined.

The ratio of ICAM-4/GAPDH was significantly higher in the control animals that were not kindled (n=5) compared to the kindled test group (n=5), suggesting that ICAM-4 was down regulated as a consequence of the kindling process. It should be noted, however, that the control group did not undergo any sham treatment so the possibility exists that ICAM-4 mRNA levels were modulated in response to the surgical treatment associated with kindling.

### EXAMPLE 18

### Serum ICAM-4 Concentration as a Marker for Neurodegenerative Disorders

Circulating serum concentrations of ICAM-4 were assessed as a possible indicator for various neurodegenerative disorders. Serum and/or plasma samples from anonymous donors were assayed as described in Example 16 above and compared to samples drawn from control donors with no previous history of neurological disorders.

### Control Donors

In order to establish a baseline average for circulating ICAM-4 in normal healthy individuals, serum samples from 100 donors were examined. The results showed that twelve individuals (12%) had circulating levels of ICAM-4 greater than 10 ng/ml. Of these twelve, the ICAM-4 concentration in five samples averaged 10-20 ng/ml, three samples showed an average ICAM-4 concentration of 20-100 ng/ml, two samples showed ICAM-4 levels between 100-500 ng/ml, and two samples contained ICAM-4 at a concentration in excess of 500 ng/ml.

Samples were taken at the same time from both donors with very high levels at varying timepoints over an eight month period to assess the stability of the observations over time. It was observed that over a period of months, the readings did fluctuate. No medical information was available on these donors, making correlations with the ICAM-4 levels and the physical well-being of the donors not possible. When both serum and plasma samples were prepared from the same individual, no difference was observed in the level of ICAM-4 present.

This observation indicated that an assay for soluble ICAM-4 would be versatile in its use of either serum or plasma. In addition, the results indicated that ICAM-4 is very stable in blood, suggesting that an elevated level of ICAM-4 as a result of some pathological state probably would not be transient. Finally, because of the apparent stability of ICAM-4 in a blood environment, assays for soluble ICAM-4 can utilize blood bank samples thus reducing the need for fresh blood with each assay.

In order to determine if the methods of collection and/or storage affected the observations, the stability of ICAM-4 serum was assessed by treating samples from the one individual with the highest level of circulating ICAM-4 in a variety of ways followed by a measurement of the levels of ICAM-4. Neither incubation at 37°C for 24 hours nor from one to three freeze/thaw cycles altered the level of detectable ICAM-4 in the serum.

### Donors with Epilepsy

The serum concentration of ICAM-4 in samples from twenty patients with Temporal Lobe Epilepsy (TLE) was measured and compared to serum samples from control group patients that had experienced Grand Mal Seizures (38 different patients). Syncope (8 patients) or were normal healthy donors (20 individuals). The assay method described in Example 15 was again employed and the results expressed as ng/ml relative to the internal standard used for the assay, soluble HuICAM-4 D1-3 recombinant protein (described in Example 13).

Serum from all 20 patients with TLE had measurable levels of ICAM-4 with an average of approximately 140 ng/ml. In serum samples from all 3 control groups, including the Grand Mal Seizure group, ICAM-4 concentration averaged below 10 ng/ml. These observations suggest that an individual's ICAM-4 serum level may represent a biochemical marker which can distinguish between focused seizures, like those experienced in TLE. and more generalized Grand Mal Seizures.

### Donors with AIDS

Serum concentration of ICAM-4 in the sera from a limited number of AIDS patients was also examined. The patients were grouped according to CD4 counts and the presence of any signs of dementia. A first group comprised sixteen early stage, asymptomatic patients with CD4 counts greater than 500 were tested. A second group comprised seven later stage patients with CD4 counts less than 300; signs of dementia were not determined for this group. The last group comprised nine late stage AIDS patients, each showing signs of dementia.

The results showed that serum samples from four of the sixteen (25%) early stage, asymptomatic patients had detectable levels of soluble ICAM-4; three of the four samples had an ICAM-4 concentration in excess of 500 ng/ml. Four of the seven (57%) serum samples from later stage patients were also positive for ICAM-4, with two of the four having ICAM-4 concentrations in excess of 500 ng/ml. Samples from the late stage patients showing signs of dementia had no detectable levels of ICAM-4. The results of this preliminary study suggest that ICAM-4 may be an early marker of the neurodegeneration associated with AIDS dementia.

### Donors with Other Neurodegenerative Diseases

The results from the study of serum from epilepsy and AIDS donors suggest that ICAM-4 levels in the blood may reflect damage to the neurons that normally express it. There are a number of other neurologic diseases that might also show, as part of their etiology, damage to specific ICAM-4 expressing neurons that could result in changes in the serum concentration of ICAM-4 in the periphery.

For example, Alzheimer's disease is associated with extensive neuronal damage in the regions of the telencephalon where ICAM-4 is expressed. Assessment of ICAM-4 levels in serum from patients with the Early-onset Familial forms of the disease, as well as patients with the sporadic form of the disease, may provide a marker for the various stages of the disease thereby permitting assessment of possible therapeutic interventions.

As another example, because other cortical dementias, such as Pick's disease, diffuse cortical Lewy body disease, and frontal lobe degeneracy, are sometimes mistaken for Alzheimer's, but may be distinguishable from each other and from Alzheimer's disease through serum ICAM-4 analysis. As another example, serum ICAM-4 concentration in patients suffering from a subcortical dementia, including Parkinson's disease, Huntington's disease, and progressive supranuclear, may be elevated as a result of common pathological indications of this class of disorders.

As another example, a number of the primary psychiatric disorders, such as depression, schizophrenia and psychosis, are characterized in part by degrees of neurodegeneration that might be associated with detectable levels of ICAM-4 in the blood.

As another example, elevated levels of ICAM-4 may be associated with a number of nongenetic dementias arising from infections, vasculitis, metabolic and nutritional disorders (*e.g.*, thyroid, vitamin B12 deficiency), vascular disorders (multiple infarct, lacunar state, Binswanger's disease), toxic encephalopathies (*e.g*., exposure to carbon monoxide, heavy metals or other industrial pollutants) and tumors.

### EXAMPLE 19

### Cloning and Analysis of Human ICAM-4 Upstream Regulatory DNA

ICAM-4 gene expression is spatially and temporally regulated, with expression limited to the most anterior or ventral region of the brain, the telencephalon. In an attempt to identify gene sequences responsible for the restricted transcriptional regulation of ICAM-4, the nucleotide region 5' to human ICAM-4 coding sequences was examined.

A 2607 base pair *Bam*HI/*Pst*I fragment derived from a 7.0 kb genomic *Bam*HI fragment (described in Example 10) was sequenced and found to contain 1684 nucleotides upstream of the ATG start codon. The complete sequence for this upstream region is set out in SEQ ID NO: 33. With respect to the position of the ICAM-4 coding region, the "A" in ATG start codon (numbered in SEQ ID NO: 33 as nucleotides 1685-1687) is designated the + I nucleotide and the nucleotide immediately 5' to the A⁺¹ nucleotide is designated -1. Thus the entire sequence is shown as extending from nucleotide -1684 to nucleotide +3, corresponding to numbering in the Sequence Listing nucleotide 1 to nucleotide 1687.

Based on the genomic HuICAM-4 sequence, oligonucleotides were synthesized and used in PCR to generate DNA molecules of various lengths within the upstream regulatory region. Each oligonucleotide set out in Table 3 contained a spacer region (shown in italics) approximately 6-10 bp to allow enzymatic digestion of the PCR product, an *Nhe*I or *Hind*III restriction site (shown in bold), and a specific hybridization primer sequence (underlined). The oligonucleotide names contain numbers that designate its location within the upstream regulatory region. In the PCR amplifications, oligonucleotides were paired as shown in Table 4 to generate DNA fragments containing specific regions of the upstream regulatory region.

The restriction sites and spacer region generated within each oligonucleotide allowed for enzymatic digestion and subsequent directional cloning of individual PCR products into the pGL3 Basic Vector (Promega, Madison, WI) which contains a luciferase reporter gene immediately downstream of a multiple cloning site (MCS). Promoter activity cloned into the MCS region of the vector drives expression of the luciferase reporter gene in transfected cell lines, and light production from expressed luciferase can be measured as an indicator of promoter activity. The pGL3 Basic Vector has

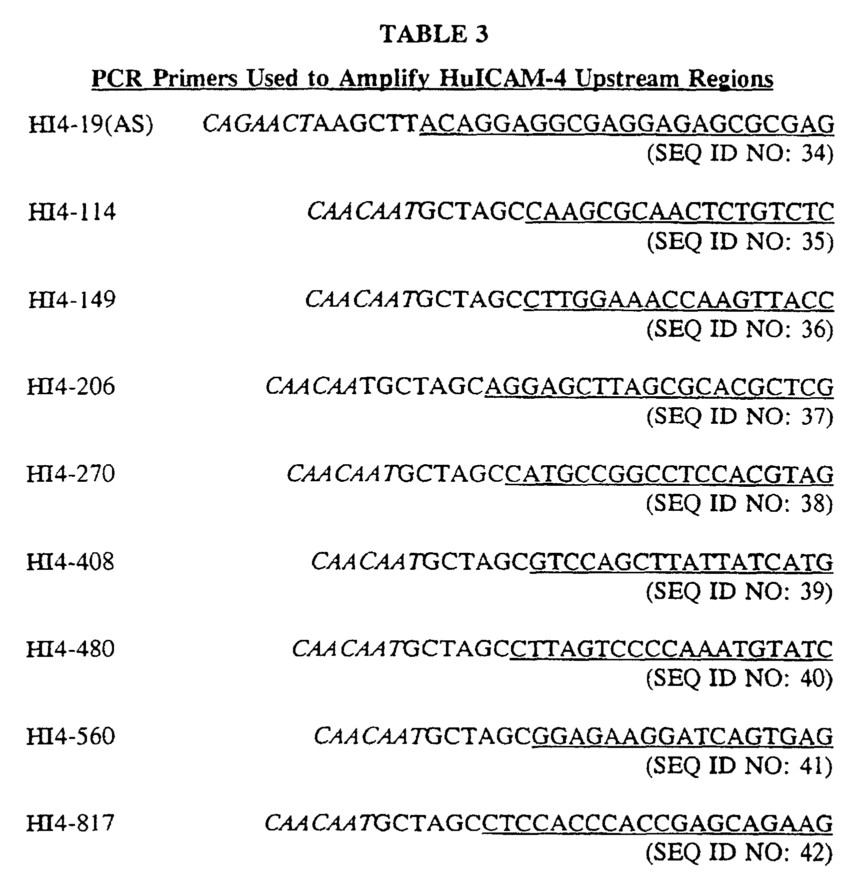

no promoter and therefore served as the negative control, while a pGL3 vector containing an SV40 promoter served as a positive control. The sequence of each expression construct was verified by restriction analysis and DNA sequencing.

Plasmids containing each of the amplified sequences described in Table 4 were transfected into mammalian cells using a Transfection MBS Mammalian Transfection Kit (Stratagene, La Jolla, CA) according to manufacturer's suggested protocol. Each plasmid was introduced into two different cell lines, COS 7 and NT2 Precursor Cells (Ntera2/D1 from Stratagene). COS 7 cells are a commonly used simian fibroblast-like cell line transformed with SV40 making them well suited for driving expression of a gene under control of the SV40 promoter in cells transfected with the positive control pGL3 Promoter Vector. NT2 precursor cells are a committed neuronal precursor cell line, and while they do not express ICAM-4, they may be more representative of a cell type that does express ICAM-4.

**TABLE 4**

| **Primers Paired and Regions Amplified** | |
|---|---|
| Oligonucleotide Pairs | Corresponding Upstream Regulatory Region |
| HI4-19 (AS) with HI4-114 | -19 → -114 |
| HI4-19 (AS) with HI4-149 | -19 → -149 |
| HI4-19 (AS) with HI4-206 | -19 → -206 |
| HI4-19 (AS) with HI4-270 | -19 → -270 |
| HI4-19 (AS) with HI4-408 | -19 → -408 |
| HI4-19 (AS) with HI4-480 | -19 → -480 |
| HI4-19 (AS) with HI4-560 | -19 → -560 |
| HI4-19 (AS) with HI4-817 | -19 → -817 |

Each well of a 6 well flat bottom tissue culture plate (Falcon) was seeded with 2.5x10⁵ cells. Transfections of COS 7 and NT2 cells were done side by side in duplicate using 5 µg of plasmid DNA for each well. The cells were cultured at 37°C for 48 hours, lysed and assayed for luciferase activity with a Luciferase Assay System (Promega).

Results of the experiment, summarized in Table 5, indicate a high level of promoter activity contained within the -408 through -19 and -480 through -19 regions of the upstream regulatory region of ICAM-4 in NT2 cells. Because NT2 cells are of neuronal origin, they may express certain transcription factors recognizing the ICAM-4 promoter that are not found in other cell types. The highest level of promoter activity in COS cell transfectants was obtained with the plasmid containing nucleotides -560 through -19. While the positive control pGL3 Promoter Vector worked well in COS cells, it showed very low promoter activity in NT2 cells, thus illustrating a cell type specific preference for certain promoter sequences.

**TABLE 5**

| **Promoter Activity of 5' ICAM-4 Regions** | | |
|---|---|---|
| Upstream Region | Luminescence | |
| | COS | NT2 |
| -114 through -19 | 0.003 | 0.376 |
| -149 through -19 | 0.008 | 0.628 |
| -206 through -19 | 0.443 | 0.622 |
| -270 through -19 | 0.056 | 1.140 |
| -408 through -19 | 0.401 | 7.970 |
| -480 through -19 | 0.274 | 4.630 |
| -560 through -19 | 3.227 | 1.232 |
| -817 through -19 | 0.035 | 4.453 |
| pGL3 Promoter Vector | 29.070 | 0.063 |
| pGL3 Basic Vector | 0.008 | 0.014 |

Since neither COS 7 or NT2 cells normally express ICAM-4, the same experiment will be repeated using primary cultured rat hippocampal neurons which do express ICAM-4 and necessarily express transcriptional machinery required for ICAM-4 promoter activity. By transfecting the individual promoter constructs described herein, as well as others, into the more natural environment, it may be possible to identify more precisely which nucleotides in the upstream regulatory region are responsible for tight regulation of the ICAM-4 gene in the brain.

The foregoing illustrative examples relate to presently preferred embodiments of the invention and numerous modifications and variations thereof will be expected to occur to those skilled in the art. Thus only such limitations as appear in the appended claims should be placed upon the scope of the present invention.

## Claims

1. A method of distinguishing between various forms of epilepsy selected from Grand Mal seizures, syncope, or temporal the epilepsy (TLE) comprising the steps of:
a) contacting a fluid sample obtained from a first individual with an antibody specifically immunoreactive with ICAM-4;
b) quantitating the level of ICAM-4/antibody binding in the sample; and
c) comparing the level of ICAM-4/antibody binding in the sample to the level of ICAM-4/antibody binding in a second individual having a knows form of epilepsy selected from Grand Mal Seizures, Syncope, or temporal lobe epilepsy (TLE).

2. The method of claim 1 wherein the fluid sample is serum.

3. The method of claim 1 wherein the fluid sample is plasma.

4. The method of claim 1 wherein the fluid sample is cerebrospinal fluid.

5. The method of claim 1 wherein the ICAM-4/antibody binding is quantitated by radioimmunoassay (RIA).

6. The method of claim 1 wherein the ICAM-4/antibody binding is quantitated by enzyme-linked immunosorbent assay (ELISA).

## Patentansprüche

1. Verfahren zur Unterscheidung von verschiedenen Formen von Epilepsie, ausgewählt aus Grand mal - Krämpfen, Synkope oder Schläfenlappen-Epilepsie (SLE), umfassend die Schritte von:
a) in Kontakt bringen einer Flüssigkeitsprobe, die von einem ersten Individuum erhalten wurde, mit einem spezifisch mit ICAM-4 immunreaktiven Antikörper;
b) Quantifizieren des Spiegels von ICAM-4/Antikörper-Bindung in der Probe; und
c) Vergleichen der Spiegel von ICAM-4/Antikörper-Bindung in der Probe mit dem Spiegel von ICAM-4/Antikörper-Bindung in einem zweiten Individuum, das eine bekannte Form von Epilepsie, ausgewählt aus Grand mal - Krämpfen, Synkope oder Schläfenlappen-Epilepsie (SLE) aufweist.

2. Verfahren nach Anspruch 1, wobei die Flüssigkeitsprobe Serum ist.

3. Verfahren nach Anspruch 1, wobei die Flüssigkeitsprobe Plasma ist.

4. Verfahren nach Anspruch 1, wobei die Flüssigkeitsprobe cerebrospinale Flüssigkeit ist.

5. Verfahren nach Anspruch 1, wobei die ICAM-4/Antikörper-Bindung durch Radioimmunassay (RIA) quantifiziert wird.

6. Verfahren nach Anspruch 1, wobei die ICAM-4/Antikörper-Bindung durch Enzymgekoppelten Immunsorbent-Assay (ELISA) quantifiziert wird.

## Revendications

1. Procédé pour distinguer différentes formes d'épilepsie, choisies parmi des apoplexies Grand Mal, une syncope ou une épilepsie du lobe temporal (TLE), comprenant les étapes de
a) mettre en contact un échantillon de fluide obtenu à partir d'un premier individu avec un anticorps spécifiquement immunoréactif avec ICAM-4;
b) quantifier le taux de liaison ICAM-4/anticorps dans l'échantillon ; et
c) comparer le taux de liaison ICAM-4/anticorps dans l'échantillon au taux de liaison ICAM-4/anticorps chez un second individu ayant une forme connue d'épilepsie choisie parmi des apoplexies Grand Mal, une syncope ou une épilepsie du lobe temporal (TLE).

2. Procédé de la revendication 1, dans lequel l'échantillon de fluide est du sérum.

3. Procédé de la revendication 1, dans lequel l'échantillon de fluide est du plasma.

4. Procédé de la revendication 1, dans lequel l'échantillon de fluide est du fluide cérébrospinal.

5. Procédé de la revendication 1, dans lequel la liaison ICAM-4/anticorps est quantifiée par test radioimmunologique (RIA).

6. Procédé de la revendication 1, dans lequel la liaison ICAM-4/anticorps est quantifiée par test immunosorbant lié à une enzyme (ELISA).


### SEQUENCE LISTING

(1) GENERAL INFORMATION:
   (i) APPLICANT: Gallatin, W. Michael
      Kilgannon, Patrick D.
   (ii) TITLE OF INVENTION: ICAM-4 Materials and Methods
   (iii) NUMBER OF SEQUENCES: 42
   (iv) CORRESPONDENCE ADDRESS:
      (A) ADDRESSEE: Marshall, O'Toole, Gerstein, Murray & Borun
      (B) STREET: 233 South Wacker Drive, 6300 Sears Tower
      (C) CITY: Chicago
      (D) STATE: Illinois
      (E) COUNTRY: United States of America
      (F) ZIP: 60606-6402
   (v) COMPUTER READABLE FORM:
      (A) MEDIUM TYPE: Floppy disk
      (B) COMPOTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 07/827,689
      (B) FILING DATE: 27-JAN-1992
   (vii) PRIOR APPLICATION DATA:
      (A) APPLICATION NUMBER: US 07/889,724
      (B) FILING DATE: 26-MAY-1992
   (vii) PRIOR APPLICATION DATA:
      (A) APPLICATION NUMBER: US 07/894,061
      (B) FILING DATE: 05-JUN-1992
   (vii) PRIOR APPLICATION DATA:
      (A) APPLICATION NUMBER: US 08/009,266
      (B) FILING DATE: 22-JAN-1993
   (vii) PRIOR APPLICATION DATA:
      (A) APPLICATION NUMBER: US 08/102,852
      (B) FILING DATE: 05-AUG-1993
   (vii) PRIOR APPLICATION DATA:
      (A) APPLICATION NUMBER: US 08/245,295
      (B) FILING DATE: 18-MAY-1994
   (vii) PRIOR APPLICATION DATA:
      (A) APPLICATION NUMBER: US 08/485,604
      (B) FILING DATE: 07-JUN-1995
   (viii) ATTORNEY/AGENT INFORMATION:
      (A) NAME: WILLIAMS, JR. JOSEPH A.
      (B) REGISTRATION NUMBER: 38,659
      (C) REFERENCE/DOCKET NUMBER: 27866/33321
   (ix) TELECOMMUNICATION INFORMATION:
      (A) TELEPHONE: 312-474-6300
      (B) TELEFAX: 312-474-0448
      (C) TELEX: 25-3856
(2) INFORMATION FOR SEQ ID NO:1:
   (i) SEQUENCE CHARACTERISTICS:
      (A) LENGTH: 2988 base pairs
      (B) TYPE: nucleic acid
      (C) STRAMDEDMESS: single
      (D) TOPOLOGY: linear
   (ii) MOLECULE TYPE: cDNA
   (ix) FEATURE:
      (A) NAME/KEY: CDS
      (B) LOCATION: 61..2814
   (xi) SEQUENCE DESCRIPTION: SEQ ID NO:1:
   AATTCGATCA CTCGCGCTCC CCTCGCCTTC TGCGCTCTCC CCTCCCTGGC AGCGGCGGCA      60
   ATG CCG GGG CCT TCA CCA GGG CTG CGC CGA ACG CTC CTC GGC CTC TGG       108
   Met Pro Gly Pro Ser Pro Gly Leu Arg Arg Thr Leu Leu Gly Leu Trp
    1               5                  10                  15
   GCT GCC CTG GGC CTG GGG ATC CTA GGC ATC TCA GCG GTC GCG CTA GAA       156
   Ala Ala Leu Gly Leu Gly Ile Leu Gly Ile Ser Ala Val Ala Leu Glu
               20                  25                  30
   CCT TTC TGG GCG GAC CTT CAG CCC CGC GTG GCG CTC GTG GAG CGC GGG       204
   Pro Phe Trp Ala Asp Leu Gln Pro Arg Val Ala Leu Val Glu Arg Gly
           35                  40                  45
   GGC TCG CTG TGG CTC AAC TGC AGC ACT AAC TGT CCG AGG CCG GAG CGC       252
   Gly Ser Leu Trp Leu Asn Cys Ser Thr Asn Cys Pro Arg Pro Glu Arg
       50                  55                  60
   GGT GGC CTG GAG ACC TCG CTA CGC CGA AAC GGG ACC CAG AGG GGT CTG       300
   Gly Gly Leu Glu Thr Ser Leu Arg Arg Asn Gly Thr Gln Arg Gly Leu
   65                  70                  75                  80
   CGC TGG CTG GCT CGA CAG CTG GTG GAC ATC CGA GAG CCT GAA ACC CAG       348
   Arg Trp Leu Ala Arg Gln Leu Val Asp Ile Arg Glu Pro Glu Thr Gln
                   85                  90                  95
   CCG GTC TGC TTC TTC CGC TGC GCG CGC CGC ACA CTC CAA GCG CGT GGG       396
   Pro Val Cys Phe Phe Arg Cys Ala Arg Arg Thr Leu Gln Ala Arg Gly
               100                 105                 110
   CTC ATC CGA ACT TTC CAG CGA CCG GAT CGG GTA GAG CTA GTG CCT CTG       444
   Leu Ile Arg Thr Phe Gln Arg Pro Asp Arg Val Glu Leu Val Pro Leu
           115                 120                 125
   CCT CCT TGG CAG CCT GTA GGT GAG AAC TTC ACC TTG AGC TGC AGG GTC       492
   Pro Pro Trp Gln Pro Val Gly Glu Asn Phe Thr Leu Ser Cys Arg Val
       130                 135                 140
   CCG GGG GCA GGA CCC CGA GCG AGC CTC ACA TTG ACC TTG CTG CGA GGC       540
   Pro Gly Ala Gly Pro Arg Ala Ser Leu Thr Leu Thr Leu Leu Arg Gly
   145                 150                 155                 160
   GGC CAG GAG CTG ATT CGC CGA AGT TTC GTA GGC GAG CCA CCC CGA GCT       588
   Gly Gln Glu Leu Ile Arg Arg Ser Phe Val Gly Glu Pro Pro Arg Ala
                   165                 170                 175
   CGG GGT GCG ATG CTC ACC GCC ACG GTC CTG GCG CGC AGA GAG GAT CAC       636
   Arg Gly Ala Met Leu Thr Ala Thr Val Leu Ala Arg Arg Glu Asp His
               180                 185                 190
   AGG GCC AAT TTC TCA TGC CTC GCG GAG CTT GAC CTG CGG CCA CAC GGC       684
   Arg Ala Asn Phe Ser Cys Leu Ala Glu Leu Asp Leu Arg Pro His Gly
           195                 200                 205
   TTG GGA CTG TTT GCA AAC AGC TCA GCC CCC AGA CAG CTC CGC ACG TTT       732
   Leu Gly Leu Phe Ala Asn Ser Ser Ala Pro Arg Gln Leu Arg Thr Phe
       210                 215                 220
   GCC ATG CCT CCA CTT TCC CCG AGC CTT ATT GCC CCA CGA TTC TTA GAA       780
   Ala Met Pro Pro Leu Ser Pro Ser Leu Ile Ala Pro Arg Phe Leu Glu
   225                 230                 235                 240
   GTG GGC TCA GAA AGG CCG GTG ACT TGC ACT TTG GAT GGA CTG TTT CCT       828
   Val Gly Ser Glu Arg Pro Val Thr Cys Thr Leu Asp Gly Leu Phe Pro
                   245                 250                 255
   GCC CCA GAA GCC GGG GTT TAC CTC TCT CTG GGA GAT CAG AGG CTT CAT       876
   Ala Pro Glu Ala Gly Val Tyr Leu Ser Leu Gly Asp Gln Arg Leu His
               260                 265                 270
   CCT AAT GTG ACC CTC GAC GGG GAG AGC CTT GTG GCC ACT GCC ACA GCT       924
   Pro Asn Val Thr Leu Asp Gly Glu Ser Leu Val Ala Thr Ala Thr Ala
           275                 280                 285
   ACA GCA AGT GAA GAA CAG GAA GGC ACC AAA CAG CTG ATG TGC ATC GTG       972
   Thr Ala Ser Glu Glu Gln Glu Gly Thr Lys Gln Leu Met Cys Ile Val
       290                 295                 300
   ACC CTC GGG GGC GAA AGC AGG GAG ACC CAG GAA AAC CTG ACT GTC TAC      1020
   Thr Leu Gly Gly Glu Ser Arg Glu Thr Gln Glu Asn Leu Thr Val Tyr
   305                 310                 315                 320
   AGC TTC CCG GCT CCT CTT CTG ACT TTA AGT GAG CCA GAA GCC CCC GAG      1068
   Ser Phe Pro Ala Pro Leu Leu Thr Leu Ser Glu Pro Glu Ala Pro Glu
                   325                 330                 335
   GGA AAG ATG GTG ACC GTA AGC TGC TGG GCA GGG GCC CGA GCC CTT GTC      1116
   Gly Lys Met Val Thr Val Ser Cys Trp Ala Gly Ala Arg Ala Leu Val
               340                 345                 350
   ACC TTG GAG GGA ATT CCA GCT GCG GTC CCT GGG CAG CCC GCT GAG CTC      1164
   Thr Leu Glu Gly Ile Pro Ala Ala Val Pro Gly Gln Pro Ala Glu Leu
           355                 360                 365
   CAG TTA AAT GTC ACA AAG AAT GAC GAC AAG CGG GGC TTC TTC TGC GAC      1212
   Gln Leu Asn Val Thr Lys Asn Asp Asp Lys Arg Gly Phe Phe Cys Asp
       370                 375                 380
   GCT GCC CTC GAT GTG GAC GGG GAA ACT CTG AGA AAG AAC CAG AGC TCT      1260
   Ala Ala Leu Asp Val Asp Gly Glu Thr Leu Arg Lys Asn Gln Ser Ser
   385                 390                 395                 400
   GAG CTT CGT GTT CTG TAC GCA CCT CGG CTG GAT GAC TTG GAC TGT CCC      1308
   Glu Leu Arg Val Leu Tyr Ala Pro Arg Leu Asp Asp Leu Asp Cys Pro
                   405                 410                 415
   AGG AGC TGG ACG TGG CCA GAG GGT CCA GAG CAG ACC CTC CAC TGC GAG        1356
   Arg Ser Trp Thr Trp Pro Glu Gly Pro Glu Gln Thr Leu His Cys Glu
               420                 425                 430
   GCC CGT GGA AAC CCT GAG CCC TCC GTG CAC TGT GCA AGG CCT GAC GGT        1404
   Ala Arg Gly Asn Pro Glu Pro Ser Val His Cys Ala Arg Pro Asp Gly
           435                 440                 445
   GGG GCG GTG CTA GCG CTG GGC CTG TTG GGT CCA GTG ACC CGT GCC CTC        1452
   Gly Ala Val Leu Ala Leu Gly Leu Leu Gly Pro Val Thr Arg Ala Leu
       450                 455                 460
   GCG GGC ACT TAC CGA TGT ACA GCA ATC AAT GGG CAA GGC CAG GCG GTC        1500
   Ala Gly Thr Tyr Arg Cys Thr Ala Ile Asn Gly Gln Gly Gln Ala Val
   465                 470                 475                 480
   AAG GAT GTG ACC CTG ACT GTG GAA TAT GCC CCA GCG CTG GAC AGT GTA        1548
   Lys Asp Val Thr Leu Thr Val Glu Tyr Ala Pro Ala Leu Asp Ser Val
                   485                 490                 495
   GGC TGC CCA GAA CGT ATT ACT TGG CTG GAG GGG ACA GAG GCA TCG CTT        1596
   Gly Cys Pro Glu Arg Ile Thr Trp Leu Glu Gly Thr Glu Ala Ser Leu
               500                 505                 510
   AGC TGT GTG GCA CAC GGG GTC CCA CCA CCT AGC GTG AGC TGT GTG CGC        1644
   Ser Cys Val Ala His Gly Val Pro Pro Pro Ser Val Ser Cys Val Arg
           515                 520                 525
   TCT GGA AAG GAG GAA GTC ATG GAA GGG CCC CTG CGT GTG GCC CGG GAG        1692
   Ser Gly Lys Glu Glu Val Met Glu Gly Pro Leu Arg Val Ala Arg Glu
       530                 535                 540
   CAC GCT GGC ACT TAC CGA TGC GAA GCC ATC AAC GCC AGG GGA TCA GCG        1740
   His Ala Gly Thr Tyr Arg Cys Glu Ala Ile Asn Ala Arg Gly Ser Ala
   545                 550                 555                 560
   GCC AAA AAT GTG GCT GTC ACG GTG GAA TAT GGT CCC AGT TTT GAG GAG        1788
   Ala Lys Asn Val Ala Val Thr Val Glu Tyr Gly Pro Ser Phe Glu Glu
                   565                 570                 575
   TTG GGC TGC CCC AGC AAC TGG ACT TGG GTA GAA GGA TCT GGA AAA CTG        1836
   Leu Gly Cys Pro Ser Asn Trp Thr Trp Val Glu Gly Ser Gly Lys Leu
               580                 585                 590
   TTT TCC TGT GAA GTT GAT GGG AAG CCG GAA CCA CGC GTG GAG TGC GTG        1884
   Phe Ser Cys Glu Val Asp Gly Lys Pro Glu Pro Arg Val Glu Cys Val
           595                 600                 605
   GGC TCG GAG GGT GCA AGC GAA GGG GTA GTG TTG CCC CTG GTG TCC TCG        1932
   Gly Ser Glu Gly Ala Ser Glu Gly Val Val Leu Pro Leu Val Ser Ser
       610                 615                 620
   AAC TCT GGT TCC AGA AAC TCT ATG ACT CCT GGT AAC CTG TCA CCG GGT        1980
   Asn Ser Gly Ser Arg Asn Ser Met Thr Pro Gly Asn Leu Ser Pro Gly
   625                 630                 635                 640
   ATT TAC CTC TGC AAC GCC ACC AAC CGG CAT GGC TCC ACA GTC AAA ACA        2028
   Ile Tyr Leu Cys Asn Ala Thr Asn Arg His Gly Ser Thr Val Lys Thr
                   645                 650                 655
   GTC GTC GTG AGC GCG GAA TCA CCG CCA CAG ATG GAT GAA TCC AGT TGC        2076
   Val Val Val Ser Ala Glu Ser Pro Pro Gln Met Asp Glu Ser Ser Cys
               660                 665                 670
   CCG AGT CAC CAG ACA TGG CTG GAA GGA GCC GAG GCT ACT GCG CTG GCC        2124
   Pro Ser His Gln Thr Trp Leu Glu Gly Ala Glu Ala Thr Ala Leu Ala
           675                 680                 685
   TGC AGT GCC AGA GGC CGC CCC TCT CCA CGC GTG CGC TGT TCC AGG GAA        2172
   Cys Ser Ala Arg Gly Arg Pro Ser Pro Arg Val Arg Cys Ser Arg Glu
       690                 695                 700
   GGT GCA GCC AGG CTG GAG AGG CTA CAG GTG TCC CGA GAG GAT GCG GGG        2220
   Gly Ala Ala Arg Leu Glu Arg Leu Gln Val Ser Arg Glu Asp Ala Gly
   705                 710                 715                 720
   ACC TAC CTG TGT GTG GCT ACC AAC GCG CAT GGC ACG GAT TCA CGG ACC        2268
   Thr Tyr Leu Cys Val Ala Thr Asn Ala His Gly Thr Asp Ser Arg Thr
                   725                 730                 735
   GTC ACT GTG GGT GTG GAA TAC CGG CCT GTG GTG GCT GAG CTG GCA GCC        2316
   Val Thr Val Gly Val Glu Tyr Arg Pro Val Val Ala Glu Leu Ala Ala
               740                 745                 750
   TCG CCC CCA AGC GTG CGG CCT GGC GGA AAC TTC ACT CTG ACC TGC CGT        2364
   Ser Pro Pro Ser Val Arg Pro Gly Gly Asn Phe Thr Leu Thr Cys Arg
           755                 760                 765
   GCA GAG GCC TGG CCT CCA GCC CAG ATC AGC TGG CGC GCG CCC CCG GGA        2412
   Ala Glu Ala Trp Pro Pro Ala Gln Ile Ser Trp Arg Ala Pro Pro Gly
       770                 775                 780
   GCT CTC AAC CTC GGT CTC TCC AGC AAC AAC AGC ACG CTG AGC GTG GCG        2460
   Ala Leu Asn Leu Gly Leu Ser Ser Asn Asn Ser Thr Leu Ser Val Ala
   785                 790                 795                 800
   GGT GCC ATG GGC AGC CAT GGT GGC GAG TAT GAG TGC GCA GCC ACC AAT        2508
   Gly Ala Met Gly Ser His Gly Gly Glu Tyr Glu Cys Ala Ala Thr Asn
                   805                 810                 815
   GCG CAT GGG CGC CAC GCA CGG CGC ATC ACG GTG CGC GTG GCC GGT CCA        2556
   Ala His Gly Arg His Ala Arg Arg Ile Thr Val Arg Val Ala Gly Pro
               820                 825                 830
   TGG CTG TGG GTC GCT GTG GGC GGT GCG GCA GGG GGC GCG GCG CTG CTG        2604
   Trp Leu Trp Val Ala Val Gly Gly Ala Ala Gly Gly Ala Ala Leu Leu
           835                 840                 845
   GCC GCA GGG GCC GGC CTG GCC TTC TAC GTG CAG TCC ACC GCT TGC AAG        2652
   Ala Ala Gly Ala Gly Leu Ala Phe Tyr Val Gln Ser Thr Ala Cys Lys
       850                 855                 860
   AAG GGA GAG TAC AAC GTC CAG GAG GCT GAG AGC TCA GGC GAG GCG GTG        2700
   Lys Gly Glu Tyr Asn Val Gln Glu Ala Glu Ser Ser Gly Glu Ala Val
   865                 870                 875                 880
   TGT CTC AAT GGC GCG GGC GGG ACA CCG GGT GCA GAA GGC GGA GCA GAG        2748
   Cys Leu Asn Gly Ala Gly Gly Thr Pro Gly Ala Glu Gly Gly Ala Glu
                   885                 890                 895
   ACC CCC GGC ACT GCC GAG TCA CCT GCA GAT GGC GAG GTT TTC GCC ATC        2796
   Thr Pro Gly Thr Ala Glu Ser Pro Ala Asp Gly Glu Val Phe Ala Ile
               900                 905                 910
   CAG CTG ACA TCT TCC TGAGCCTGTA TCCAGCTCCC CCAGGGGCCT CGAAAGCACA        2851
   Gln Leu Thr Ser Ser
           915
   GGGGTGGACG TATGTATTGT TCACTCTCTA TTTATTCAAC TCCAGGGGCG TCGTCCCCGT 2911
   TTTCTACCCA TTCCCTTAAT AAAGTTTTTA TAGGAGAAAA AAAAAAAAAA AAAAAAAAAA 2971
   AAAAAAAAAA AAAAAAA 2988
(2) INFORMATION FOR SEQ ID NO:2:
   (i) SEQUENCE CHARACTERISTICS:
      (A) LENGTH: 917 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Pro Gly Pro Ser Pro Gly Leu Arg Arg Thr Leu Leu Gly Leu Trp
   1               5                   10                  15
   Ala Ala Leu Gly Leu Gly Ile Leu Gly Ile Ser Ala Val Ala Leu Glu
               20                  25                  30
   Pro Phe Trp Ala Asp Leu Gln Pro Arg Val Ala Leu Val Glu Arg Gly
           35                  40                  45
   Gly Ser Leu Trp Leu Asn Cys Ser Thr Asn Cys Pro Arg Pro Glu Arg
       50                  55                  60
   Gly Gly Leu Glu Thr Ser Leu Arg Arg Asn Gly Thr Gln Arg Gly Leu
   65                  70                  75                  80
   Arg Trp Leu Ala Arg Gln Leu Val Asp Ile Arg Glu Pro Glu Thr Gln
                   85                  90                  95
   Pro Val Cys Phe Phe Arg Cys Ala Arg Arg Thr Leu Gln Ala Arg Gly
               100                 105                 110
   Leu Ile Arg Thr Phe Gln Arg Pro Asp Arg Val Glu Leu Val Pro Leu
           115                 120                 125
   Pro Pro Trp Gln Pro Val Gly Glu Asn Phe Thr Leu Ser Cys Arg Val
       130                 135                 140
   Pro Gly Ala Gly Pro Arg Ala Ser Leu Thr Leu Thr Leu Leu Arg Gly
   145                 150                 155                 160
   Gly Gln Glu Leu Ile Arg Arg Ser Phe Val Gly Glu Pro Pro Arg Ala
                   165                 170                 175
   Arg Gly Ala Met Leu Thr Ala Thr Val Leu Ala Arg Arg Glu Asp His
               180                 185                 190
   Arg Ala Asn Phe Ser Cys Leu Ala Glu Leu Asp Leu Arg Pro His Gly
           195                 200                 205
   Leu Gly Leu Phe Ala Asn Ser Ser Ala Pro Arg Gln Leu Arg Thr Phe
       210                 215                 220
   Ala Met Pro Pro Leu Ser Pro Ser Leu Ile Ala Pro Arg Phe Leu Glu
   225                 230                 235                 240
   Val Gly Ser Glu Arg Pro Val Thr Cys Thr Leu Asp Gly Leu Phe Pro
   245 250 255
   Ala Pro Glu Ala Gly Val Tyr Leu Ser Leu Gly Asp Gln Arg Leu His
   260 265 270
   Pro Asn Val Thr Leu Asp Gly Glu Ser Leu Val Ala Thr Ala Thr Ala
   275 280 285
   Thr Ala Ser Glu Glu Gln Glu Gly Thr Lys Gln Leu Met Cys Ile Val
   290 295 300
   Thr Leu Gly Gly Glu Ser Arg Glu Thr Gln Glu Asn Leu Thr Val Tyr
   305 310 315 320
   Ser Phe Pro Ala Pro Leu Leu Thr Leu Ser Glu Pro Glu Ala Pro Glu
   325 330 335
   Gly Lys Met Val Thr Val Ser Cys Trp Ala Gly Ala Arg Ala Leu Val
   340 345 350
   Thr Leu Glu Gly Ile Pro Ala Ala Val Pro Gly Gln Pro Ala Glu Leu
   355 360 365
   Gln Leu Asn Val Thr Lys Asn Asp Asp Lys Arg Gly Phe Phe Cys Asp
   370 375 380
   Ala Ala Leu Asp Val Asp Gly Glu Thr Leu Arg Lys Asn Gln Ser Ser
   385 390 395 400
   Glu Leu Arg Val Leu Tyr Ala Pro Arg Leu Asp Asp Leu Asp Cys Pro
   405 410 415
   Arg Ser Trp Thr Trp Pro Glu Gly Pro Glu Gln Thr Leu His Cys Glu
   420 425 430
   Ala Arg Gly Asn Pro Glu Pro Ser Val His Cys Ala Arg Pro Asp Gly
   435 440 445
   Gly Ala Val Leu Ala Leu Gly Leu Leu Gly Pro Val Thr Arg Ala Leu
   450 455 460
   Ala Gly Thr Tyr Arg Cys Thr Ala Ile Asn Gly Gln Gly Gln Ala Val
   465 470 475 480
   Lys Asp Val Thr Leu Thr Val Glu Tyr Ala Pro Ala Leu Asp Ser Val
   485 490 495
   Gly Cys Pro Glu Arg Ile Thr Trp Leu Glu Gly Thr Glu Ala Ser Leu
   500 505 510
   Ser Cys Val Ala His Gly Val Pro Pro Pro Ser Val Ser Cys Val Arg
   515 520 525
   Ser Gly Lys Glu Glu Val Met Glu Gly Pro Leu Arg Val Ala Arg Glu
   530 535 540
   His Ala Gly Thr Tyr Arg Cys Glu Ala Ile Asn Ala Arg Gly Ser Ala
   545 550 555 560
   Ala Lys Asn Val Ala Val Thr Val Glu Tyr Gly Pro Ser Phe Glu Glu
   565 570 575
   Leu Gly Cys Pro Ser Asn Trp Thr Trp Val Glu Gly Ser Gly Lys Leu
   580 585 590
   Phe Ser Cys Glu Val Asp Gly Lys Pro Glu Pro Arg Val Glu Cys Val
   595 600 605
   Gly Ser Glu Gly Ala Ser Glu Gly Val Val Leu Pro Leu Val Ser Ser
   610 615 620
   Asn Ser Gly Ser Arg Asn Ser Met Thr Pro Gly Asn Leu Ser Pro Gly
   625 630 635 640
   Ile Tyr Leu Cys Asn Ala Thr Asn Arg His Gly Ser Thr Val Lys Thr
   645 650 655
   Val Val Val Ser Ala Glu Ser Pro Pro Gln Met Asp Glu Ser Ser Cys
   660 665 670
   Pro Ser His Gln Thr Trp Leu Glu Gly Ala Glu Ala Thr Ala Leu Ala
   675 680 685
   Cys Ser Ala Arg Gly Arg Pro Ser Pro Arg Val Arg Cys Ser Arg Glu
   690 695 700
   Gly Ala Ala Arg Leu Glu Arg Leu Gln Val Ser Arg Glu Asp Ala Gly
   705 710 715 720
   Thr Tyr Leu Cys Val Ala Thr Asn Ala His Gly Thr Asp Ser Arg Thr
   725 730 735
   Val Thr Val Gly Val Glu Tyr Arg Pro Val Val Ala Glu Leu Ala Ala
   740 745 750
   Ser Pro Pro Ser Val Arg Pro Gly Gly Asn Phe Thr Leu Thr Cys Arg
   755 760 765
   Ala Glu Ala Trp Pro Pro Ala Gln Ile Ser Trp Arg Ala Pro Pro Gly
   770 775 780
   Ala Leu Asn Leu Gly Leu Ser Ser Asn Asn Ser Thr Leu Ser Val Ala
   785 790 795 800
   Gly Ala Met Gly Ser His Gly Gly Glu Tyr Glu Cys Ala Ala Thr Asn
   805 810 815
   Ala His Gly Arg His Ala Arg Arg Ile Thr Val Arg Val Ala Gly Pro
   820 825 830
   Trp Leu Trp Val Ala Val Gly Gly Ala Ala Gly Gly Ala Ala Leu Leu
   835 840 845
   Ala Ala Gly Ala Gly Leu Ala Phe Tyr Val Gln Ser Thr Ala Cys Lys
   850 855 860
   Lys Gly Glu Tyr Asn Val Gln Glu Ala Glu Ser Ser Gly Glu Ala Val
   865 870 875 880
   Cys Leu Asn Gly Ala Gly Gly Thr Pro Gly Ala Glu Gly Gly Ala Glu
   885 890 895
   Thr Pro Gly Thr Ala Glu Ser Pro Ala Asp Gly Glu Val Phe Ala Ile
   900 905 910
   Gln Leu Thr Ser Ser
   915
(2) INFORMATION FOR SEQ ID NO:3:
   (i) SEQUENCE CHARACTERISTICS:
      (A) LENGTH: 315 base pairs
      (B) TYPE: nucleic acid
      (C) STRAHDEDNESS: single
      (D) TOPOLOGY: linear
   (ii) MOLECULE TYPE: DMA (genomic)
   (ix) FEATURE:
      (A) HAMS/KEY: CDS
      (B) LOCATION: 1..315
   (xi) SEQUENCE DESCRIPTION: SEQ ID NO:3:
   CCG GAT CGG GTA GAG CTA GTG CCT CTG CCT CCT TGG CAG CCT GTA GGT      48
   Pro Asp Arg Val Glu Leu Val Pro Leu Pro Pro Trp Gln Pro Val Gly
     1               5                  10                  15
   GAG AAC TTC ACC TTG AGC TGC AGG GTC CCG GGG GCA GGA CCC CGA GCG      96
   Glu Asn Phe Thr Leu Ser Cys Arg Val Pro Gly Ala Gly Pro Arg Ala
                20                  25                  30
   AGC CTC ACA TTG ACC TTG CTG CGA GGC GGA CAG GAG CTG ATT CGC CGA     144
   Ser Leu Thr Leu Thr Leu Leu Arg Gly Gly Gln Glu Leu Ile Arg Arg
            35                  40                  45
   AGT TTC GTA GGC GAG CCA CCC CGA GCT CGG TGT GCG ATG CTC ACC GCC     192
   Ser Phe Val Gly Glu Pro Pro Arg Ala Arg Cys Ala Met Leu Thr Ala
        50                  55                  60
   ACG GTC CTG GCG CGC AGA GAG GAT CAC AGG GAC AAT TTC TCA TGC CTC     240
   Thr Val Leu Ala Arg Arg Glu Asp His Arg Asp Asn Phe Ser Cys Leu
    65                  70                  75                  80
   GCG GAG CTT GAC CTG CGG ACA CAC GGC TTG GGA CTG TTT GCA AAC AGC     288
   Ala Glu Leu Asp Leu Arg Thr His Gly Leu Gly Leu Phe Ala Asn Ser
                    85                  90                  95
   TCA GCC CCC AGA CAG CTC CGC ACG TTT                                 315
   Ser Ala Pro Arg Gln Leu Arg Thr Phe
               100                 105
(2) INFORMATION FOR SEQ ID NO:4:
   (i) SEQUENCE CHARACTERISTICS:
      (A) LENGTH: 1781 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 16..1659
   (xi) SEQUENCE DESCRIPTION: SEQ ID NO:4:
   CAGCTCTCTG TCAGA ATG GCC ACC ATG GTA CCA TCC GTG TTG TGG CCC AGG     51
                    Met Ala Thr Met Val Pro Ser Val Leu Trp Pro Arg
                     1               5                   10
   GCC TGC TGG ACT CTG CTG GTC TGC TGT CTG CTG ACC CCA GGT GTC CAG      99
   Ala Cys Trp Thr Leu Leu Val Cys Cys Leu Leu Thr Pro Gly Val Gln
           15                  20                  25
   GGG CAG GAG TTC CTT TTG CGG GTG GAG CCC CAG AAC CCT GTG CTC TCT     147
   Gly Gln Glu Phe Leu Leu Arg Val Glu Pro Gln Asn Pro Val Leu Ser
       30                  35                  40
   GCT GGA GGG TCC CTG TTT GTG AAC TGC AGT ACT GAT TGT CCC AGC TCT     195
   Ala Gly Gly Ser Leu Phe Val Asn Cys Ser Thr Asp Cys Pro Ser Ser
    45                 50                  55                  60
   GAG AAA ATC GCC TTG GAG ACG TCC CTA TCA AAG GAG CTG GTG GCC AGT     243
   Glu Lys Ile Ala Leu Glu Thr Ser Leu Ser Lys Glu Leu Val Ala Ser
                   65                  70                  75
   GGC ATG GGC TGG GCA GCC TTC AAT CTC AGC AAC GTG ACT GGC AAC AGT     291
   Gly Met Gly Trp Ala Ala Phe Asn Leu Ser Asn Val Thr Gly Asn Ser
               80                  85                  90
   CGG ATC CTC TGC TCA GTG TAC TGC AAT GGC TCC CAG ATA ACA GGC TCC     339
   Arg Ile Leu Cys Ser Val Tyr Cys Asn Gly Ser Gln Ile Thr Gly Ser
           95                  100                 105
   TCT AAC ATC ACC GTG TAC GGG CTC CCG GAG CGT GTG GAG CTG GCA CCC     387
   Ser Asn Ile Thr Val Tyr Gly Leu Pro Glu Arg Val Glu Leu Ala Pro
       110                 115                 120
   CTG CCT CCT TGG CAG CCG GTG GGC CAG AAC TTC ACC CTG CGC TGC CAA     435
   Leu Pro Pro Trp Gln Pro Val Gly Gln Asn Phe Thr Leu Arg Cys Gln
   125                 130                 135                 140
   GTG GAG GGT GGG TCG CCC CGG ACC AGC CTC ACG GTG GTG CTG CTT CGC     483
   Val Glu Gly Gly Ser Pro Arg Thr Ser Leu Thr Val Val Leu Leu Arg
                   145                 150                 155
   TGG GAG GAG GAG CTG AGC CGG CAG CCC GCA GTG GAG GAG CCA GCG GAG     531
   Trp Glu Glu Glu Leu Ser Arg Gln Pro Ala Val Glu Glu Pro Ala Glu
               160                 165                 170
   GTC ACT GCC ACT GTG CTG GCC AGC AGA GAC GAC CAC GGA GCC CCT TTC     579
   Val Thr Ala Thr Val Leu Ala Ser Arg Asp Asp His Gly Ala Pro Phe
           175                 180                 185
   TCA TGC CGC ACA GAA CTG GAC ATG CAG CCC CAG GGG CTG GGA CTG TTC     627
   Ser Cys Arg Thr Glu Leu Asp Met Gln Pro Gln Gly Leu Gly Leu Phe
       190                 195                 200
   GTG AAC ACC TCA GCC CCC CGC CAG CTC CGA ACC TTT GTC CTG CCC GTG     675
   Val Asn Thr Ser Ala Pro Arg Gln Leu Arg Thr Phe Val Leu Pro Val
   205                 210                 215                 220
   ACC CCC CCG CGC CTC GTG GCC CCC CGG TTC TTG GAG GTG GAA ACG TCG     723
   Thr Pro Pro Arg Leu Val Ala Pro Arg Phe Leu Glu Val Glu Thr Ser
                   225                 230                 235
   TGG CCG GTG GAC TGC ACC CTA GAC GGG CTT TTT CCA GCC TCA GAG GCC     771
   Trp Pro Val Asp Cys Thr Leu Asp Gly Leu Phe Pro Ala Ser Glu Ala
               240                 245                 250
   CAG GTC TAC CTG GCG CTG GGG GAC CAG ATG CTG AAT GCG ACA GTC ATG         819
   Gln Val Tyr Leu Ala Leu Gly Asp Gln Met Leu Asn Ala Thr Val Met
           255                 260                 265
   AAC CAC GGG GAC ACG CTA ACG GCC ACA GCC ACA GCC ACG GCG CGC GCG         867
   Asn His Gly Asp Thr Leu Thr Ala Thr Ala Thr Ala Thr Ala Arg Ala
       270                 275                 280
   GAT CAG GAG GGT GCC CGG GAG ATC GTC TGC AAC GTG ACC CTA GGG GGC         915
   Asp Gln Glu Gly Ala Arg Glu Ile Val Cys Asn Val Thr Leu Gly Gly
   285                 290                 295                 300
   GAG AGA CGG GAG GCC CGG GAG AAC TTG ACG GTC TTT AGC TTC CTA GGA         963
   Glu Arg Arg Glu Ala Arg Glu Asn Leu Thr Val Phe Ser Phe Leu Gly
                   305                 310                 315
   CCC ATT GTG AAC CTC AGC GAG CCC ACC GCC CAT GAG GGG TCC ACA GTG        1011
   Pro Ile Val Asn Leu Ser Glu Pro Thr Ala His Glu Gly Ser Thr Val
               320                 325                 330
   ACC GTG AGT TGC ATG GCT GGG GCT CGA GTC CAG GTC ACG CTG GAC GGA        1059
   Thr Val Ser Cys Met Ala Gly Ala Arg Val Gln Val Thr Leu Asp Gly
           335                 340                 345
   GTT CCG GCC GCG GCC CCG GGG CAG ACA GCT CAA CTT CAG CTA AAT GCT        1107
   Val Pro Ala Ala Ala Pro Gly Gln Thr Ala Gln Leu Gln Leu Asn Ala
       350                 355                 360
   ACC GAG AGT GAC GAC GGA CGC AGC TTC TTC TGC AGT GCC ACT CTC GAG        1155
   Thr Glu Ser Asp Asp Gly Arg Ser Phe Phe Cys Ser Ala Thr Leu Glu
   365                 370                 375                 380
   GTG GAC GGC GAG TTC TTG CAC AGG AAC AGT AGC GTC CAG CTG CGA GTC        1203
   Val Asp Gly Glu Phe Leu His Arg Asn Ser Ser Val Gln Leu Arg Val
                   385                 390                 395
   CTG TAT GGT CCC AAA ATT GAC CGA GCC ACA TGC CCC CAG CAC TTG AAA        1251
   Leu Tyr Gly Pro Lys Ile Asp Arg Ala Thr Cys Pro Gln His Leu Lys
               400                 405                 410
   TGG AAA GAT AAA ACG AGA CAC GTC CTG CAG TGC CAA GCC AGG GGC AAC        1299
   Trp Lys Asp Lys Thr Arg His Val Leu Gln Cys Gln Ala Arg Gly Asn
           415                 420                 425
   CCG TAC CCC GAG CTG CGG TGT TTG AAG GAA GGC TCC AGC CGG GAG GTG        1347
   Pro Tyr Pro Glu Leu Arg Cys Leu Lys Glu Gly Ser Ser Arg Glu Val
       430                 435                 440
   CCG GTG GGG ATC CCG TTC TTC GTC AAC GTA ACA CAT AAT GGT ACT TAT        1395
   Pro Val Gly Ile Pro Phe Phe Val Asn Val Thr His Asn Gly Thr Tyr
   445                 450                 455                 460
   CAG TGC CAA GCG TCC AGC TCA CGA GGC AAA TAC ACC CTG GTC GTG GTG        1443
   Gln Cys Gln Ala Ser Ser Ser Arg Gly Lys Tyr Thr Leu Val Val Val
                   465                 470                 475
   ATG GAC ATT GAG GCT GGG AGC TCC CAC TTT GTC CCC GTC TTC GTG GCG        1491
   Met Asp Ile Glu Ala Gly Ser Ser His Phe Val Pro Val Phe Val Ala
               480                 485                 490
   GTG TTA CTG ACC CTG GGC GTG GTG ACT ATC GTA CTG GCC TTA ATG TAC        1539
   Val Leu Leu Thr Leu Gly Val Val Thr Ile Val Leu Ala Leu Met Tyr
           495                 500                 505
   GTC TTC AGG GAG CAC CAA CGG AGC GGC AGT TAC CAT GTT AGG GAG GAG 1587
   Val Phe Arg Glu His Gln Arg Ser Gly Ser Tyr His Val Arg Glu Glu
   510 515 520
   AGC ACC TAT CTG CCC CTC ACG TCT ATG CAG CCG ACA GAA GCA ATG GGG 1635
   Ser Thr Tyr Leu Pro Leu Thr Ser Met Gln Pro Thr Glu Ala Met Gly
   525 530 535 540
   GAA GAA CCG TCC AGA GCT GAG TGACGCTGGG ATCCGGGATC AAAGTTGGCG 1686
   Glu Glu Pro Ser Arg Ala Glu
   545
   GGGGCTTGGC TGTGCCCTCA GATTCCGCAC CAATAAAGCC TTCAAACTCC CAAAAAAAAA 1746
   AAAAAAAAAA AAAAAAAAAA AAAAAAAAAA AAAAA 1781
(2) INFORMATION FOR SEQ ID NO:5:
   (i) SEQUENCE CHARACTERISTICS:
      (A) LENGTH: 4900 base pairs
      (B) TYPE: nucleic acid
      (C) STRANDEDNESS: single
      (D) TOPOLOGY: linear
   (ii) MOLECULE TYPE: DMA (genomic)
   (xi) SEQUENCE DESCRIPTION: SEQ ID NO:5:
   CCGAACGCTC CTCGGCCTCT GGTCTNCTCT GGNCCTGGGG ATCCTAGGCA TCTCAGGTAA 60
   GAAGAGCCCG CCCGTGGAGC NAGGTGGATA AGGCGGGGGC GGAATTGAAG GACCAGAGAG 120
   GGCGGCCCGG GTGTCCCCCT CCAGGCTCCG CCCTCTTCTA GCTTCCCACG CTTCTGTCAC 180
   CACCTGGAGN TCGGGGCTTC TCCCCGTCCT TCCTCCACCC CAACACACCT CAATCTTTCA 240
   GANCTGAACC CAGCACCTTT TCTGGANTNG GGGNNTTGCA CCTAACCTGT CTCAGGAGAN 300
   ACTGTGGCTC TCCTGTCCTC TCCTGCTCTG TNATGCCCTA TGGTTCACAG ACTGGCATCA 360
   TCCCTATTCA TGATCCTCAA AGACNCCATC TCCTCAACTG TCATAACTCA GAGCTCTATT 420
   CCCCCTCCAC CTGGAGCCCT GGAAACCGGC TTTCTAGGGC TTTTCTCCGC GGTTCTTTCC 480
   CGGAGTTCAG CGTTGTGGCT TTTTGTCCAA GTTACTCAAG TTTGGGGACA ATCTCCTTTA 540
   AGCCTTTGAC TCAGTCTCAT TTCCACTTTG CTTTTGCCCC AAGCCTCTGT GTCTCTCCCC 600
   CATTTCCTGA CGATCTGTCA GAGTCTTAAG AGTGATTTGG TTCCCCATCC CCCCTCCAAC 660
   TGGAGTCTCC TCCTCACTAT TGATGTGTGC ATCTGAGACC CCCATCCCCG CACCGAGTTT 720
   CCCCATCTCT GTCAGTAAAG AGCAAGGCTT CCAGAGACAA CCCTCTAATA GCGCGTCAGT 780
   CCCGAATCTT GAGTGGGATG CGGGACTCCC GTGCTATTTC TTGGCGGAGG TCTTTCCTGG 840
   TCCTTATGGA CACCCCTGGT TTGGGATATG GGGGCCGCTA AGATTTCAGA GATGGGGTCC 900
   CTAGGCTGAG NCCGCGTTTT CCCGGGCAGC GGTCGCGCTA GAACCTTTCT GGGCGGACCT 960
   TCAGCCCCGC GTGGCGCTCG TGGAGCGCGG GGGCTCGCTG TGGCTCAACT GCAGCACTAA 1020
   CTGTCCGAGG CCGGAGCGCG GTGGCCTGGA GACCTCGCTA CGCCGAAACG GGACCCAGAG 1080
   GGGTCTGNAC TGNCTGGCTC GACAGCTGGT GGACATCCGA GANCCTGAAA CCCAGCCGGT 1140
   CTGCTTCTTC CNCTGCGCGC GCCGCACACT CCAAGCGCGT GGGCTCATCC GAACTTTCCG 1200
   TGAGTTCAGG GTGGGCACNC CCCTTGGGTC TCTGGACCTC CCCCTCAAGC TCCTCCCACC 1260
   CGCCCTCTGA TCCTCCTGCT TGTTCTGAAA GTACTACAGC TGGCTAGAGC GGAGTTTTTG 1320
   GTCCCTTGCA GAGCGACCGG ATCGGGTAGA GCTAGTGCCT CTGCCTCCTT GGCAGCCTGT 1380
   AGGTGAGAAC TTCACCTTGA GCTGCAGGGT CCCGGGGGCA GGACCCCGAG CGAGCCTCAC 1440
   ATTGACCTTG CTGCGAGGCG GCCAGGAGCT GATTCGCCGA AGTTTCGTAG GCGAGCCACC 1500
   CCGAGCTCGG GGTGCGATGC TCACCGCCAC GGTCCTGGCG CGCAGAGAGG ATCACAGGGC 1560
   CAATTTCTCA TGCCTCGCGG AGCTTGACCT GCGNCCACAC GGCTTGGGAC TGTTTGCANA 1620
   CAGCTCAGCC CCCAGACAGC TCCGCACGTT TGGTGAGTGT GGACCCTAAC TGACAGATTT 1680
   TAAGAAGTTT AGGGCAGCCA GGCGTGGTGG CATGGTGTCG TAGGCCCTAA GTCCCAGCCC 1740
   AAGCAGANCT AAGNCGGATC TCTTGTGAAT TAAAAGTCTA GCTCGTCTAC ATAACGAGGN 1800
   CTGCATAGTT AAATCCCCCA AAAGTCTAAG CAGCTAGCCC TTACTTCCAA CACAAGTACT 1860
   AGCTTAAGTA CTTTCTCCTG TGAGCTTTTT CCTTTATGTA TTTACTCGTT GAGAGAAAAA 1920
   GAGAGTGTGT GTACGTGCCT TTATGCACAT GCCGCAGTGC TTGTATGGAA GTTAAAGAAT 1980
   AAGGAGGCGT TCTGCCCTTC CATCCTGTGG GTCCTAGGGG TGGTATTAGC TCCTCAGGCT 2040
   TTGTTAGTNA CAAGCGCCTA GGCTTGGGGA GCCATCTCGC CCGCTCCTCT GTATCTTTAG 2100
   GGTGAAACCA GACAATGCAT GCAAATTGGT TGATCAACAC TGAATGTTTA GTTCGTAAAT 2160
   TCAAGCTCTG TTCTTTGTCT TCCTCAGCCA TGCCTCCACT TTCCCCCGAG CCTTATTGCC 2220
   CCACGATTCT TAGAAGTGGG CTCAGAAAGG CCGGTGACKT GCACTTTGGA TGGACTGTTT 2280
   CCTGCCCCAG AAGCCGGGGT TTACTTCTCT CTGGGAGATC AGAGGCTTCA TCCTAATGTG 2340
   ACCCTCGACG GGGAGAGCCT TGTGGCCACT GCCACAGCTA CAGCAAGTGA AGAACAGGAA 2400
   GGCACCAAAC AGCTGATGTG CATCGTGACC CTCGGGGGCG AAAGCAGGGA GACCCAGGAA 2460
   AACCTGACTG TCTACAGTAA GGGGAATCCA ACAAGACCTT CAATAGCTCA GACTGGGGCT 2520
   GGGGCTGGGT CTGGGTCTGG GGCCAGAGTC TCACAAAGGC GGAGCCTATA AAGTGGGCGG 2580
   GACCTCCACA CCAGAACAAG CCGGGCGGGA GAGTTCCAGG GCAGGAGCAG ATAGAAGTTG 2640
   GAAATTAATA GATTGGGTTG AGTTCCCTGA GTGGGGAGTG AACCCCACCC AATTCTCTGT 2700
   CCCCAGGCTT CCCGGCTCCT CTTCTGACTT TAAGTGAGCC AGAAGCCCCC GAGGGAAAGA 2760
   TGGTGACCGT AAGCTGCTGG GCAGGGGCCC GAGCCCTTGT CACCTTGGAG GGAATTCCAA 2820
   GGACCCTCTT ACCGGCCCCA TCTTTAACCT TATCGTATCC CCTCTGCCTC ATGCCCGCAG 2880
   ACGCACCTCG GCTGGATGAC TTGGACTGTC CCAGGAGCTG GACGTGGCCA GAGGGTCCAG 2940
   AGCAGACCCT CCACTGCGAG GCCCGTGGAA ACCCTGAGCC CTCCGTGCAC TGTGCAAGGC 3000
   CTGACGGTGG GGCGGTGCTA GCGCTGGGCC TGTTGGGTCC AGTGACCCGT GCCCTCGCGG 3060
   GCACTTACCG ATGTACAGCA ATCAATGGGC AAGGCCAGGC GGTCAAGGAT GTGACCCTGA 3120
   CTGTGGAATG TGAGTAGGGG GAGGTGGGCA TGCTTATCCC TTTAAGGTCA CGGAGTGTAC 3180
   TGGGAGACTG GCTATACGGA AAGGAAAGAA GCCTAGGTTC AGCAGGGATT GGGAAAACAC 3240
   TGAAGGAAAG TGGTGTGGTG TTTACAAACT TAACGGTGGT AACTGGGCAC GGTCTGGCAA 3300
   AAACAGACAG CCAAGAGAGT GTGCCTGGGA AGCTGCAATG GGGGCTTTGT GGGAATTGGT 3360
   CAACAGCACC CTGAGATCTC AGGAAAGGGG CCTGAAGTTA TCTCCAGAAC CCATGTGAAG 3420
   GCAGGAAGAG AGAACGCCCA CCTTTTCCTG CTCCCCCCAA CCCCCCCCCA CATATCACAC 3480
   GGAGTATATA AATAAATAAA ATGGCTCCTG CCGGAGGGAG TGAGAAGCTG TCTCCTGCAG 3540
   GCTCAGAGCA GTGGTAGTGC ATGCCTTTAA TCCCAGCACT CGGTAGGCAA AGGCAGGCAG 3600
   ATCTCTGTGA ATGTGGGGCC AGCCTGGTCT GTACAGAGAA ATCCTGTCTC AAAACAAACC 3660
   AGCAAAGAAA CAAAACCAAA ATCAATTCCA GATGCCCCAG CGCTGGACAG TGTAGGCTGC 3720
   CCANGACGTA TTACTTGNCT GGAGGGGACA GAGGCATCGC TTAGCTGTGT GGCACACGGG 3780
   GTCCCACCAC CTAGCGTGAG CTGTGTGCGC TCTGGAAAGG AGGAAGTCAT GGAAGGGCCC 3840
   CTGCGTGTGG CCCGGGAGCA CGCTGGCACT TACCGATGCG AAGCCATCAA CGCCAGGGGA 3900
   TCAGCGGNCA AAAATGTGGC TGTCACGGTG GAATGTGAGT AGGGGTGGCT ACGGAAATGT 3960
   CCACACCTGC GTCCTCTGTC CTCAGTGTGA ACTCCTATTT CCCTGCTTCC TAGATGGTCC 4020
   CAGTTNTGAG GAGTTGGGCT GCCCCAGCAA CTGGACTTGG GTAGAAGGAT CTGGAAAACT 4080
   GTTTTCCTGT GAAGTTGATG GGAAGCCGGA ACCACGCGTG GAGTGCGTGG GCTCGGAGGG 4140
   TGCAAGCGAA GGGGTAGTGT TGCCCCTGGT GTCCTCGAAC TCTGGTTCCA GAAACTCTAT 4200
   GACTCCTGGT AACCTGTCAC CGGGTATTTA CCTCTGCAAC GCCACCAACC GGCATGGCTC 4260
   CACAGTCAAA ACAGTCGTCG TGAGCGCGGA ATGTGAGCAG GGGCCCAGGT GGGCGGAGAG 4320
   TACCGGGTGT CCCAGGATCT TTTCTTTCCC TGATGCCCCT CCTTATGGTG GCTGATCTGC 4380
   AGCACCGCCA CAGATGGATG AATCCAGTTG CCCGAGTCAC CAGACATGGC TGGAAGGAGC 4440
   CGAGGCTACT GCGCTGGCCT GCAGTGACAG GGGNCGCCCC TCTCCACGCG TGCGCTGTTC 4500
   CAGGGAAGGT GCAGCCAGGC TGGAGAGGCT ACAGGTGTCC CGAGAGGATG CGGGGACCTA 4560
   CCTGTGTGTG GCTACCAACG CGCATGGCAC GGATTCACGG ACCGTCACTG TGGGTGTGGA 4620
   ATGTGAGTGA GGACAGCGCT GAATGAAGAC GACTCAGACC GCCAGAAAAG TGCCTTGAGG 4680
   CCTGGGATGT ATGATCCAGT GGGTAGAGTG CTCAATTAGC ACTCACTAAA ATGTATATTC 4740
   TATTCCTAAT ACTCTTTAAT TTTANCCTTT GGGAGGCAGA GACAGGCAGA TCTCTGTTCC 4800
   GGGATAACCT GCTCTCTGTC TAGGACAGCT TGGTCTACAG AGGGGNTACA GGCCCCCCCT 4860
   CCCAAGATTG NATAGCAACC CTCTGGCTCC CTGTCTCTCT 4900
(2) INFORMATION FOR SEQ ID NO:6:
   (i) SEQUENCE CHARACTERISTICS:
      (A) LENGTH: 1295 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 6:
   NGAATTCCGG CGGATCGGGT AGAGCTAGTG CCTCTGCCTC CTTGGCAGCC TGTAGGTGAG     60
   AACTTCACCT TGAGCTGCAG GGTCCCGGGG GCAGGACCCC GAGCGAGCCT CACATTGACC    120
   TTGCTGCGAG GCGGCCAGGA GCTGATTCGC CGAAGTTTCG TAGGCGAGCC ACCCCGAGCT    180
   CGGGGTGCGA TGCTCACCGC CACGGTCCTG GCGCGCAGAG AGGATCACAG GGCCAATTTC    240
   TCATGCCTCG CGGAGCTTGA CCTGCGGCCA CACGGCTTGG GACTGTTTGC AAACAGCTCA    300
   GCCCCCAGAC AGCTCCGCAC GTTTGCCATG CCTCCACTTT CCCCGAGCCT TATTGCCCCA    360
   CGATTCTTAG AAGTGGGCTC AGAAAGGCCG GTGACTTGCA CTTTGGATGG ACTGTTTCCT    420
   GCCCCAGAAG CCGGGGTTTA CCTCTCTCTG GGAGATCAGA GGCTTCATCC TAATGTGACC    480
   CTCGACGGGG AGAGCCTTGT GGCCACTGCC ACAGCTACAG CAAGTGAAGA ACAGGAAGGC    540
   ACCAAACAGC TGATGTGCAT CGTGACCCTC GGGGGCGAAA GCAGGGAGAC CCAGGAAAAC    600
   CTGACTGTCT ACAGCTTCCC GGCTCCTCTT CTGACTTTAA GTGAGCCAGA AGCCCCCGAG    660
   GGAAAGATGG TGACCGTAAG CTGCTGGGCA GGGGCCCGAG CCCTTGTCAC CTTGGAGGGA    720
   ATTCCAAGGA CCCTCTTACC GGCCCCATCT TTAACCTTAT CGTATCCCCT CTGCCTCATG    780
   CCCGCAGACG CACCTCGGCT GGATGACTTG GACTGTCCCA GGAGCTGGAC GTGGCCAGAG    840
   GGTCCAGAGC AGACCCTCCA CTGCGAGGCC CGTGGAAACC CTGAGCCCTC CGTGCACTGT    900
   GCAAGGCCTG ACGGTGGGGC GGTGCTAGCG CTGGGCCTGT TGGGTCCAGT GACCCGTGCC    960
   CTCGCGGGCA CTTACCGATG TACAGCAATC AATGGGCAAG GCCAGGCGGT CAAGGATGTG   1020
   ACCCTGACTG TGGAATATGC CCCAGCGCTG GACAGTGTAG GCTGCCCAGA ACGTATTACT   1080
   TGGCTGGAGG GGACAGAGGC ATCGCTTAGC TGTGTGGCAC ACGGGGTCCC ACCACCTAGC   1140
   GTGAGCTGTG TGCGCTCTGG AAAGGAGGAA GTCATGGAAG GGCCCCTGCG TTTTGGCCGG   1200
   GAGCACGCTG GCACTTACCG ATGCGAAGCC ATCAACGCCA GGGGATCAGC GGCCAAAAAT   1260
   GTGGCTGTCA CGGTGGAATA TGGTCCCCGG AATTC                              1295
(2) INFORMATION FOR SEQ ID NO:7:
   (i) SEQUENCE CHARACTERISTICS:
      (A) LENGTH: 2214 base pairs
      (B) TYPE: nucleic acid
      (C) STRAHDEDNESS: single
      (D) TOPOLOGY: linear
   (ii) MOLECULE TYPE: CDNA
   (xi) SEQUENCE DESCRIPTION: SEQ ID NO:7:
   CGAATCTTGA GTGGGATGCG GGACTCCCGT GCTATTTCTT GGCGGAGGTC TTTCCTGGTC     60
   CTTATGGACA CCCCTGGTTT GGGATATGGG GGCCGCTAAG ATTTCAGAGA TGGGGTCCCT    120
   AGGCTGAGCC CGCGTTTTCC CGGGCAGCGG TCGCGCTAGA ACCTTTCTGG GCGGACCTTC    180
   AGCCCCGCGT GGCGCTCGTG GAGCGCGGGG GCTCGCTGTG GCTCAACTGC AGCACTAACT    240
   GTCCGAGGCC GGAGCGCGGT GGYCTGGAGA CCTCGCTACG CCGAAACGGG ACCCAGAGGG    300
   GTCTGCGCTG GCTGGCTCGA CAGMTGGTGG ACATCCGAGA GCCTGAAACC CAGTCGGTCT    360
   GCTTCTTCCG CTGGGCGCGC CGCACACTCC AAGNGAGTGG GCTCATCCGA ACTTTCCAGC    420
   GACCGGATCG GGTAGAGCTA GTGCCTCTGN CTCCTTGGCA GCCTGTAGGT GAGAACTTCA    480
   CCTTGAGCTG CAGGGTCCCG GGGGCAGGAC CCCGAGCGAG CCTCACATTG ACCTTGCTGC    540
   GAGGCGGCCA GGAGCTGATT CGCCGAAGTT TCGTAGGCGA GCCACCCCGA GCTCGGGGTG    600
   CGATGCTCAC CGCCACGGTC CTGGCGCGCA GAGAGGATCA CAGGGCCAAT TTCTCATGCC    660
   TCGCGGAGCT TGACCTGCGG ACACACGGCT TGGGACTGTT TGCAAACAGC TCAGCCCCCA    720
   GACAGCTCCG CACGTTTGGC ATGCCTCCAC TTTCCCCGAG CCTTATTGNC CCACGATTCT    780
   TAGAAGTGGG CTCAGAAAGG CCGGTGACTT GCACTTTGGA TGGACTGTTT CCTGCCCCAG    840
   AAGCCGGGGT TTACCTCTCT CTGGGAGATC AGAGGCTTCA TCCTAATGTG ACCCTCGACG    900
   GGGAGAGCCT TGTGGCCACT GNCACAGMTA CAGCAAGTGA AGAACAGGAA GGCACCAAAC    960
   AGCTGATGTG CATCGTGACC CTCGGGGGCG AAAGCAGGGA GACCCAGGAA AACCTGACTG   1020
   TCTACAGCTT CCCGGCTCCT CTTCTGACTT TAAGTGAGCC AGAAGCCCCC GAGGGAAAGA   1080
   TGGTGACCGT AAGCTGCTGG GCAGGGGCCC GAGCCCTTGT CACCTTGGAG GGAATTCCAG   1140
   CTGCGGTCCC TGGGCAGCCC GCTGAGCTCC AGTTAAATGT CACAAAGAAT GACGACAAGC   1200
   GGGGCTTCTT CTGCGACGCT GCCCTCGATG TGGACGGGGA AACTCTGAGA AAGAACCAGA   1260
   GCTCTGAGCT TCGTGTTCTG TACGCACCTC GGCTGGATGA CTTGGACTGT CCCAGGAGCT   1320
   GGACGTGGCC AGAGGGTCCA GAGCAGACCC TCCACTGCGA GGCCCGTGGA AACCCTGAGC   1380
   CCTCCGTGCA CTGTGCAAGG CCTGACGGTG GGGCGGTGCT AGCGCTGGGC CTGTTGGGTC   1440
   CAGTGACCCG TGCCCTCGCG GGAACTTACC GATGTACAGC AATCAATGGG CAAGGCCAGG   1500
   CGGTCAAGGA TGTGACCCTG ACTGTGGAAT ATGCCCCAGC GCTGGACAGT GTAGGCTGCC   1560
   CAGAACGTAT TACTTGGCTG GAGGGGACAG AGGCATCGCT TAGCTGTGTG GCACACGGGG   1620
   TCCCACCACC TAGCGTGAGC TGTGTGCGCT CTGGAAAGGA GGAAGTCATG GAAGGGCCCC   1680
   TGCGTGTGGC CCGGGAGCAC GCTGGCACTT ACCGATGCGA AGCCATCAAC GNCAGGGGAT   1740
   CAGCGGWCAA AAATGTGGCT GTCACGGTGG AATATGGTCC CAGTTTGGAG GAGTTGGGCT   1800
   GCCCCAGYAA CTGGACTTGG GTAGAAGGAT CTGGAAAACT GTTTTCCTGT GAAGTTGATG 1860
   GGAAGCCGGA ACCACGCGTG GAGTGCGTGG GCTCGGAGGG TGCAAGCGAA GGGGTAGTGT 1920
   TGCCCCTGGT GTCCTCGAAC TCTGGTTCCA GAAACTCTAT GACTCCTGGT AACCTGTCAC 1980
   CGGGTATTTA CCTCTGCAAC GCCACCAACC GGMATGGNTC CACAGTCAAA ACAGTCGTCG 2040
   TGAGCGCGGA ATCACCGCCA CAGATGGATG AATCCAGTTG CCCGAGTCAC CAGACATGGN 2100
   TGGAAGGAGC CGAGGNTACT GCGCTGGCCT GCAGTGCCAG AGGNCGCCCC TCTCCACGCG 2160
   TGCGCTGTTC CAGGGAAGGT GCAGMCAGGC TGGAGAGGNT ACAGGTGTCC CGAG 2214
(2) INFORMATION FOR SEQ ID NO:8:
   (i) SEQUENCE CHARACTERISTICS:
      (A) LENGTH: 5077 base pairs
      (B) TYPE: nucleic acid
      (C) STRAMDEDNESS: single
      (D) TOPOLOGY: linear
   (ii) MOLECULE TYPE: DNA (genomic)
   (xi) SEQUENCE DESCRIPTION: SEQ ID NO:8:
   CCGAACGCTC CTCGGCCTCT GGTCTNCTCT GGNCCTGGGG ATCCTAGGCA TCTCAGGTAA 60
   GAAGAGCCCG CCCGTGGAGC NAGGTGGATA AGGCGGGGGC GGAATTGAAG GACCAGAGAG 120
   GGCGGCCCGG GTGTCCCCCT CCAGGCTCCG CCCTCTTCTA GCTTCCCACG CTTCTGTCAC 180
   CACCTGGAGN TCGGGGCTTC TCCCCGTCCT TCCTCCACCC CAACACACCT CAATCTTTCA 240
   GANCTGAACC CAGCACCTTT TCTGGANTNG GGGNNTTGCA CCTAACCTGT CTCAGGAGAN 300
   ACTGTGGCTC TCCTGTCCTC TCCTGCTCTG TNATGCCCTA TGGTTCACAG ACTGGCATCA 360
   TCCCTATTCA TGATCCTCAA AGACNCCATC TCCTCAACTG TCATAACTCA GAGCTCTATT 420
   CCCCCTCCAC CTGGAGCCCT GGAAACCGGC TTTCTAGGGC TTTTCTCCGC GGTTCTTTCC 480
   CGGAGTTCAG CGTTGTGGCT TTTTGTCCAA GTTACTCAAG TTTGGGGACA ATCTCCTTTA 540
   AGCCTTTGAC TCAGTCTCAT TTCCACTTTG CTTTTGCCCC AAGCCTCTGT GTCTCTCCCC 600
   CATTTCCTGA CGATCTGTCA GAGTCTTAAG AGTGATTTGG TTCCCCATCC CCCCTCCAAC 660
   TGGAGTCTCC TCCTCACTAT TGATGTGTGC ATCTGAGACC CCCATCCCCG CACCGAGTTT 720
   CCCCATCTCT GTCAGTAAAG AGCAAGGCTT CCAGAGACAA CCCTCTAATA GCGCGTCAGT 780
   CCCGAATCTT GAGTGGGATG CGGGACTCCC GTGCTATTTC TTGGCGGAGG TCTTTCCTGG 840
   TCCTTATGGA CACCCCTGGT TTGGGATATG GGGGCCGCTA AGATTTCAGA GATGGGGTCC 900
   CTAGGCTGAG NCCGCGTTTT CCCGGGCAGC GGTCGCGCTA GAACCTTTCT GGGCGGACCT 960
   TCAGCCCCGC GTGGCGCTCG TGGAGCGCGG GGGCTCGCTG TGGCTCAACT GCAGCACTAA 1020
   CTGTCCGAGG CCGGAGCGCG GTGGCCTGGA GACCTCGCTA CGCCGAAACG GGACCCAGAG 1080
   GGGTCTGNAC TGNCTGGCTC GACAGCTGGT GGACATCCGA GANCCTGAAA CCCAGCCGGT 1140
   CTGCTTCTTC CNCTGCGCGC GCCGCACACT CCAAGCGCGT GGGCTCATCC GAACTTTCCG 1200
   TGAGTTCAGG GTGGGCACNC CCCTTGGGTC TCTGGACCTC CCCCTCAAGC TCCTCCCACC 1260
   CGCCCTCTGA TCCTCCTGCT TGTTCTGAAA GTACTACAGC TGGCTAGAGC GGAGTTTTTG 1320
   GTCCCTTGCA GAGCGACCGG ATCGGGTAGA GCTAGTGCCT CTGCCTCCTT GGCAGCCTGT 1380
   AGGTGAGAAC TTCACCTTGA GCTGCAGGGT CCCGGGGGCA GGACCCCGAG CGAGCCTCAC 1440
   ATTGACCTTG CTGCGAGGCG GCCAGGAGCT GATTCGCCGA AGTTTCGTAG GCGAGCCACC 1500
   CCGAGCTCGG GGTGCGATGC TCACCGCCAC GGTCCTGGCG CGCAGAGAGG ATCACAGGGC 1560
   CAATTTCTCA TGCCTCGCGG AGCTTGACCT GCGNCCACAC GGCTTGGGAC TGTTTGCANA 1620
   CAGCTCAGCC CCCAGACAGC TCCGCACGTT TGGTGAGTGT GGACCCTAAC TGACAGATTT 1680
   TAAGAAGTTT AGGGCAGCCA GGCGTGGTGG CATGGTGTCG TAGGCCCTAA GTCCCAGCCC 1740
   AAGCAGANCT AAGNCGGATC TCTTGTGAAT TAAAAGTCTA GCTCGTCTAC ATAACGAGGN 1800
   CTGCATAGTT AAATCCCCCA AAAGTCTAAG CAGCTAGCCC TTACTTCCAA CACAAGTACT 1860
   AGCTTAAGTA CTTTCTCCTG TGAGCTTTTT CCTTTATGTA TTTACTCGTT GAGAGAAAAA 1920
   GAGAGTGTGT GTACGTGCCT TTATGCACAT GCCGCAGTGC TTGTATGGAA GTTAAAGAAT 1980
   AAGGAGGCGT TCTGCCCTTC CATCCTGTGG GTCCTAGGGG TGGTATTAGC TCCTCAGGCT 2040
   TTGTTAGTNA CAAGCGCCTA GGCTTGGGGA GCCATCTCGC CCGCTCCTCT GTATCTTTAG 2100
   GGTGAAACCA GACAATGCAT GCAAATTGGT TGATCAACAC TGAATGTTTA GTTCGTAAAT 2160
   TCAAGCTCTG TTCTTTGTCT TCCTCAGCCA TGCCTCCACT TTCCCCCGAG CCTTATTGCC 2220
   CCACGATTCT TAGAAGTGGG CTCAGAAAGG CCGGTGACKT GCACTTTGGA TGGACTGTTT 2280
   CCTGCCCCAG AAGCCGGGGT TTACTTCTCT CTGGGAGATC AGAGGCTTCA TCCTAATGTG 2340
   ACCCTCGACG GGGAGAGCCT TGTGGCCACT GCCACAGCTA CAGCAAGTGA AGAACAGGAA 2400
   GGCACCAAAC AGCTGATGTG CATCGTGACC CTCGGGGGCG AAAGCAGGGA GACCCAGGAA 2460
   AACCTGACTG TCTACAGTAA GGGGAATCCA ACAAGACCTT CAATAGCTCA GACTGGGGCT 2520
   GGGGCTGGGT CTGGGTCTGG GGCCAGAGTC TCACAAAGGC GGAGCCTATA AAGTGGGCGG 2580
   GACCTCCACA CCAGAACAAG CCGGGCGGGA GAGTTCCAGG GCAGGAGCAG ATAGAAGTTG 2640
   GAAATTAATA GATTGGGTTG AGTTCCCTGA GTGGGGAGTG AACCCCACCC AATTCTCTGT 2700
   CCCCAGGCTT CCCGGCTCCT CTTCTGACTT TAAGTGAGCC AGAAGCCCCC GAGGGAAAGA 2760
   TGGTGACCGT AAGCTGCTGG GCAGGGGCCC GAGCCCTTGT CACCTTGGAG GGAATTCCAG 2820
   CTGCGGTCCC TGGGCAGCCC GCTGAGCTCC AGTTAAATGT CACAAAGAAT GACGACAAGC 2880
   GGGGCTTCTT CTGCGACGCT GCCCTCGATG TGGACGGGGA AACTCTGAGA AAGAACCAGA 2940
   GCTCTGAGCT TCGTGTTCTG TGTGAGTGGA TGTTCACTTT ATCTCTGTGA ATTCCAAGGA 3000
   CCCTCTTACC GGCCCCATCT TTAACCTTAT CGTATCCCCT CTGCCTCATG CCCGCAGACG 3060
   CACCTCGGCT GGATGACTTG GACTGTCCCA GGAGCTGGAC GTGGCCAGAG GGTCCAGAGC 3120
   AGACCCTCCA CTGCGAGGCC CGTGGAAACC CTGAGCCCTC CGTGCACTGT GCAAGGCCTG 3180
   ACGGTGGGGC GGTGCTAGCG CTGGGCCTGT TGGGTCCAGT GACCCGTGCC CTCGCGGGCA 3240
   CTTACCGATG TACAGCAATC AATGGGCAAG GCCAGGCGGT CAAGGATGTG ACCCTGACTG 3300
   TGGAATGTGA GTAGGGGGAG GTGGGCATGC TTATCCCTTT AAGGTCACGG AGTGTACTGG 3360
   GAGACTGGCT ATACGGAAAG GAAAGAAGCC TAGGTTCAGC AGGGATTGGG AAAACACTGA 3420
   AGGAAAGTGG TGTGGTGTTT ACAAACTTAA CGGTGGTAAC TGGGCACGGT CTGGCAAAAA 3480
   CAGACAGCCA AGAGAGTGTG CCTGGGAAGC TGCAATGGGG GCTTTGTGGG AATTGGTCAA 3540
   CAGCACCCTG AGATCTCAGG AAAGGGGCCT GAAGTTATCT CCAGAACCCA TGTGAAGGCA 3600
   GGAAGAGAGA ACGCCCACCT TTTCCTGCTC CCCCCAACCC CCCCCCACAT ATCACACGGA 3660
   GTATATAAAT AAATAAAATG GCTCCTGCCG GAGGGAGTGA GAAGCTGTCT CCTGCAGGCT 3720
   CAGAGCAGTG GTAGTGCATG CCTTTAATCC CAGCACTCGG TAGGCAAAGG CAGGCAGATC 3780
   TCTGTGAATG TGGGGCCAGC CTGGTCTGTA CAGAGAAATC CTGTCTCAAA ACAAACCAGC 3840
   AAAGAAACAA AACCAAAATC AATTCCAGAT GCCCCAGCGC TGGACAGTGT AGGCTGCCCA 3900
   NGACGTATTA CTTGNCTGGA GGGGACAGAG GCATCGCTTA GCTGTGTGGC ACACGGGGTC 3960
   CCACCACCTA GCGTGAGCTG TGTGCGCTCT GGAAAGGAGG AAGTCATGGA AGGGCCCCTG 4020
   CGTGTGGCCC GGGAGCACGC TGGCACTTAC CGATGCGAAG CCATCAACGC CAGGGGATCA 4080
   GCGGNCAAAA ATGTGGCTGT CACGGTGGAA TGTGAGTAGG GGTGGCTACG GAAATGTCCA 4140
   CACCTGCGTC CTCTGTCCTC AGTGTGAACT CCTATTTCCC TGCTTCCTAG ATGGTCCCAG 4200
   TTNTGAGGAG TTGGGCTGCC CCAGCAACTG GACTTGGGTA GAAGGATCTG GAAAACTGTT 4260
   TTCCTGTGAA GTTGATGGGA AGCCGGAACC ACGCGTGGAG TGCGTGGGCT CGGAGGGTGC 4320
   AAGCGAAGGG GTAGTGTTGC CCCTGGTGTC CTCGAACTCT GGTTCCAGAA ACTCTATGAC 4380
   TCCTGGTAAC CTGTCACCGG GTATTTACCT CTGCAACGCC ACCAACCGGC ATGGCTCCAC 4440
   AGTCAAAACA GTCGTCGTGA GCGCGGAATG TGAGCAGGGG CCCAGGTGGG CGGAGAGTAC 4500
   CGGGTGTCCC AGGATCTTTT CTTTCCCTGA TGCCCCTCCT TATGGTGGCT GATCTGCAGC 4560
   ACCGCCACAG ATGGATGAAT CCAGTTGCCC GAGTCACCAG ACATGGCTGG AAGGAGCCGA 4620
   GGCTACTGCG CTGGCCTGCA GTGACAGGGG NCGCCCCTCT CCACGCGTGC GCTGTTCCAG 4680
   GGAAGGTGCA GCCAGGCTGG AGAGGCTACA GGTGTCCCGA GAGGATGCGG GGACCTACCT 4740
   GTGTGTGGCT ACCAACGCGC ATGGCACGGA TTCACGGACC GTCACTGTGG GTGTGGAATG 4800
   TGAGTGAGGA CAGCGCTGAA TGAAGACGAC TCAGACCGCC AGAAAAGTGC CTTGAGGCCT 4860
   GGGATGTATG ATCCAGTGGG TAGAGTGCTC AATTAGCACT CACTAAAATG TATATTCTAT 4920
   TCCTAATACT CTTTAATTTT ANCCTTTGGG AGGCAGAGAC AGGCAGATCT CTGTTCCGGG 4980
   ATAACCTGCT CTCTGTCTAG GACAGCTTGG TCTACAGAGG GGNTACAGGC CCCCCCTCCC 5040
   AAGATTGNAT AGCAACCCTC TGGCTCCCTG TCTCTCT 5077
(2) INFORMATION FOR SEQ ID NO:9:
   (i) SEQUENCE CHARACTERISTICS:
      (A) LENGTH: 1472 base pairs
      (B) TYPE: nucleic acid
      (C) STRAMDEDNESS: single
      (D) TOPOLOGY: linear
   (ii) MOLECULE TYPE: CDNA
   (xi) SEQUENCE DESCRIPTION: SEQ ID NO:9:
   NGAATTCCGG CGGATCGGGT AGAGCTAGTG CCTCTGCCTC CTTGGCAGCC TGTAGGTGAG 60
   AACTTCACCT TGAGCTGCAG GGTCCCGGGG GCAGGACCCC GAGCGAGCCT CACATTGACC 120
   TTGCTGCGAG GCGGCCAGGA GCTGATTCGC CGAAGTTTCG TAGGCGAGCC ACCCCGAGCT 180
   CGGGGTGCGA TGCTCACCGC CACGGTCCTG GCGCGCAGAG AGGATCACAG GGCCAATTTC 240
   TCATGCCTCG CGGAGCTTGA CCTGCGGCCA CACGGCTTGG GACTGTTTGC AAACAGCTCA 300
   GCCCCCAGAC AGCTCCGCAC GTTTGCCATG CCTCCACTTT CCCCGAGCCT TATTGCCCCA 360
   CGATTCTTAG AAGTGGGCTC AGAAAGGCCG GTGACTTGCA CTTTGGATGG ACTGTTTCCT 420
   GCCCCAGAAG CCGGGGTTTA CCTCTCTCTG GGAGATCAGA GGCTTCATCC TAATGTGACC 480
   CTCGACGGGG AGAGCCTTGT GGCCACTGCC ACAGCTACAG CAAGTGAAGA ACAGGAAGGC 540
   ACCAAACAGC TGATGTGCAT CGTGACCCTC GGGGGCGAAA GCAGGGAGAC CCAGGAAAAC 600
   CTGACTGTCT ACAGCTTCCC GGCTCCTCTT CTGACTTTAA GTGAGCCAGA AGCCCCCGAG 660
   GGAAAGATGG TGACCGTAAG CTGCTGGGCA GGGGCCCGAG CCCTTGTCAC CTTGGAGGGA 720
   ATTCCAGCTG CGGTCCCTGG GCAGCCCGCT GAGCTCCAGT TAAATGTCAC AAAGAATGAC 780
   GACAAGCGGG GCTTCTTCTG CGACGCTGCC CTCGATGTGG ACGGGGAAAC TCTGAGAAAG 840
   AACCAGAGCT CTGAGCTTCG TGTTCTGTGT GAGTGGATGT TCACTTTATC TCTGTGAATT 900
   CCAAGGACCC TCTTACCGGC CCCATCTTTA ACCTTATCGT ATCCCCTCTG CCTCATGCCC 960
   GCAGACGCAC CTCGGCTGGA TGACTTGGAC TGTCCCAGGA GCTGGACGTG GCCAGAGGGT 1020
   CCAGAGCAGA CCCTCCACTG CGAGGCCCGT GGAAACCCTG AGCCCTCCGT GCACTGTGCA 1080
   AGGCCTGACG GTGGGGCGGT GCTAGCGCTG GGCCTGTTGG GTCCAGTGAC CCGTGCCCTC 1140
   GCGGGCACTT ACCGATGTAC AGCAATCAAT GGGCAAGGCC AGGCGGTCAA GGATGTGACC 1200
   CTGACTGTGG AATATGCCCC AGCGCTGGAC AGTGTAGGCT GCCCAGAACG TATTACTTGG 1260
   CTGGAGGGGA CAGAGGCATC GCTTAGCTGT GTGGCACACG GGGTCCCACC ACCTAGCGTG 1320
   AGCTGTGTGC GCTCTGGAAA GGAGGAAGTC ATGGAAGGGC CCCTGCGTTT TGGCCGGGAG 1380
   CACGCTGGCA CTTACCGATG CGAAGCCATC AACGCCAGGG GATCAGCGGC CAAAAATGTG 1440
   GCTGTCACGG TGGAATATGG TCCCCGGAAT TC 1472
(2) INFORMATION FOR SEQ ID NO:10:
   (i) SEQUENCE CHARACTERISTICS:
      (A) LENGTH: 2550 base pairs
      (B) TYPE: nucleic acid
      (C) STRANDEDMESS: single
      (D) TOPOLOGY: linear
   (ii) MOLECULE TYPE: CDNA
   (xi) SEQUENCE DESCRIPTION: SEQ ID NO:10:
   CCTCTGCCTC CTTGGCAGCC TGTAGGTGAG AACTTCACCT TGAGCTGCAG GGTCCCGGGG     60
   GCAGGACCCC GAGCGAGCCT CACATTGACC TTGCTGCGAG GCGGCCAGGA GCTGATTCGC    120
   CGAAGTTTCG TAGGCGAGCC ACCCCGAGCT CGGGGTGCGA TGCTCACCGC CACGGTCCTG    180
   GCGCGCAGAG AGGATCACAG GGCCAATTTC TCATGCCTCG CGGAGCTTGA CCTGCGGCCA    240
   CACGGCTTGG GACTGTTTGC AAACAGCTCA GCCCCCAGAC AGCTCCGCAC GTTTGCCATG    300
   CCTCCACTTT CCCCGAGCCT TATTGCCCCA CGATTCTTAG AAGTGGGCTC AGAAAGGCCG    360
   GTGACTTGCA CTTTGGATGG ACTGTTTCCT GCCCCAGAAG CCGGGGTTTA CCTCTCTCTG    420
   GGAGATCAGA GGCTTCATCC TAATGTGACC CTCGACGGGG AGAGCCTTGT GGCCACTGCC    480
   ACAGCTACAG CAAGTGAAGA ACAGGAAGGC ACCAAACAGC TGATGTGCAT CGTGACCCTC    540
   GGGGGCGAAA GCAGGGAGAC CCAGGAAAAC CTGACTGTCT ACAGCTTCCC GGCTCCTCTT    600
   CTGACTTTAA GTGAGCCAGA AGCCCCCGAG GGAAAGATGG TGACCGTAAG CTGCTGGGCA    660
   GGGGCCCGAG CCCTTGTCAC CTTGGAGGGA ATTCCAGCTG CGGTCCCTGG GCAGCCCGCT    720
   GAGCTCCAGT TAAATGTCAC AAAGAATGAC GACAAGCGGG GCTTCTTCTG CGACGCTGCC    780
   CTCGATGTGG ACGGGGAAAC TCTGAGAAAG AACCAGAGCT CTGAGCTTCG TGTTCTGTAC    840
   GCACCTCGGC TGGATGACTT GGACTGTCCC AGGAGCTGGA CGTGGCCAGA GGGTCCAGAG    900
   CAGACCCTCC ACTGCGAGGC CCGTGGAAAC CCTGAGCCCT CCGTGCACTG TGCAAGGCCT    960
   GACGGTGGGG CGGTGCTAGC GCTGGGCCTG TTGGGTCCAG TGACCCGTGC CCTCGCGGGC   1020
   ACTTACCGAT GTACAGCAAT CAATGGGCAA GGCCAGGCGG TCAAGGATGT GACCCTGACT   1080
   GTGGAATATG CCCCAGCGCT GGACAGTGTA GGCTGCCCAG AACGTATTAC TTGGCTGGAG   1140
   GGGACAGAGG CATCGCTTAG CTGTGTGGCA CACGGGGTCC CACCACCTAG CGTGAGCTGT   1200
   GTGCGCTCTG GAAAGGAGGA AGTCATGGAA GGGCCCCTGC GTGTGGCCCG GGAGCACGCT   1260
   GGCACTTACC GATGCGAAGC CATCAACGCC AGGGGATCAG CGGCCAAAAA TGTGGCTGTC   1320
   ACGGTGGAAT ATGGTCCCAG TTTTGAGGAG TTGGGCTGCC CCAGCAACTG GACTTGGGTA   1380
   GAAGGATCTG GAAAACTGTT TTCCTGTGAA GTTGATGGGA AGCCGGAACC ACGCGTGGAG   1440
   TGCGTGGGCT CGGAGGGTGC AAGCGAAGGG GTAGTGTTGC CCCTGGTGTC CTCGAACTCT   1500
   GGTTCCAGAA ACTCTATGAC TCCTGGTAAC CTGTCACCGG GTATTTACCT CTGCAACGCC 1560
   ACCAACCGGC ATGGCTCCAC AGTCAAAACA GTCGTCGTGA GCGCGGAATC ACCGCCACAG 1620
   ATGGATGAAT CCAGTTGCCC GAGTCACCAG ACATGGCTGG AAGGAGCCGA GGCTACTGCG 1680
   CTGGCCTGCA GTGCCAGAGG CCGCCCCTCT CCACGCGTGC GCTGTTCCAG GGAAGGTGCA 1740
   GCCAGGCTGG AGAGGCTACA GGTGTCCCGA GAGGATGCGG GGACCTACCT GTGTGTGGCT 1800
   ACCAACGCGC ATGGCACGGA TTCACGGACC GTCACTGTGG GTGTGGAATA CCGGCCTGTG 1860
   GTGGCTGAGC TGGCAGCCTC GCCCCCAAGC GTGCGGCCTG GCGGAAACTT CACTCTGACC 1920
   TGCCGTGCAG AGGCCTGGCC TCCAGCCCAG ATCAGCTGGC GCGCGCCCCC GGGAGCTCTC 1980
   AACCTCGGTC TCTCCAGCAA CAACAGCACG CTGAGCGTGG CGGGTGCCAT GGGCAGCCAT 2040
   GGTGGCGAGT ATGAGTGCGC AGCCACCAAT GCGCATGGGC GCCACGCACG GCGCATCACG 2100
   GTGCGCGTGG CCGGTCCATG GCTGTGGGTC GCTGTGGGCG GTGCGGCAGG GGGCGCGGCG 2160
   CTGCTGGCCG CAGGGGCCGG CCTGGCCTTC TACGTGCAGT CCACCGCTTG CAAGAAGGGA 2220
   GAGTACAACG TCCAGGAGGC TGAGAGCTCA GGCGAGGCGG TGTGTCTCAA TGGCGCGGGC 2280
   GGGACACCGG GTGCAGAAGG CGGAGCAGAG ACCCCCGGCA CTGCCGAGTC ACCTGCAGAT 2340
   GGCGAGGTTT TCGCCATCCA GCTGACATCT TCCTGAGCCT GTATCCAGCT CCCCCAGGGG 2400
   CCTCGAAAGC ACAGGGGTGG ACGTATGTAT TGTTCACTCT CTATTTATTC AACTCCAGGG 2460
   GCGTCGTCCC CGTTTTCTAC CCATTCCCTT AATAAAGTTT TTATAGGAGA AAAAAAAAAA 2520
   AAAAAAAAAA AAAAAAAAAA AAAAAAAAAA 2550
(2) INFORMATION FOR SEQ ID NO:11:
   (i) SEQUENCE CHARACTERISTICS:
      (A) LENGTH: 222 base pairs
      (B) TYPE: nucleic acid
      (C) STRAMDEDMESS: single
      (D) TOPOLOGY: linear
   (ii) MOLECULE TYPE: cDNA
   (xi) SEQUENCE DESCRIPTION: SEQ ID NO:11:
   AATTCGATCA CTCGCGCTCC CCTCGCCTTC TGCGCTCTCC CCTCCCTGGC AGCGGCGGCA 60
   ATGCCGGGGC CTTCACCAGG GCTGCGCCGA ACGCTCCTCG GCCTCTGGGC TGCCCTGGGC 120
   CTGGGGATCC TAGGCATCTC AGCGGTCGCG CTAGAACCTT TCTGGGCGGA CCTTCAGCCC 180
   CGCGTGGCGC TCGTGGAGCG CGGGGGCTCG CTGTGGCTCA AC 222
(2) INFORMATION FOR SEQ ID NO:12:
   (i) SEQUENCE CHARACTERISTICS:
      (A) LENGTH: 292 base pairs
      (B) TYPE: nucleic acid
      (C) STRAMDBDNESS: single
      (D) TOPOLOGY: linear
   (ii) MOLECOLE TYPE: cDNA
   (xi) SEQUENCE DESCRIPTION: SEQ ID NO:12:
   TGTGGAGCTG GCACCCCTGC CTCCTTGGCA GCCGGTGGGC CAGAACTTCA CCCTGCGCTG    60
   CCAAGTGGAG GGTGGGTCGC CCCGGACCAG CCTCACGGTG GTGCTGCTTC GCTGGGAGGA   120
   GGAGCTGAGC CGGCAGCCCG CAGTGGAGGA GCCAGCGGAG GTCACTGCCA CTGTGCTGGC   180
   CAGCAGAGAC GACCACGGAG CCCCTTTCTC ATGCCGCACA GAACTGGACA TGCAGCCCCA   240
   GGGGCTGGGA CTGTTCGTGA ACACCTCAGC CCCCCGCCAG CTCCGAACCT TT           292
(2) INFORMATION FOR SEQ ID NO:13:
   (i) SEQUENCE CHARACTERISTICS:
      (A) LENGTH: 105 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:13:
   Pro Asp Arg Val Glu Leu Val Pro Leu Pro Pro Trp Gln Pro Val Gly
    1               5                  10                  15
   Glu Asn Phe Thr Leu Ser Cys Arg Val Pro Gly Ala Gly Pro Arg Ala
               20                  25                  30
   Ser Leu Thr Leu Thr Leu Leu Arg Gly Gly Gln Glu Leu Ile Arg Arg
           35                  40                  45
   Ser Phe Val Gly Glu Pro Pro Arg Ala Arg Cys Ala Met Leu Thr Ala
       50                  55                  60
   Thr Val Leu Ala Arg Arg Glu Asp His Arg Asp Asn Phe Ser Cys Leu
   65                  70                  75                  80
   Ala Glu Leu Asp Leu Arg Thr His Gly Leu Gly Leu Phe Ala Asn Ser
                   85                  90                  95
   Ser Ala Pro Arg Gln Leu Arg Thr Phe
               100                 105
(2) INFORMATION FOR SEQ ID NO:14:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: CDNA
   (xi) SEQUENCE DESCRIPTION: SEQ ID NO:14:
   GAACTCGAGG CCATGCCTCC ACTTTCC 27
(2) INFORMATION FOR SEQ ID NO:15:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:15:
   CCATAAGCTT TATTCCACCG TGACAGCCAC 30
(2) INFORMATION FOR SEQ ID NO:16:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:16:
   AACGTGCGGA GCTGTCTG 18
(2) INFORMATION FOR SEO ID NO:17:
   (i) SEQDEHCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:17:
   ACGGAATTCG AAGCCATCAA CGCCAGG 27
(2) INFORMATION FOR SEO ID NO:18:
   (i) SEQUENCE CHARACTERISTICS:
      (A) LENGTH: 27 base pairs
      (B) TYPE: nucleic acid
      (C) STRRMDEDMESS: single
      (D) TOPOLOGY: linear
   (ii) MOLECULE TYPE: cDNA
   (xi) SEQDENCE DESCRIPTION: SEQ ID NO:18:
(2) INFORMATION FOR SEQ ID NO:19:
   (i) SEQUENCE CHARACTERISTICS:
      (A) LENGTH: 27 base pairs
      (B) TYPE: nucleic acid
      (C) STRAMDEDMESS: single
      (D) TOPOLOGY: linear
   (ii) MOLECULE TYPE: cDNA
   (xi) SEQUENCE DESCRIPTION: SEQ ID NO:19:
   ATAGAATTCC TCGGGACACC TGTAGCC 27
(2) INFORMATION FOR SEQ ID NO:20:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:20:
   CARGGTGACA AGGGCTCG 18
(2) INFORMATION FOR SEQ ID NO:21:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
   TATGAATTCA GTTGAGCCAC AGCGAGC 27
(2) INFORMATION FOR SEQ ID NO:22:
   (i) SEQUENCE CHARACTERISTICS:
      (A) LENGTH: 24 base pairs
      (B) TYPE: nucleic acid
      (C) STRANDEDKESS: single
      (D) TOPOLOGY: linear
   (ii) MOLECULE TYPE: CDNA
   (xi) SEQUENCE DESCRIPTION: SEQ ID NO:22:
   CCGGGTCCTA GAGGTGGACA CGCA 24
(2) INFORMATION FOR SBQ ID NO:23:
   (i) SEQUENCE CHARACTERISTICS:
      (A) LENGTH: 24 base pairs
      (B) TYPE: nucleic acid
      (C) STRAMDEDNESS: single
      (D) TOPOLOGY: linear
   (ii) MOLBCOXiE TYPE: cDNA
   (xi) SEQUENCE DESCRIPTION: SEQ ID NO: 23:
(2) INFORMATION FOR SEQ ID NO:24:
   (i) SEQUENCE CHARACTERISTICS:
      (A) LENGTH: 992 base pairs
      (B) TYPE: nucleic acid
      (C) STRAHDEDMESS: single
      (D) TOPOLOGY: linear
   (ii) MOLECULE TYPE: cDNA
   (xi) SEQUENCE DESCRIPTION: SEQ ID NO:24:
   GCGAAAACCG GGAGACCCGG GAGAACGTGA CCATCTACAG CTTCCCGGCA CCACTCCTGA 60
   CCCTGAGCGA ACCCAGCGTC TCCGAGGGGC AGATGGTGAC AGTAACCTGC GCAGCTGGGG 120
   CCCAAGCTCT GGTCACACTG GAGGGAGTTC CAGCCGCGGT CCCGGGGCAG CCCGCCCAGC 180
   TTCAGCTAAA TGCCACCGAG AACGACGACA GACGCAGCTT CTTCTGCGAC GCCACCCTCG 240
   ATGTGGACGG GGAGACCCTG ATCAAGAACA GGAGCGCAGA GCTTCGTGTC CTATACGCTC 300
   CCCGGCTAGA CGATTCGGAC TGCCCCAGGA GTTGGACGTG GCCCGAGGGC CCAGAGCAGA 360
   CGCTGCGCTG CGAGGCCCGC GGGAACCCAG AACCCTCAGT GCACTGTGCG CGCTCCGACG 420
   GCGGGGCCGT GCTGGCTCTG GGCCTGCTGG GTCCAGTCAC TCGGGCGCTC TCAGGCACTT 480
   ACCGCTGCAA GGCGGCCAAT GATCAAGGCG AGGCGGTCAA GGACGTAACG CTAACGGTGG 540
   AGTACGCACC AGCGCTGGAC AGCGTGGGCT GCCCAGAACG CATTACTTGG CTGGAGGGAA 600
   CAGAAGCCTC GCTGAGCTGT GTGGCGCACG GGGTACCGCC GCCTGATGTG ATCTGCGTGC 660
   GCTCTGGAGA ACTCGGGGCC GTCATCGAGG GGCTGTTGCG TGTGGCCCGG GAGCATGCGG 720
   GCACTTACCG CTGCGAAGCC ACCAACCCTC GGGGCTCTGC GGCCAAAAAT GTGGCCGTCA 780
   CGGTGGAATA TGGCCCCAGG TTTGAGGAGC CGAGCTGCCC CAGCAATTGG ACATGGGTGG 840
   AAGGATCTGG GCGCCTGTTT TCCTGTGAGG TCGATGGGAA GCCACAGCCA AGCGTGAAGT 900
   GCGTGGGCTC CGGGGGCACC ACTGAGGGGG TGCTGCTGCC GCTGGCACCC CCAGACCCTA 960
   GTCCCAGAGC TCCCAGAATC CCTAGAGTCC TG 992
(2) INFORMATION FOR SEQ ID NO:25:
   (i) SEQUENCE CHARACTERISTICS:
      (A) LENGTH: 2775 base pairs
      (B) TYPE: nucleic acid
      (C) STRANDEDNESS: single
      (D) TOPOLOGY: linear
   (ii) MOLECDLE TYPE: CDNA
   (xi) SEQDSNCE DESCRIPTION: SEQ ID NO:25:
(2) INFORMATION FOR SEQ ID NO:26:
   (i) SEQDENCE CHARACTERISTICS:
      (A) LENGTH: 1557 base pairs
      (B) TYPE: nucleic acid
      (C) STRANDEDKESS: single
      (D) TOPOLOGY: linear
   (ii) MOLECULE TYPE: cDNA
   (xi) SEQUENCE DESCRIPTION: SEQ ID NO:26:
   CGCGCTCTCC TCGCCTCCTG TGCTTTCCCC GCCGCGGCGA TGCCAGGGCC TTCGCCAGGG      60
   CTGCGCCGGG CGCTACTCGG CCTCTGGGCT GCTCTGGGCC TGGGGCTCTT CGGCCTCTCA     120
   GCGGTCTCGC AGGAGCCCTT CTGGGCGGAC CTGCAGCCTC GCGTGGCGTT CGTGGAGCGC     180
   GGGGGCTCGC TGTGGCTGAA TTGCAGCACC AACTGCCCTC GGCCGGAGCG CGGTGGCCTG     240
   GAGACCTCGC TGCGCCGAAA CGGGACCCAG AGGGGTTTGC GTTGGTTGGC GCGGCAGCTG     300
   GTGGACATTC GCGAGCCGGA GACTCAGCCC GTCTGCTTCT TCCGCTGCGC GCGGCGCACA     360
   CTACAGGCGC GTGGGCTCAT TCGCACTTTC CAGCGACCAG ATCGCGTAGA GCTGATGCCG     420
   CTGCCTCCCT GGCAGCCGGT GGGCGAGAAC TTCACCCTGA GCTGTAGGGT CCCCGGCGCC     480
   GGGCCCCGTG CGAGCCTCAC GCTGACCCTG CTGCGGGGCG CCCAGGAGCT GATCCGCCGC     540
   AGCTTCGCCG GTGAACCACC CCGAGCGCGG GGCGCGGTGC TCACAGCCAC GGTACTGGCT     600
   CGGAGGGAGG ACCATGGAGC CAATTTCTCG TGTCGCGCCG AGCTGGACCT GCGGCCGCAC     660
   GGACTGGGAC TGTTTGAAAA CAGCTCGGCC CCCAGAGAGC TCCGAACCTT CTCCCTGTCT     720
   CCGGATGCCC CGCGCCTCGC TGCTCCCCGG CTCTTGGAAG TTGGCTCGGA AAGGCCCGTG     780
   AGCTGCACTC TGGACGGACT GTTTCCAGCC TCAGAGGCCA GGGTCTACCT CGCACTGGGG     840
   GACCAGAATC TGAGTCCTGA TGTCACCCTC GAAGGGGACG CATTCGTGGC CACTGCCACA     900
   GCCACAGCTA GCGCAGAGCA GGAGGGTGCC AGGCAGCTGG TCTGCAACGT CACCCTGGGG     960
   GGCGAAAACC GGGAGACCCG GGAGAACGTG ACCATCTACA GCTTCCCGGC ACCACTCCTG    1020
   ACCCTGAGCG AACCCAGCGT CTCCGAGGGG CAGATGGTGA CAGTAACCTG CGCAGCTGGG    1080
   GCCCAAGCTC TGGTCACACT GGAGGGAGTT CCAGCCGCGG TCCCGGGGCA GCCCGCCCAG    1140
   CTTCAGCTAA ATGCCACCGA GAACGACGAC AGACGCAGCT TCTTCTGCGA CGCCACCCTC    1200
   GATGTGGACG GGGAGACCCT GATCAAGAAC AGGAGCGCAG AGCTTCGTGT CCTATACGCT    1260
   CCCCGGCTAG ACGATTCGGA CTGCCCCAGG AGTTGGACGT GGCCCGAGGG CCCAGAGCAG    1320
   ACGCTGCGCT GCGAGGCCCG CGGGAACCCA GAACCCTCAG TGCACTGTGC GCGCTCCGAC    1380
   GGCGGGGCCG TGCTGGCTCT GGGCCTGCTG GGTCCAGTCA CTCGGGCGCT CTCAGGCACT    1440
   TACCGCTGCA AGGCGGCCAA TGATCAAGGC GAGGCGGTCA AGGACGTAAC GCTAACGGTG    1500
   GAGTACGCAC CAGCGCTGGA CAGCGTGGGC TGCCCAGAAC GCATTACTTG GCTGGAG       1557
(2) INFORMATION FOR SEQ ID NO:27:
   (i) SEQUENCE CHARACTERISTICS:
      (A) LENGTH: 2927 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 40..2814
   (xi) SEQUENCE DESCRIPTION: SEQ ID NO:27:
   CGCGCTCTCC TCGCCTCCTG TGCTTTCCCC GCCGCGGCG ATG CCA GGG CCT TCG      54
                                             Met Pro Gly Pro Ser
                                              1               5
   CCA GGG CTG CGC CGG GCG CTA CTC GGC CTC TGG GCT GCT CTG GGC CTG     102
   Pro Gly Leu Arg Arg Ala Leu Leu Gly Leu Trp Ala Ala Leu Gly Leu
                    10                  15                  20
   GGG CTC TTC GGC CTC TCA GCG GTC TCG CAG GAG CCC TTC TGG GCG GAC     150
   Gly Leu Phe Gly Leu Ser Ala Val Ser Gln Glu Pro Phe Trp Ala Asp
                25                  30                  35
   CTG CAG CCT CGC GTG GCG TTC GTG GAG CGC GGG GGC TCG CTG TGG CTG     198
   Leu Gln Pro Arg Val Ala Phe Val Glu Arg Gly Gly Ser Leu Trp Leu
            40                  45                  50
   AAT TGC AGC ACC AAC TGC CCT CGG CCG GAG CGC GGT GGC CTG GAG ACC     246
   Asn Cys Ser Thr Asn Cys Pro Arg Pro Glu Arg Gly Gly Leu Glu Thr
        55                  60                  65
   TCG CTG CGC CGA AAC GGG ACC CAG AGG GGT TTG CGT TGG TTG GCG CGG     294
   Ser Leu Arg Arg Asn Gly Thr Gln Arg Gly Leu Arg Trp Leu Ala Arg
    70                  75                  80                  85
   CAG CTG GTG GAC ATT CGC GAG CCG GAG ACT CAG CCC GTC TGC TTC TTC      342
   Gln Leu Val Asp Ile Arg Glu Pro Glu Thr Gln Pro Val Cys Phe Phe
                90                  95                  100
   CGC TGC GCG CGG CGC ACA CTA CAG GCG CGT GGG CTC ATT CGC ACT TTC      390
   Arg Cys Ala Arg Arg Thr Leu Gln Ala Arg Gly Leu Ile Arg Thr Phe
               105                 110                 115
   CAG CGA CCA GAT CGC GTA GAG CTG ATG CCG CTG CCT CCC TGG CAG CCG      438
   Gln Arg Pro Asp Arg Val Glu Leu Met Pro Leu Pro Pro Trp Gln Pro
           120                 125                 130
   GTG GGC GAG AAC TTC ACC CTG AGC TGT AGG GTC CCC GGC GCC GGG CCC      486
   Val Gly Glu Asn Phe Thr Leu Ser Cys Arg Val Pro Gly Ala Gly Pro
       135                 140                 145
   CGT GCG AGC CTC ACG CTG ACC CTG CTG CGG GGC GCC CAG GAG CTG ATC      534
   Arg Ala Ser Leu Thr Leu Thr Leu Leu Arg Gly Ala Gln Glu Leu Ile
   150                 155                 160                 165
   CGC CGC AGC TTC GCC GGT GAA CCA CCC CGA GCG CGG GGC GCG GTG CTC      582
   Arg Arg Ser Phe Ala Gly Glu Pro Pro Arg Ala Arg Gly Ala Val Leu
                   170                 175                 180
   ACA GCC ACG GTA CTG GCT CGG AGG GAG GAC CAT GGA GCC AAT TTC TCG      630
   Thr Ala Thr Val Leu Ala Arg Arg Glu Asp His Gly Ala Asn Phe Ser
               185                 190                 195
   TGT CGC GCC GAG CTG GAC CTG CGG CCG CAC GGA CTG GGA CTG TTT GAA      678
   Cys Arg Ala Glu Leu Asp Leu Arg Pro His Gly Leu Gly Leu Phe Glu
           200                 205                 210
   AAC AGC TCG GCC CCC AGA GAG CTC CGA ACC TTC TCC CTG TCT CCG GAT      726
   Asn Ser Ser Ala Pro Arg Glu Leu Arg Thr Phe Ser Leu Ser Pro Asp
       215                 220                 225
   GCC CCG CGC CTC GCT GCT CCC CGG CTC TTG GAA GTT GGC TCG GAA AGG      774
   Ala Pro Arg Leu Ala Ala Pro Arg Leu Leu Glu Val Gly Ser Glu Arg
   230                 235                 240                 245
   CCC GTG AGC TGC ACT CTG GAC GGA CTG TTT CCA GCC TCA GAG GCC AGG      822
   Pro Val Ser Cys Thr Leu Asp Gly Leu Phe Pro Ala Ser Glu Ala Arg
                   250                 255                 260
   GTC TAC CTC GCA CTG GGG GAC CAG AAT CTG AGT CCT GAT GTC ACC CTC      870
   Val Tyr Leu Ala Leu Gly Asp Gln Asn Leu Ser Pro Asp Val Thr Leu
               265                 270                 275
   GAA GGG GAC GCA TTC GTG GCC ACT GCC ACA GCC ACA GCT AGC GCA GAG      918
   Glu Gly Asp Ala Phe Val Ala Thr Ala Thr Ala Thr Ala Ser Ala Glu
           280                 285                 290
   CAG GAG GGT GCC AGG CAG CTG GTC TGC AAC GTC ACC CTG GGG GGC GAA      966
   Gln Glu Gly Ala Arg Gln Leu Val Cys Asn Val Thr Leu Gly Gly Glu
       295                 300                 305
   AAC CGG GAG ACC CGG GAG AAC GTG ACC ATC TAC AGC TTC CCG GCA CCA     1014
   Asn Arg Glu Thr Arg Glu Asn Val Thr Ile Tyr Ser Phe Pro Ala Pro
   310                 315                 320                 325
   CTC CTG ACC CTG AGC GAA CCC AGC GTC TCC GAG GGG CAG ATG GTG ACA     1062
   Leu Leu Thr Leu Ser Glu Pro Ser Val Ser Glu Gly Gln Met Val Thr
                   330                 335                 340
   GTA ACC TGC GCA GCT GGG GCC CAA GCT CTG GTC ACA CTG GAG GGA GTT      1110
   Val Thr Cys Ala Ala Gly Ala Gln Ala Leu Val Thr Leu Glu Gly Val
               345                 350                 355
   CCA GCC GCG GTC CCG GGG CAG CCC GCC CAG CTT CAG CTA AAT GCC ACC      1158
   Pro Ala Ala Val Pro Gly Gln Pro Ala Gln Leu Gln Leu Asn Ala Thr
           360                 365                 370
   GAG AAC GAC GAC AGA CGC AGC TTC TTC TGC GAC GCC ACC CTC GAT GTG      1206
   Glu Asn Asp Asp Arg Arg Ser Phe Phe Cys Asp Ala Thr Leu Asp Val
       375                 380                 385
   GAC GGG GAG ACC CTG ATC AAG AAC AGG AGC GCA GAG CTT CGT GTC CTA      1254
   Asp Gly Glu Thr Leu Ile Lys Asn Arg Ser Ala Glu Leu Arg Val Leu
   390                 395                 400                 405
   TAC GCT CCC CGG CTA GAC GAT TCG GAC TGC CCC AGG AGT TGG ACG TGG      1302
   Tyr Ala Pro Arg Leu Asp Asp Ser Asp Cys Pro Arg Ser Trp Thr Trp
                   410                 415                 420
   CCC GAG GGC CCA GAG CAG ACG CTG CGC TGC GAG GCC CGC GGG AAC CCA      1350
   Pro Glu Gly Pro Glu Gln Thr Leu Arg Cys Glu Ala Arg Gly Asn Pro
               425                 430                 435
   GAA CCC TCA GTG CAC TGT GCG CGC TCC GAC GGC GGG GCC GTG CTG GCT      1398
   Glu Pro Ser Val His Cys Ala Arg Ser Asp Gly Gly Ala Val Leu Ala
           440                 445                 450
   CTG GGC CTG CTG GGT CCA GTC ACT CGG GCG CTC TCA GGC ACT TAC CGC      1446
   Leu Gly Leu Leu Gly Pro Val Thr Arg Ala Leu Ser Gly Thr Tyr Arg
       455                 460                 465
   TGC AAG GCG GCC AAT GAT CAA GGC GAG GCG GTC AAG GAC GTA ACG CTA      1494
   Cys Lys Ala Ala Asn Asp Gln Gly Glu Ala Val Lys Asp Val Thr Leu
   470                 475                 480                 485
   ACG GTG GAG TAC GCA CCA GCG CTG GAC AGC GTG GGC TGC CCA GAA CGC      1542
   Thr Val Glu Tyr Ala Pro Ala Leu Asp Ser Val Gly Cys Pro Glu Arg
                   490                 495                 500
   ATT ACT TGG CTG GAG GGA ACA GAA GCC TCG CTG AGC TGT GTG GCG CAC      1590
   Ile Thr Trp Leu Glu Gly Thr Glu Ala Ser Leu Ser Cys Val Ala His
               505                 510                 515
   GGG GTA CCG CCG CCT GAT GTG ATC TGC GTG CGC TCT GGA GAA CTC GGG      1638
   Gly Val Pro Pro Pro Asp Val Ile Cys Val Arg Ser Gly Glu Leu Gly
           520                 525                 530
   GCC GTC ATC GAG GGG CTG TTG CGT GTG GCC CGG GAG CAT GCG GGC ACT      1686
   Ala Val Ile Glu Gly Leu Leu Arg Val Ala Arg Glu His Ala Gly Thr
       535                 540                 545
   TAC CGC TGC GAA GCC ACC AAC CCT CGG GGC TCT GCG GCC AAA AAT GTG      1734
   Tyr Arg Cys Glu Ala Thr Asn Pro Arg Gly Ser Ala Ala Lys Asn Val
   550                 555                 560                 565
   GCC GTC ACG GTG GAA TAT GGC CCC AGG TTT GAG GAG CCG AGC TGC CCC      1782
   Ala Val Thr Val Glu Tyr Gly Pro Arg Phe Glu Glu Pro Ser Cys Pro
                   570                 575                 580
   AGC AAT TGG ACA TGG GTG GAA GGA TCT GGG CGC CTG TTT TCC TGT GAG      1830
   Ser Asn Trp Thr Trp Val Glu Gly Ser Gly Arg Leu Phe Ser Cys Glu
               585                 590                 595
   GTC GAT GGG AAG CCA CAG CCA AGC GTG AAG TGC GTG GGC TCC GGG GGC        1878
   Val Asp Gly Lys Pro Gln Pro Ser Val Lys Cys Val Gly Ser Gly Gly
           600                 605                 610
   ACC ACT GAG GGG GTG CTG CTG CCG CTG GCA CCC CCA GAC CCT AGT CCC        1926
   Thr Thr Glu Gly Val Leu Leu Pro Leu Ala Pro Pro Asp Pro Ser Pro
       615                 620                 625
   AGA GCT CCC AGA ATC CCT AGA GTC CTG GCA CCC GGT ATC TAC GTC TGC        1974
   Arg Ala Pro Arg Ile Pro Arg Val Leu Ala Pro Gly Ile Tyr Val Cys
   630                 635                 640                 645
   AAC GCC ACC AAC CGC CAC GGC TCC GTG GCC AAA ACA GTC GTC GTG AGC        2022
   Asn Ala Thr Asn Arg His Gly Ser Val Ala Lys Thr Val Val Val Ser
                   650                 655                 660
   GCG GAG TCG CCA CCG GAG ATG GAT GAA TCT ACC TGC CCA AGT CAC CAG        2070
   Ala Glu Ser Pro Pro Glu Met Asp Glu Ser Thr Cys Pro Ser His Gln
               665                 670                 675
   ACG TGG CTG GAA GGG GCT GAG GCT TCC GCG CTG GCC TGC GCC GCC CGG        2118
   Thr Trp Leu Glu Gly Ala Glu Ala Ser Ala Leu Ala Cys Ala Ala Arg
           680                 685                 690
   GGT CGC CCT TCC CCA GGA GTG CGC TGC TCT CGG GAA GGC ATC CCA TGG        2166
   Gly Arg Pro Ser Pro Gly Val Arg Cys Ser Arg Glu Gly Ile Pro Trp
       695                 700                 705
   CCT GAG CAG CAG CGC GTG TCC CGA GAG GAC GCG GGC ACT TAC CAC TGT        2214
   Pro Glu Gln Gln Arg Val Ser Arg Glu Asp Ala Gly Thr Tyr His Cys
   710                 715                 720                 725
   GTG GCC ACC AAT GCG CAT GGC ACG GAC TCC CGG ACC GTC ACT GTG GGC        2262
   Val Ala Thr Asn Ala His Gly Thr Asp Ser Arg Thr Val Thr Val Gly
                   730                 735                 740
   GTG GAA TAC CGG CCA GTG GTG GCC GAA CTT GCT GCC TCG CCC CCT GGA        2310
   Val Glu Tyr Arg Pro Val Val Ala Glu Leu Ala Ala Ser Pro Pro Gly
               745                 750                 755
   GGC GTG CGC CCA GGA GGA AAC TTC ACG TTG ACC TGC CGC GCG GAG GCC        2358
   Gly Val Arg Pro Gly Gly Asn Phe Thr Leu Thr Cys Arg Ala Glu Ala
           760                 765                 770
   TGG CCT CCA GCC CAG ATC AGC TGG CGC GCG CCC CCG AGG GCC CTC AAC        2406
   Trp Pro Pro Ala Gln Ile Ser Trp Arg Ala Pro Pro Arg Ala Leu Asn
       775                 780                 785
   ATC GGC CTG TCG AGC AAC AAC AGC ACA CTG AGC GTG GCA GGC GCC ATG        2454
   Ile Gly Leu Ser Ser Asn Asn Ser Thr Leu Ser Val Ala Gly Ala Met
   790                 795                 800                 805
   GGA AGC CAC GGC GGC GAG TAC GAG TGC GCA CGC ACC AAC GCG CAC GGG        2502
   Gly Ser His Gly Gly Glu Tyr Glu Cys Ala Arg Thr Asn Ala His Gly
                   810                 815                 820
   CGC CAC GCG CGG CGC ATC ACG GTG CGC GTG GCC GGT CCG TGG CTA TGG        2550
   Arg His Ala Arg Arg Ile Thr Val Arg Val Ala Gly Pro Trp Leu Trp
               825                 830                 835
   GTC GCC GTG GGC GGC GCG GCG GGG GGC GCG GCG CTG CTG GCC GCG GGG        2598
   Val Ala Val Gly Gly Ala Ala Gly Gly Ala Ala Leu Leu Ala Ala Gly
           840                 845                 850
   GCC GGC CTG GCC TTC TAC GTG CAG TCC ACC GCC TGC AAG AAG GGC GAG      2646
   Ala Gly Leu Ala Phe Tyr Val Gln Ser Thr Ala Cys Lys Lys Gly Glu
       855                 860                 865
   TAC AAC GTG CAG GAG GCC GAG AGC TCA GGC GAG GCC GTG TGT CTG AAC      2694
   Tyr Asn Val Gln Glu Ala Glu Ser Ser Gly Glu Ala Val Cys Leu Asn
   870                 875                 880                 885
   GGA GCG GGC GGC GGC GCT GGC GGG GCG GCA GGC GCG GAG GGC GGA CCC      2742
   Gly Ala Gly Gly Gly Ala Gly Gly Ala Ala Gly Ala Glu Gly Gly Pro
                   890                 895                 900
   GAG GCG GCG GGG GGC GCG GCC GAG TCG CCG GCG GAG GGC GAG GTC TTC      2790
   Glu Ala Ala Gly Gly Ala Ala Glu Ser Pro Ala Glu Gly Glu Val Phe
               905                 910                 915
   GCC ATA CAG CTG ACA TCG GCG TGAGCCCGCT CCCCTCTCCG CGGGCCGGGA         2841
   Ala Ile Gln Leu Thr Ser Ala
           920                 925
   CGCCCCCCAG ACTCACACGG GGGCTTATTT ATTGCTTTAT TTATTTACTT ATTCATTTAT    2901
   TTATGTATTC AACTCCAAGG GAATTC                                         2927
(2) INFORMATION FOR SEQ ID NO:28:
   (i) SEQUENCE CHARACTERISTICS:
      (A) LENGTH: 924 amino acids
      (B) TYPE: amino acid
      (D) TOPOLOGY: linear
   (ii) MOLECULE TYPE: protein
   (xi) SEQUENCE DESCRIPTION: SEQ ID NO:28:
   Met Pro Gly Pro Ser Pro Gly Leu Arg Arg Ala Leu Leu Gly Leu Trp
   1 5 10 15
   Ala Ala Leu Gly Leu Gly Leu Phe Gly Leu Ser Ala Val Ser Gln Glu
   20 25 30
   Pro Phe Trp Ala Asp Leu Gln Pro Arg Val Ala Phe Val Glu Arg Gly
   35 40 45
   Gly Ser Leu Trp Leu Asn Cys Ser Thr Asn Cys Pro Arg Pro Glu Arg
   50 55 60
   Gly Gly Leu Glu Thr Ser Leu Arg Arg Asn Gly Thr Gln Arg Gly Leu
   65 70 75 80
   Arg Trp Leu Ala Arg Gln Leu Val Asp Ile Arg Glu Pro Glu Thr Gln
   85 90 95
   Pro Val Cys Phe Phe Arg Cys Ala Arg Arg Thr Leu Gln Ala Arg Gly
   100 105 110
   Leu Ile Arg Thr Phe Gln Arg Pro Asp Arg Val Glu Leu Met Pro Leu
   115 120 125
   Pro Pro Trp Gln Pro Val Gly Glu Asn Phe Thr Leu Ser Cys Arg Val
   130 135 140
   Pro Gly Ala Gly Pro Arg Ala Ser Leu Thr Leu Thr Leu Leu Arg Gly
   145 150 155 160
   Ala Gln Glu Leu Ile Arg Arg Ser Phe Ala Gly Glu Pro Pro Arg Ala
   165 170 175
   Arg Gly Ala Val Leu Thr Ala Thr Val Leu Ala Arg Arg Glu Asp His
   180 185 190
   Gly Ala Asn Phe Ser Cys Arg Ala Glu Leu Asp Leu Arg Pro His Gly
   195 200 205
   Leu Gly Leu Phe Glu Asn Ser Ser Ala Pro Arg Glu Leu Arg Thr Phe
   210 215 220
   Ser Leu Ser Pro Asp Ala Pro Arg Leu Ala Ala Pro Arg Leu Leu Glu
   225 230 235 240
   Val Gly Ser Glu Arg Pro Val Ser Cys Thr Leu Asp Gly Leu Phe Pro
   245 250 255
   Ala Ser Glu Ala Arg Val Tyr Leu Ala Leu Gly Asp Gln Asn Leu Ser
   260 265 270
   Pro Asp Val Thr Leu Glu Gly Asp Ala Phe Val Ala Thr Ala Thr Ala
   275 280 285
   Thr Ala Ser Ala Glu Gln Glu Gly Ala Arg Gln Leu Val Cys Asn Val
   290 295 300
   Thr Leu Gly Gly Glu Asn Arg Glu Thr Arg Glu Asn Val Thr Ile Tyr
   305 310 315 320
   Ser Phe Pro Ala Pro Leu Leu Thr Leu Ser Glu Pro Ser Val Ser Glu
   325 330 335
   Gly Gln Met Val Thr Val Thr Cys Ala Ala Gly Ala Gln Ala Leu Val
   340 345 350
   Thr Leu Glu Gly Val Pro Ala Ala Val Pro Gly Gln Pro Ala Gln Leu
   355 360 365
   Gln Leu Asn Ala Thr Glu Asn Asp Asp Arg Arg Ser Phe Phe Cys Asp
   370 375 380
   Ala Thr Leu Asp Val Asp Gly Glu Thr Leu Ile Lys Asn Arg Ser Ala
   385 390 395 400
   Glu Leu Arg Val Leu Tyr Ala Pro Arg Leu Asp Asp Ser Asp Cys Pro
   405 410 415
   Arg Ser Trp Thr Trp Pro Glu Gly Pro Glu Gln Thr Leu Arg Cys Glu
   420 425 430
   Ala Arg Gly Asn Pro Glu Pro Ser Val His Cys Ala Arg Ser Asp Gly
   435 440 445
   Gly Ala Val Leu Ala Leu Gly Leu Leu Gly Pro Val Thr Arg Ala Leu
   450 455 460
   Ser Gly Thr Tyr Arg Cys Lys Ala Ala Asn Asp Gln Gly Glu Ala Val
   465 470 475 480
   Lys Asp Val Thr Leu Thr Val Glu Tyr Ala Pro Ala Leu Asp Ser Val
   485 490 495
   Gly Cys Pro Glu Arg Ile Thr Trp Leu Glu Gly Thr Glu Ala Ser Leu
   500 505 510
   Ser Cys Val Ala His Gly Val Pro Pro Pro Asp Val Ile Cys Val Arg
   515 520 525
   Ser Gly Glu Leu Gly Ala Val Ile Glu Gly Leu Leu Arg Val Ala Arg
   530 535 540
   Glu His Ala Gly Thr Tyr Arg Cys Glu Ala Thr Asn Pro Arg Gly Ser
   545 550 555 560
   Ala Ala Lys Asn Val Ala Val Thr Val Glu Tyr Gly Pro Arg Phe Glu
   565 570 575
   Glu Pro Ser Cys Pro Ser Asn Trp Thr Trp Val Glu Gly Ser Gly Arg
   580 585 590
   Leu Phe Ser Cys Glu Val Asp Gly Lys Pro Gln Pro Ser Val Lys Cys
   595 600 605
   Val Gly Ser Gly Gly Thr Thr Glu Gly Val Leu Leu Pro Leu Ala Pro
   610 615 620
   Pro Asp Pro Ser Pro Arg Ala Pro Arg Ile Pro Arg Val Leu Ala Pro
   625 630 635 640
   Gly Ile Tyr Val Cys Asn Ala Thr Asn Arg His Gly Ser Val Ala Lys
   645 650 655
   Thr Val Val Val Ser Ala Glu Ser Pro Pro Glu Met Asp Glu Ser Thr
   660 665 670
   Cys Pro Ser His Gln Thr Trp Leu Glu Gly Ala Glu Ala Ser Ala Leu
   675 680 685
   Ala Cys Ala Ala Arg Gly Arg Pro Ser Pro Gly Val Arg Cys Ser Arg
   690 695 700
   Glu Gly Ile Pro Trp Pro Glu Gln Gln Arg Val Ser Arg Glu Asp Ala
   705 710 715 720
   Gly Thr Tyr His Cys Val Ala Thr Asn Ala His Gly Thr Asp Ser Arg
   725 730 735
   Thr Val Thr Val Gly Val Glu Tyr Arg Pro Val Val Ala Glu Leu Ala
   740 745 750
   Ala Ser Pro Pro Gly Gly Val Arg Pro Gly Gly Asn Phe Thr Leu Thr
   755 760 765
   Cys Arg Ala Glu Ala Trp Pro Pro Ala Gln Ile Ser Trp Arg Ala Pro
   770 775 780
   Pro Arg Ala Leu Asn Ile Gly Leu Ser Ser Asn Asn Ser Thr Leu Ser
   785 790 795 800
   Val Ala Gly Ala Met Gly Ser His Gly Gly Glu Tyr Glu Cys Ala Arg
   805 810 815
   Thr Asn Ala His Gly Arg His Ala Arg Arg Ile Thr Val Arg Val Ala
   820 825 830
   Gly Pro Trp Leu Trp Val Ala Val Gly Gly Ala Ala Gly Gly Ala Ala
   835 840 845
   Leu Leu Ala Ala Gly Ala Gly Leu Ala Phe Tyr Val Gln Ser Thr Ala
   850 855 860
   Cys Lys Lys Gly Glu Tyr Asn Val Gln Glu Ala Glu Ser Ser Gly Glu
   865 870 875 880
   Ala Val Cys Leu Asn Gly Ala Gly Gly Gly Ala Gly Gly Ala Ala Gly
   885 890 895
   Ala Glu Gly Gly Pro Glu Ala Ala Gly Gly Ala Ala Glu Ser Pro Ala
   900 905 910
   Glu Gly Glu Val Phe Ala Ile Gln Leu Thr Ser Ala
   915 920
(2) INFORMATION FOR SEQ ID NO:29:
   (i) SEQUENCE CHARACTERISTICS:
      (A) LENGTH: 65 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:29:
   GTACTTACAG GATCCGCGGT CTCGCAGGAG CCCTTCTGGG CGGACCTACA GCCTGCGTGG 60
   CGTTC 65
(2) INFORMATION FOR SEQ ID NO:30:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:30:
   ATTTCTCTCG AGGATGGTCA CGTTCTCCCG G 31
(2) INFORMATION FOR SEQ ID NO:31:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:31:
   ATTTCTGGAT CCTACAGCTT CCCGGCACCA CTC 33
(2) INFORMATION FOR SEQ ID NO:32:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:32:
   ATTTCTCTCG AGTTCCACGC CCACAGTGAC GG 32
(2) INFORMATION FOR SEQ ID NO:33:
   (i) SEQUENCE CHARACTERISTICS
      (A) LENGTH: 1687 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:33:
   GGATCCTTTG AGCCCTGAAA GTCGAGGTTG CAGTGAGCCT TGATCGTGCC ACTGCACTCC 60
   AGCCTGGGGG ACAGAGCACG ACCCTGTCTC CAAAAATAAA ATAAAAATAA AAATAAATAT 120
   TGGCGGGGGA ACCCTCTGGA ATCAATAAAG GCTTCCTTAA CCAGCCTCTG TCCTGTGACC 180
   TAAGGGTCCG CATTACTGCC CTTCTTCGGA GGAACTGGTT TGTTTTTGTT GTTGTTGTTG 240
   TTTTTGCGAT CACTTTCTCC AAGTTCCTTG TCTCCCTGAG GGCACCTGAG GTTTCCTCAC 300
   TCAGGGCCCA CCTGGGGTCC CGAAGCCCCA GACTCTGTGT ATCCCCAGCG GGTGTCACAG 360
   AAACCTCTCC TTCTGCTGGC CTTATCGAGT GGGATCAGCG CGGCCGGGGA GAGCCACGGG 420
   CAGGGGCGGG GTGGGGTTCA TGGTATGGCT TTCCTGATTG GCGCCGCCGC CACCACGCGG 480
   CAGCTCTGAT TGGATGTTAA GTTTCCTATC CCAGCCCCAC CTTCAGACCC TGTGCTTTCC 540
   TGGAGGCCAA ACAACTGTGG AGCGAGAACT CATCTCCAAA ATAACTTACC ACGCTGGAGT 600
   GAGACCACGA ATGGTGGGGA GGGGAGGGTC CCACGGACAT ATTGAGGGAC GTGGATACGC 660
   AGAAGAGGTA TCCATGTGGT GGCAGCCGGG AAGGGGTGAT CAGATGGTCC ACAGGGAATA 720
   TCACAAACTC GAATTCTGAC GATGTTCTGG TAGTCACCCA GCCAGATGAG CGCATGGAGT 780
   TGGCGGTGGG GGGTGTCAAA GCTTGGGGCC CGGAAGCGGA GTCAAAAGCA TCACCCTCGG 840
   TCCCTTGTTC TCGCGTGGAT GTCAGGGCCT CCACCCACCG AGCAGAAGGC GGACTCAGGG 900
   GCGCTCCAGG GTGGCTCGAG CTCACACACG CTGAGTAGAC ACGTGCCCGC TGCACCCTGG 960
   GTAAATACAG ACCCGGAGCC GAGCGGATTC TAATTTAGAC GCCCGCGAAC GCTGCGCGCA 1020
   CGCACACGTG TCCTCGGCTC GCTGGCACTT TCGTCCCGCC CCCTCCGTCG CGTGCCGGAG 1080
   CTGACCCGGA GGGGTGCTTA GAGGTATGGC TCCGCGGGGT CAAAAGGAGA AGGATCAGTG 1140
   AGAGAGGATC CCCACACCCT CCCCTAGAAC TGTCCTTTCC CCATCCAGTG CCTCCCAAAT 1200
   CTCTCTTAGT CCCCAAATGT ATCCCCGCCC TAAGGGGCGC TGGTGGGAGG AGCTAAATGT 1260
   GGGGGCGGAG CTCGGAGTCC AGCTTATTAT CATGGCATCT CAGCCAGGGC TGGGGTAGGG 1320
   GTTTGGGAAG GGCAACCCAG CATCCCCCGA TCCCAGAGTC GCGGCCGGGG ATGACGCGAG 1380
   AGAGCGTGGT CGCCCCCAGA AGGCCCTGGG CCATCATGCC GGCCTCCACG TAGACCCCAG 1440
   GGGTCGCTCA CTCCTGCCAG CTCGCCTTCA CCAAGGCCAG GAGCTTAGCG CACGCTCGCC 1500
   TCCCGCCCCC CCGCCGCCTC TGCCGCCGCC CCCTCCTTGG AAACCAAGTT ACCAACGTTA 1560
   AACCAATCCC CAAGCGCAAC TCTGTCTCCC CCACACCCCA CCCGCCGCGC CGCGCGGAGC 1620
   CGTCCTCTAG CCCAGCTCCT CGGCTCGCGC TCTCCTCGCC TCCTGTGCTT TCCCCGCCGC 1680
   GGCGATG 1687
(2) INFORMATION FOR SEQ ID NO:34:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:34:
   CAGAACTAAG CTTACAGGAG GCGAGGAGAG CGCGAG 36
(2) INFORMATION FOR SEQ ID NO: 35;
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:35:
   CAACAATGCT AGCCAAGCGC AACTCTGTCT C 31
(2) INFORMATION FOR SEQ ID NO:36
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:36:
   CAACAATGCT AGCCTTGGAA ACCAAGTTAC C 31
(2) INFORMATION FOR SEQ ID NO:37:
   (i) SEQUENCE CHARACTERISTICS:
      (A) LENGTH: 33 base pairs
      (B) TYPE: nucleic acid
      (C) STOAHDEDNESS: single
      (D) TOPOLOGY: linear
   (ii) MOLECULE TYPE: DNA
   (xi) SEQUENCE DESCRIPTION: SEQ ID NO:37:
   CAACAATGCT AGCAGGAGCT TAGCGCACGC TCG 33
(2) INFORMATION FOR SEQ ID NO:38:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:38:
   CAACAATGCT AGCCATGCCG GCCTCCACGT AG 32
(2) INFORMATION FOR SEQ ID NO:39:
   (i) SEQUENCE CHARACTERISTICS:
      (A) LENGTH: 31 base pairs
      (B) TYPE: nucleic acid
      (C) STRAKDEDNESS: single
      (D) TOPOLOGY: linear
   (ii) MOLECULE TYPE: DNA
   (xi) SEQUENCE DESCRIPTION: SEQ ID NO:39:
   CAACAATGCT AGCGTCCAGC TTATTATCAT G 31
(2) INFORMATION FOR SEQ ID NO:40:
   (i) SEQUENCE CHARACTERISTICS:
      (A) LENGTH: 32 base pairs
      (B) TYPE: nucleic acid
      (C) STRAHDEDNESS: single
      (D) TOPOLOGY: linear
   (ii) MOLECULE TYPE: DNA
   (xi) SEQUENCE DESCRIPTION: SEQ ID NO:40:
   CAACAATGCT AGCCTTAGTC CCCAAATGTA TC 32
(2) INFORMATION FOR SEQ ID NO:41:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA
   (xi) SEQUENCE DESCRIPTION: SEQ ID NO:41:
   CAACAATGCT AGCGGAGAAG GATCAGTGAG 30
(2) INFORMATION FOR SEQ ID NO:42:
   (i) SEQUENCE CHARACTERISTICS:
      (A) LENGTH: 33 base pairs
      (B) TYPE: nucleic acid
      (C) STRAMDEDNESS: single
      (D) TOPOLOGY; linear
   (ii) MOLECULE TYPE: DNA
   (xi) SEQUENCE DESCRIPTION: SEQ ID NO:42:
   CAACAATGCT AGCCTCCACC CACCGAGCAG AAG 33